# EUROPEAN PATENT APPLICATION

(11) **EP 2 080 761 A1**
(43) Date of publication of application: **22.07.2009**
(21) Application number: 08382002.7
(22) Date of filing: 18.01.2008
(51) Int. Cl.: C07D 471/04, A61K 31/4375, A61P 31/04

(54) **Compounds**

(71) Applicant: GLAXO GROUP LIMITED, Greenford, Middlesex UB6 0NN (GB)
(72) Inventor: The designation of the inventor has not yet been filed
(74) Representative: Elzaburu Marquez, Alberto

(57) **Abstract**

Compounds of Formula (I), 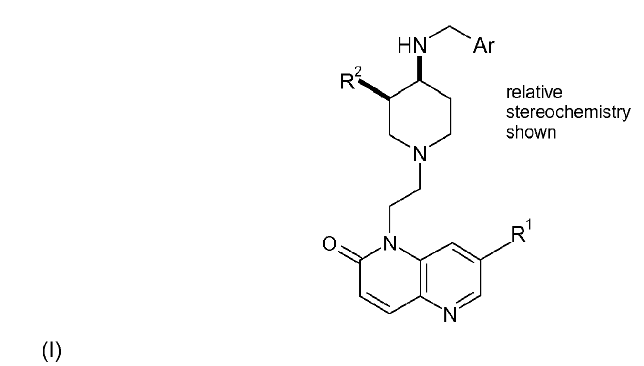 compositions containing them, their use in therapy, including their use as antibacterials, for example in the treatment of tuberculosis, and methods for the preparation of such compounds, are provided.

## Description

### Field of the invention

This invention relates to compounds, compositions containing them, their use in therapy, including their use as antibacterials, for example in the treatment of tuberculosis, and methods for the preparation of such compounds.

### Background of the invention

PCT patent publications WO02/08224, WO02/50061, WO02/56882, WO02/96907, W02003087098, W02003010138, W02003064421, W02003064431, W02004002992, W02004002490, W02004014361, W02004041210, W02004096982, W02002050036, W02004058144, W02004087145, W02006002047, W02006014580, W02006010040, W02006017326, W02006012396, W02006017468, W02006020561, W02006081179, W02006081264, W02006081289, W02006081178, W02006081182, WO01/25227, WO02/40474, WO02/07572, W02004024712, W02004024713, W02004035569, W02004087647, W02004089947, W02005016916, W02005097781, W02006010831, W02006021448, W02006032466, W02006038172, W02006046552, W02006099884, W02006105289, W02006081178, W02006081182, W02007016610, W02007081597, W02007071936, W02007115947, W02007118130 and W02007122258 disclose quinoline, naphthyridine, morpholine, cyclohexane, piperidine and piperazine derivatives and also tricyclic condensed ring compounds, having antibacterial activity. W02004104000 discloses tricyclic condensed ring compounds capable of selectively acting on cannabinoid receptors.

Synthetic drugs for treating tuberculosis (TB) have been available for over half a century, but incidences of the disease continue to rise world-wide. In 2004, it is estimated that 24,500 people developed active disease and close to 5,500 died each day from TB (World Health Organization, Global Tuberculosis Control: Surveillance, Planning, Financing. WHO Report 2006, Geneva, Switzerland, ISBN 92-4 156314-1). Co-infection with HIV is driving the increase in incidence (Williams, B. G.; Dye, C. Science, 2003, 301, 1535) and the cause of death in 31 % of AIDS patients in Africa can be attributed to TB (Corbett, E. L.; Watt, C. J.; Catherine, J.; Walker, N.; Maher D.; Williams, B. G.; Raviglione, M. C.; Dye, C. Arch. Intl. Med., 2003, 163, 1009, Septkowitz, A.; Raffalli, J.; Riley, T.; Kiehn, T. E.; Armstrong, D. Clin. Microbiol. Rev. 1995, 8, 180). When coupled with the emergence of multi-drug resistant strains of *Mycobacterium tuberculosis* (MDR-TB), the scale of the problem is amplified. It is now more than a decade since the WHO declared TB "a global health emergency" (World Health Organization, Global Tuberculosis Control: Surveillance, Planning, Financing. WHO Report 2006, Geneva, Switzerland, ISBN 92-4 156314-1).

The limitations of tuberculosis therapy and prevention are well known. The current available vaccine, BCG was introduced in 1921 and fails to protect most people past childhood. Patients who do become infected with active disease currently endure combination therapy with isoniazid, rifampin, pyrazinamide and ethambutol for two months and then continue taking isoniazid and rifampin for a further four months. Daily dosing is required and poor compliance drives the emergence and spread of multi-drug-resistant strains, which are challenging to treat. A recently published detailed review discusses many aspects of TB such as pathogenesis, epidemiology, drug discovery and vaccine development to date (Nature Medicine, Vol 13(3), pages 263-312).

Shorter courses of more active agents which can be taken less frequently and which present a high barrier to the emergence of resistance, i.e. agents which are effective against multi-drug resistant strains of TB (MDR-TB), are urgently required. There is therefore a need to discover and develop new chemical entities to treat TB (recent synthetic leads are reviewed in: Ballell, L.; Field, R. A.; Duncan, K.; Young, R. J. Antimicrob. Agents Chemother. 2005, 49, 2153).

### Detailed description of the invention

The present invention provides a compound of Formula (I) or a pharmaceutically acceptable salt, solvate or N-oxide thereof: 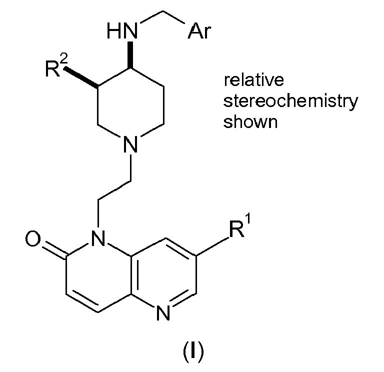 Wherein:
R¹ represents hydrogen; halo; or C₁₋₃alkoxy-;
R² represents hydrogen or hydroxy;
Ar represents a group selected from: phenyl, pyridyl, pyridazinyl, pyrimidinyl, pyrazinyl, thiazolyl, furanyl, imidazolyl and thiophenyl;
   wherein
   Ar is substituted by a first group R³, wherein R³ represents a group selected from: halo, CF₃, C₁₋₃alkyl, nitro and cyano; when represents pyridyl, R³ alternatively represents C₁₋₃alkoxy-;
   Ar is optionally substituted by a second group R⁴;
   when R³ represents halo, the optional R⁴ group represents halo;
   when R³ represents CF₃, the optional R⁴ group represents halo;
   when R³ represents C₁₋₃alkyl, the optional R⁴ group is selected from halo, CF₃, C₁₋₃alkyl, nitro and C₁₋₃alkoxy-;
   when R³ represents nitro, the optional R⁴ group is selected from halo and CF₃;
   when R₃ represents cyano, the optional R⁴ group is selected from halo, CF₃, C₁₋₃alkyl and nitro;
   when R³ represents C₁₋₃alkoxy-, the optional R⁴ group is selected from halo and nitro.

The invention further provides a pharmaceutical composition comprising a compound of Formula (I), or a pharmaceutically acceptable salt, solvate or N-oxide thereof, and one or more pharmaceutically acceptable carriers, excipients or diluents.

The invention also provides a method of treatment of tuberculosis in mammals, particularly in man, which method comprises the administration to a mammal in need of such treatment an effective amount of a compound of Formula (I), or a pharmaceutically acceptable salt, solvate or N-oxide thereof.

This invention further provides a method of treatment of bacterial infections in mammals, particularly in man, which method comprises the administration to a mammal in need of such treatment an effective amount of a compound of formula (I), or a pharmaceutically acceptable salt, solvate or N-oxide thereof.

The invention further provides a compound of Formula (I), or a pharmaceutically acceptable salt, solvate or N-oxide thereof, for use in therapy.

The invention yet further provides a compound of Formula (I), or a pharmaceutically acceptable salt, solvate or N-oxide thereof, for use in the treatment of tuberculosis in mammals, particularly in man.

The invention yet further provides a compound of Formula (I), or a pharmaceutically acceptable salt, solvate or N-oxide thereof, for use in the treatment of bacterial infections in mammals, particularly in man.

The invention still further provides the use of a compound of Formula (I), or a pharmaceutically acceptable salt, solvate or N-oxide thereof, in the manufacture of a medicament for use in the treatment of tuberculosis in mammals, particularly in man.

The invention still further provides the use of a compound of Formula (I), or a pharmaceutically acceptable salt, solvate or N-oxide thereof, in the manufacture of a medicament for use in the treatment of bacterial infections in mammals, particularly in man.

The invention also provides a pharmaceutical composition comprising a compound of Formula (I), or a pharmaceutically acceptable salt, solvate or N-oxide thereof, and one or more pharmaceutically acceptable carriers, excipients or diluents, for use in the treatment of tuberculosis in mammals, particularly in man.

The invention also provides a pharmaceutical composition comprising a compound of Formula (I), or a pharmaceutically acceptable salt, solvate or N-oxide thereof, and one or more pharmaceutically acceptable carriers, excipients or diluents, for use in the treatment of bacterial infections in mammals, particularly in man.

In one aspect of the invention, when Ar represents phenyl, the substituent R³ and optional substituent R⁴ occur in the meta- or para- positions relative to the point of attachment of Ar to the remainder of the molecule.

In one aspect of the invention, when R² represents hydroxy, the absolute stereochemistry of the compound of Formula (I), or a pharmaceutically acceptable salt, solvate or N-oxide thereof, is

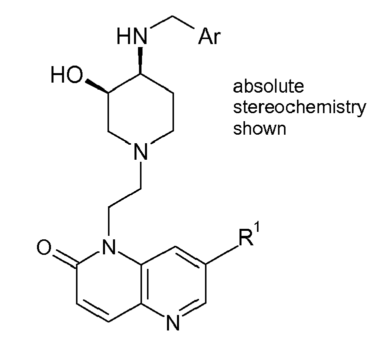

In one aspect of the invention, Ar represents a group selected from: phenyl, pyridyl, pyridazinyl, pyrazinyl, thiazolyl, furanyl and thiophenyl.

In one aspect of the invention,
when R³ represents halo, the optional R⁴ group represents halo;
when R³ represents CF₃, the optional R⁴ group represents halo;
when R³ represents C₁₋₃alkyl, the optional R⁴ group is selected from halo, C₁₋₃alkyl and nitro;
when R³ represents C₁₋₃alkoxy-, the optional R⁴ group is selected from halo and nitro.

In one aspect of the invention, the substituent R⁴ is present. In another aspect of the invention, the substituent R³ is halo, for example chloro, and the substituent R⁴ is present and is halo, for example chloro.

In one aspect, compounds which are useful in the present invention are the pharmaceutically acceptable salts of a compound of Formula (I).

In one aspect, compounds which are useful in the present invention include those mentioned in the examples and their pharmaceutically acceptable salts, solvates or N-oxides.

In another aspect, compounds which are useful in the present invention include:
1-[2-(4-{[(5-bromo-2-pyridinyl)methyl]amino}-1-piperidinyl)ethyl]-7-fluoro-1,5-naphthyridin-2(1 H)-one;
1-[2-(4-{[(5-bromo-2-pyridinyl)methyl]amino}-1-piperidinyl)ethyl]-7-fluoro-1,5-naphthyridin-2(1 H)-one dihydrochloride;
1-[2-(4-{[(2-bromo-1,3-thiazol-5-yl)methyl]amino}-1-piperidinyl)ethyl]-7-(methyloxy)-1,5-naphthyridin-2(1H)-one;
1-[2-(4-{[(2-bromo-1,3-thiazol-5-yl)methyl]amino}-1-piperidinyl)ethyl]-7-(methyloxy)-1,5-naphthyridin-2(1H)-one hydrochloride;
1-[2-(4-{[(3,4-dichlorophenyl)methyl]amino}-1-piperidinyl)ethyl]-7-fluoro-1,5-naphthyridin-2(1 H)-one;
1-[2-(4-{[(3,4-dichlorophenyl)methyl]amino}-1-piperidinyl)ethyl]-7-fluoro-1,5-naphthyridin-2(1 H)-one;
7-(methyloxy)-1-{2-[4-({[6-(trifluoromethyl)-3-pyridinyl]methyl}amino)-1-piperidinyl]ethyl}-1,5-naphthyridin-2(1H)-one;
7-(methyloxy)-1-{2-[4-({[6-(trifluoromethyl)-3-pyridinyl]methyl}amino)-1-piperidinyl]ethyl}-1,5-naphthyridin-2(1H)-one hydrochloride;
1-[2-(4-{[(4-fluoro-3-methylphenyl)methyl]amino}-1-piperidinyl)ethyl]-7-(methyloxy)-1,5-naphthyridin-2(1H)-one;
1-[2-(4-{[(4,5-dimethyl-1,3-thiazol-2-yl)methyl]amino}-1-piperidinyl)ethyl]-7-(methyloxy)-1,5-naphthyridin-2(1H)-one;
1-[2-(4-{[(4,5-dichloro-1,3-thiazol-2-yl)methyl]amino}-1-piperidinyl)ethyl]-7-(methyloxy)-1,5-naphthyridin-2(1H)-one;
1-[2-(4-{[(3,4-dichlorophenyl)methyl]amino}-1-piperidinyl)ethyl]-1,5-naphthyridin-2(1H)-one;
1-[2-(4-{[(3,4-dichlorophenyl)methyl]amino}-1-piperidinyl)ethyl]-1,5-naphthyridin-2(1H)-one dihydrochloride;
1-[2-(4-{[(6-bromo-3-pyridinyl)methyl]amino}-1-piperidinyl)ethyl]-1,5-naphthyridin-2(1H)-one;
7-fluoro-1-{2-[4-({[6-(trifluoromethyl)-3-pyridinyl]methyl}amino)-1-piperidinyl]ethyl}-1,5-naphthyridin-2(1H)-one;
1-[2-(4-{[(3,4-dichlorophenyl)methyl]amino}-1-piperidinyl)ethyl]-7-(methyloxy)-1,5-naphthyridin-2(1H)-one;
1-[2-(4-{[(3,4-dichlorophenyl)methyl]amino}-1-piperidinyl)ethyl]-7-(methyloxy)-1,5-naphthyridin-2(1H)-one hydrochloride;
1-[2-(4-{[(3,4-dichlorophenyl)methyl]amino}-1-piperidinyl)ethyl]-7-(methyloxy)-1,5-naphthyridin-2(1H)-one dihydrochloride;
1-[2-(4-{[(4-chlorophenyl)methyl]amino}-1-piperidinyl)ethyl]-7-(methyloxy)-1,5-naphthyridin-2(1H)-one;
1-[2-(4-{[(4-chlorophenyl)methyl]amino}-1-piperidinyl)ethyl]-7-(methyloxy)-1,5-naphthyridin-2(1H)-one dihydrochloride;
1-[2-(4-{[(4-chlorophenyl)methyl]amino}-1-piperidinyl)ethyl]-7-(methyloxy)-1,5-naphthyridin-2(1H)-one hydrochloride;
1-[2-(4-{[(6-bromo-3-pyridinyl)methyl]amino}-1-piperidinyl)ethyl]-7-fluoro-1,5-naphthyridin-2(1H)-one;
7-fluoro-1-[2-(4-{[(4-fluorophenyl)methyl]amino}-1-piperidinyl)ethyl]-1,5-naphthyridin-2(1H)-one;
7-(methyloxy)-1-{2-[4-({[6-(trifluoromethyl)-3-pyridazinyl]methyl}amino)-1-piperidinyl]ethyl}-1,5-naphthyridin-2(1H)-one;
1-[2-(4-{[(5-Bromo-3-isothiazolyl)methyl]amino}-1-piperidinyl)ethyl]-7-(methyloxy)-1,5-naphthyridin-2(1H)-one;
1-[2-(4-{[(4-chlorophenyl)methyl]amino}-1-piperidinyl)ethyl]-7-(ethyloxy)-1,5-naphthyridin-2(1H)-one;
7-(ethyloxy)-1-{2-[4-({[6-(trifluoromethyl)-3-pyridinyl]methyl}amino)-1-piperidinyl]ethyl}-1,5-naphthyridin-2(1H)-one;
1-[2-(4-{[(3,4-dichlorophenyl)methyl]amino}-1-piperidinyl)ethyl]-7-(ethyloxy)-1,5-naphthyridin-2(1H)-one;
1-[2-(4-{[(5-chloro-6-methyl-3-pyridinyl)methyl]amino}-1-piperidinyl)ethyl]-7-fluoro-1,5-naphthyridin-2(1H)-one;
1-[2-(4-{[(4-bromo-2-pyridinyl)methyl]amino}-1-piperidinyl)ethyl]-7-(methyloxy)-1,5-naphthyridin-2(1H)-one;
1-[2-(4-{[(5-bromo-3-pyridinyl)methyl]amino}-1-piperidinyl)ethyl]-7-(methyloxy)-1,5-naphthyridin-2(1H)-one;
1-[2-(4-{[(5-chloro-6-methyl-3-pyridinyl)methyl]amino}-1-piperidinyl)ethyl]-7-(methyloxy)-1,5-naphthyridin-2(1H)-one;
1-[2-(4-{[(4-chloro-3-methylphenyl)methyl]amino}-1-piperidinyl)ethyl]-7-(methyloxy)-1,5-naphthyridin-2(1H)-one;
1-[2-(4-{[(3-chloro-4-methylphenyl)methyl]amino}-1-piperidinyl)ethyl]-1,5-naphthyridin-2(1H)-one;
1-[2-((3R,4S)-4-{[(3,4-dichlorophenyl)methyl]amino}-3-hydroxy-1-piperidinyl)ethyl]-1,5-naphthyridin-2(1H)-one;
1-[2-(4-{[(3,4-dimethylphenyl)methyl]amino}-1-piperidinyl)ethyl]-7-fluoro-1,5-naphthyridin-2(1H)-one;
1-[2-(4-{[(4-chloro-3-methylphenyl)methyl]amino}-1-piperidinyl)ethyl]-1,5-naphthyridin-2(1H)-one;
1-[2-(4-{[(5-chloro-6-methyl-3-pyridinyl)methyl]amino}-1-piperidinyl)ethyl]-1,5-naphthyridin-2(1H)-one;
1-[2-(4-{[(5-bromo-6-methyl-2-pyridinyl)methyl]amino}-1-piperidinyl)ethyl]-7-fluoro-1,5-naphthyridin-2(1H)-one;
1-[2-(4-{[(3-fluoro-4-methylphenyl)methyl]amino}-1-piperidinyl)ethyl]-1,5-naphthyridin-2(1H)-one;
1-[2-(4-{[(3,4-dimethylphenyl)methyl]amino}-1-piperidinyl)ethyl]-1,5-naphthyridin-2(1H)-one;
1-[2-(4-{[(4-chloro-5-fluoro-2-pyridinyl)methyl]amino}-1-piperidinyl)ethyl]-7-fluoro-1,5-naphthyridin-2(1H)-one;
1-[2-(4-{[(5-bromo-2-furanyl)methyl]amino}-1-piperidinyl)ethyl]-7-fluoro-1,5-naphthyridin-2(1H)-one;
1-{2-[4-({[6-(trifluoromethyl)-3-pyridinyl]methyl}amino)-1-piperidinyl]ethyl}-1,5-naphthyridin-2(1H)-one;
1-{2-[4-({[4-chloro-3-(trifluoromethyl)phenyl]methyl}amino)-1-piperidinyl]ethyl}-1,5-naphthyridin-2(1H)-one;
7-(methyloxy)-1-[2-(4-{[(4-nitrophenyl)methyl]amino}-1-piperidinyl)ethyl]-1,5-naphthyridin-2(1H)-one;
1-[2-(4-{[(2,5-dichlorophenyl)methyl]amino}-1-piperidinyl)ethyl]-7-fluoro-1,5-naphthyridin-2(1H)-one;
1-[2-(4-{[(6-bromo-3-pyridinyl)methyl]amino}-1-piperidinyl)ethyl]-7-(methyloxy)-1,5-naphthyridin-2(1H)-one;
1-[2-(4-{[(3,5-dichlorophenyl)methyl]amino}-1-piperidinyl)ethyl]-7-fluoro-1,5-naphthyridin-2(1H)-one;
1-[2-(4-{[(4-fluorophenyl)methyl]amino}-1-piperidinyl)ethyl]-7-(methyloxy)-1,5-naphthyridin-2(1H)-one;
1-{2-[4-({[4-(trifluoromethyl)phenyl]methyl}amino)-1-piperidinyl]ethyl}-1,5-naphthyridin-2(1H)-one;
1-[2-(4-{[(4-chlorophenyl)methyl]amino}-1-piperidinyl)ethyl]-1,5-naphthyridin-2(1H)-one;
1-[2-(4-{[(5-chloro-2-thienyl)methyl]amino}-1-piperidinyl)ethyl]-7-fluoro-1,5-naphthyridin-2(1H)-one;
1-{2-[4-({[5-chloro-6-(methyloxy)-3-pyridinyl]methyl}amino)-1-piperidinyl]ethyl}-7-fluoro-1,5-naphthyridin-2(1H)-one;
1-[2-(4-{[(3,5-dichlorophenyl)methyl]amino}-1-piperidinyl)ethyl]-7-(methyloxy)-1,5-naphthyridin-2(1H)-one;
1-{2-[4-({[5-chloro-6-(methyloxy)-3-pyridinyl]methyl}amino)-1-piperidinyl]ethyl}-7-(methyloxy)-1,5-naphthyridin-2(1H)-one;
1-[2-(4-{[(5-bromo-2-thienyl)methyl]amino}-1-piperidinyl)ethyl]-1,5-naphthyridin-2(1H)-one;
1-[2-(4-{[(2-bromo-1,3-thiazol-5-yl)methyl]amino}-1-piperidinyl)ethyl]-1,5-naphthyridin-2(1H)-one;
1-[2-(4-{[(4,5-dibromo-2-thienyl)methyl]amino}-1-piperodinyl)ethyl]-7-(methyloxy)-1,5-naphthyridin-2(1H)-one;
1-{2-[4-({[3-chloro-4-(methyloxy)phenyl]methyl}amino)-1-piperidinyl]ethyl}-1,5-naphthyridin-2(1H)-one;
1-[2-(4-{[(3,4-dibromophenyl)methyl]amino}-1-piperidinyl)ethyl]-1,5-naphthyridin-2(1H)-one;
4-{[(1-{2-[7-(methyloxy)-2-oxo-1,5-naphthyridin-1(2H)-yl]ethyl}-4-piperidinyl)amino]methyl}benzonitrile;
1-[2-(4-{[(5-bromo-2-thienyl)methyl]amino}-1-piperidinyl)ethyl]-7-(methyloxy)-1,5-naphthyridin-2(1H)-one;
1-[2-(4-{[(4,5-dibromo-2-thienyl)methyl]amino}-1-piperidinyl)ethyl]-1,5-naphthyridin-2(1H)-one;
1-[2-(4-{[(5-bromo-4-methyl-2-thienyl)methyl]amino}-1-piperidinyl)ethyl]-7-fluoro-1,5-naphthyridin-2(1H)-one;
1-[2-(4-{[(3-chlorophenyl)methyl]amino}-1-piperidinyl)ethyl]-1,5-naphthyridin-2(1H)-one;
1-[2-(4-{[(3,5-dichlorophenyl)methyl]amino}-1-piperidinyl)ethyl]-1,5-naphthyridin-2(1H)-one;
7-fluoro-1-[2-(4-{[(4-methyl-3-nitrophenyl)methyl]amino}-1-piperidinyl)ethyl]-1,5-naphthyridin-2(1H)-one;
1-[2-(4-{[(4-bromo-1,3-thiazol-2-yl)methyl]amino}-1-piperidinyl)ethyl]-7-fluoro-1,5-naphthyridin-2(1H)-one;
1-[2-(4-{[(3-chloro-4-methylphenyl)methyl]amino}-1-piperidinyl)ethyl]-7-fluoro-1,5-naphthyridin-2(1H)-one;
7-fluoro-1-{2-[4-({[5-fluoro-6-(methyloxy)-3-pyridinyl]methyl}amino)-1-piperidinyl]ethyl}-1,5-naphthyridin-2(1H)-one;
1-[2-(4-{[(5-bromo-2-furanyl)methyl]amino}-1-piperidinyl)ethyl]-7-(methyloxy)-1,5-naphthyridin-2(1H)-one;
1-[2-(4-{[(4-bromo-1,3-thiazol-2-yl)methyl]amino}-1-piperidinyl)ethyl]-7-(methyloxy)-1,5-naphthyridin-2(1H)-one;
1-[2-(4-{[(5-bromo-4-methyl-2-thienyl)methyl]amino}-1-piperidinyl)ethyl]-1,5-naphthyridin-2(1H)-one;
1-{2-[4-({[5-fluoro-6-(methyloxy)-3-pyridinyl]methyl}amino)-1-piperidinyl]ethyl}-7-(methyloxy)-1,5-naphthyridin-2(1H)-one;
7-(methyloxy)-1-{2-[4-({[6-(methyloxy)-3-pyridinyl]methyl}amino)-1-piperidinyl]ethyl}-1,5-naphthyridin-2(1H)-one;
1-[2-(4-{[(5-fluoro-6-methyl-2-pyridinyl)methyl]amino}-1-piperidinyl)ethyl]-7-(methyloxy)-1,5-naphthyridin-2(1H)-one;
1-[2-(4-{[(3-chloro-4-methylphenyl)methyl]amino}-1-piperidinyl)ethyl]-7-(methyloxy)-1,5-naphthyridin-2(1H)-one;
1-[2-(4-{[(6-fluoro-5-methyl-3-pyridinyl)methyl]amino}-1-piperidinyl)ethyl]-7-(methyloxy)-1,5-naphthyridin-2(1H)-one;
1-[2-(4-{[(4-chloro-5-fluoro-2-pyridinyl)methyl]amino}-1-piperidinyl)ethyl]-7-(methyloxy)-1,5-naphthyridin-2(1H)-one;
1-[2-(4-{[(5-bromo-4-methyl-1,3-thiazol-2-yl)methyl]amino}-1-piperidinyl)ethyl]-7-fluoro-1,5-naphthyridin-2(1H)-one;
1-[2-(4-{[(3,5-Dimethylphenyl)methyl]amino}-1-piperidinyl)ethyl]-7-(methyloxy)-1,5-naphthyridin-2(1H)-one;
1-[2-(4-{[(3,4-Dimethylphenyl)methyl]amino}-1-piperidinyl)ethyl]-7-(methyloxy)-1,5-naphthyridin-2(1H)-one;
1-[2-(4-{[(3-fluoro-5-methylphenyl)methyl]amino}-1-piperidinyl)ethyl]-7-(methyloxy)-1,5-naphthyridin-2(1H)-one;
1-[2-(4-{[(4-bromo-3-methylphenyl)methyl]amino}-1-piperidinyl)ethyl]-7-(methyloxy)-1,5-naphthyridin-2(1H)-one;
1-[2-(4-{[(5,6-dichloro-3-pyridinyl)methyl]amino}-1-piperidinyl)ethyl]-7-(methyloxy)-1,5-naphthyridin-2(1H)-one;
1-[2-(4-{[(3-fluoro-4-methylphenyl)methyl]amino}-1-piperidinyl)ethyl]-7-(methyloxy)-1,5-naphthyridin-2(1H)-one;
1-[2-(4-{[(4-chloro-3-methylphenyl)methyl]amino}-1-piperidinyl)ethyl]-7-fluoro-1,5-naphthyridin-2(1H)-one;
1-[2-(4-{[(5-bromo-3-pyridinyl)methyl]amino}-1-piperidinyl)ethyl]-7-fluoro-1,5-naphthyridin-2(1H)-one;
1-[2-(4-{[(4-bromo-2-thienyl)methyl]amino}-1-piperidinyl)ethyl]-1,5-naphthyridin-2(1H)-one;
1-[2-(4-{[(3,4-dibromophenyl)methyl]amino}-1-piperidinyl)ethyl]-7-fluoro-1,5-naphthyridin-2(1H)-one;
1-[2-(4-{[(5-bromo-2-thienyl)methyl]amino}-1-piperidinyl)ethyl]-7-fluoro-1,5-naphthyridin-2(1H)-one;
7-fluoro-1-{2-[4-({[4-(trifluoromethyl)phenyl]methyl}amino)-1-piperidinyl]ethyl}-1,5-naphthyridin-2(1H)-one;
1-[2-(4-{[(3,4-difluorophenyl)methyl]amino}-1-piperidinyl)ethyl]-7-fluoro-1,5-naphthyridin-2(1H)-one;
1-[2-(4-{[(5-bromo-6-methyl-2-pyridinyl)methyl]amino}-1-piperidinyl)ethyl]-7-(methyloxy)-1,5-naphthyridin-2(1H)-one;
1-[2-(4-{[(3,4-dibromophenyl)methyl]amino}-1-piperidinyl)ethyl]-7-(methyloxy)-1,5-naphthyridin-2(1H)-one;
7-(methyloxy)-1-{2-[4-({[4-(trifluoromethyl)phenyl]methyl}amino)-1-piperidinyl]ethyl}-1,5-naphthyridin-2(1H)-one;
1-[2-(4-{[(3,4-difluorophenyl)methyl]amino}-1-piperidinyl)ethyl]-7-(methyloxy)-1,5-naphthyridin-2(1H)-one;
1-[2-(4-{[(3-chlorophenyl)methyl]amino}-1-piperidinyl)ethyl]-7-(methyloxy)-1,5-naphthyridin-2(1H)-one;
1-{2-[4-({[4-chloro-3-(trifluoromethyl)phenyl]methyl}amino)-1-piperidinyl]ethyl}-7-(methyloxy)-1,5-naphthyridin-2(1H)-one;
1-[2-(4-{[(2,4-dichlorophenyl)methyl]amino}-1-piperidinyl)ethyl]-7-(methyloxy)-1,5-naphthyridin-2(1H)-one;
1-[2-(4-{[(5-bromo-2-pyridinyl)methyl]amino}-1-piperidinyl)ethyl]-7-(methyloxy)-1,5-naphthyridin-2(1H)-one;
1-[2-(4-{[(2-chloro-1,3-thiazol-5-yl)methyl]amino}-1-piperidinyl)ethyl]-7-(methyloxy)-1,5-naphthyridin-2(1H)-one;
1-[2-(4-{[(5-bromo-4-methyl-2-thienyl)methyl]amino}-1-piperidinyl)ethyl]-7-(methyloxy)-1,5-naphthyridin-2(1H)-one
1-[2-((3R,4S)-4-{[(3,4-dichlorophenyl)methyl]amino}-3-hydroxy-1-piperidinyl)ethyl]-7-fluoro-1,5-naphthyridin-2(1H)-one;
1-{2-[(3R,4S)-3-hydroxy-4-({[6-(trifluoromethyl)-3-pyridinyl]methyl}amino)-1-piperidinyl]ethyl}-7-(methyloxy)-1,5-naphthyridin-2(1H)-one;
1-[2-((3R,4S)-4-{[(3,4-dichlorophenyl)methyl]amino}-3-hydroxy-1-piperidinyl)ethyl]-7-(methyloxy)-1,5-naphthyridin-2(1H)-one;
1-{2-[(3S,4R)-3-hydroxy-4-({[6-(trifluoromethyl)-3-pyridinyl]methyl}amino)-1-piperidinyl]ethyl}-7-(methyloxy)-1,5-naphthyridin-2(1H)-one;
1-[2-((3S,4R)-4-{[(3,4-dichlorophenyl)methyl]amino}-3-hydroxy-1-piperidinyl)ethyl]-7-(methyloxy)-1,5-naphthyridin-2(1H)-one;
1-{2-[(3R,4S)-3-hydroxy-4-({[6-(trifluoromethyl)-3-pyridinyl]methyl}amino)-1-piperidinyl]ethyl}-1,5-naphthyridin-2(1H)-one;
7-fluoro-1-{2-[(3R,4S)-3-hydroxy-4-({[6-(trifluoromethyl)-3-pyridinyl]methyl}amino)-1-piperidinyl]ethyl}-1,5-naphthyridin-2(1H)-one;
1-[2-(4-{[(3-chlorophenyl)methyl]amino}-1-piperidinyl)ethyl]-7-fluoro-1,5-naphthyridin-2(1H)-one;
1-[2-(4-{[(4-chlorophenyl)methyl]amino}-1-piperidinyl)ethyl]-7-fluoro-1,5-naphthyridin-2(1H)-one;
1-[2-(4-{[(4-bromo-2-thienyl)methyl]amino}-1-piperidinyl)ethyl]-7-(methyloxy)-1,5-naphthyridin-2(1H)-one;
1-[2-(4-{[(5-chloro-2-thienyl)methyl]amino}-1-piperidinyl)ethyl]-7-(methyloxy)-1,5-naphthyridin-2(1H)-one;
1-[2-(4-{[(2-bromo-1,3-thiazol-5-yl)methyl]amino}-1-piperidinyl)ethyl]-7-fluoro-1,5-naphthyridin-2(1H)-one;
1-[2-(4-{[(4,5-dibromo-2-thienyl)methyl]amino}-1-piperidinyl)ethyl]-7-fluoro-1,5-naphthyridin-2(1H)-one;
1-[2-(4-{[(4-bromo-2-thienyl)methyl]amino}-1-piperidinyl)ethyl]-7-fluoro-1,5-naphthyridin-2(1H)-one;
7-(methyloxy)-1-{2-[4-({[5-(methyloxy)-6-nitro-2-pyridinyl]methyl}amino)-1-piperidinyl]ethyl}-1,5-naphthyridin-2(1H)-one;
1-[2-(4-{[(6-chloro-2-pyrazinyl)methyl]amino}-1-piperidinyl)ethyl]-7-(methyloxy)-1,5-naphthyridin-2(1H)-one;
or a pharmaceutically acceptable salt, solvate or N-oxide thereof.

### Terms and definitions

The term "(C₁₋₃)alkyl" as used herein refers to a straight or branched chain alkyl group having 1 to 3 carbon atoms. Examples of (C₁₋₃)alkyl groups include methyl, ethyl, n-propyl, iso-propyl.

The term "halo" as used herein refers to fluoro, chloro, bromo and iodo groups. In one aspect, the term "halo" as used herein refers to fluoro, chloro and bromo groups. In another aspect, the term "halo" as used herein refers to chloro, bromo and iodo groups.

The term "(C₁₋₃)alkoxy" as used herein refers to a straight or branched chain alkoxy group having 1 to 3 carbon atoms. Examples of (C₁₋₃)alkoxy groups include, methoxy, ethoxy, propoxy and isopropoxy.

The term "compounds of the invention" as used herein means a compound of Formula (I) or a pharmaceutically acceptable salt, solvate or N-oxide thereof. The term "a compound of the invention" means any one of the compounds of the invention as defined above.

Furthermore, it will be understood that phrases such as "a compound of Formula (I) or a pharmaceutically acceptable salt, solvate or N-oxide thereof" or "compounds of the invention" are intended to encompass the compound of Formula (I), a pharmaceutically acceptable salt, solvate or N-oxide of the compound of Formula (I), or any pharmaceutically acceptable combination of these. Thus by way of non-limiting example used here for illustrative purpose, "a compound of Formula (I) or a pharmaceutically acceptable salt, solvate or N-oxide thereof" encompasses a pharmaceutically acceptable salt of a compound of Formula (I) which is present as a solvate, or this phrase may include a mixture of a compound of Formula (I) and a salt of a compound of Formula (I).

It will be further appreciated that all crystalline forms, polymorphs and enantiomers of the compounds of the invention, or mixtures thereof, are contemplated to be within the scope of the present invention. Unless otherwise specified (for example when the absolute stereochemistry is shown), for compounds of the invention which possesses stereocentres and which can therefore form enantiomers, (for example, when R² represents hydroxy), the compound contains a 1:1 mixture of enantiomers, i.e. a racemic mixture of enantiomers. These may be separated using conventional techniques such as chiral HPLC.

Some of the compounds of this invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Since the compounds of Formula (I) are intended for use in pharmaceutical compositions it will readily be understood that in particular embodiments they are provided in substantially pure form, for example at least 60% pure, more suitably at least 75% pure and particularly at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5% and more particularly from 10 to 59% of a compound of Formula (I) or pharmaceutically acceptable salt, solvate and/or N-oxide thereof.

Pharmaceutically acceptable salts of the compounds of Formula (I) include the acid addition or quaternary ammonium salts, for example their salts with mineral acids e.g. hydrochloric, hydrobromic, sulphuric nitric or phosphoric acids, or organic acids, e.g. acetic, fumaric, succinic, maleic, citric, benzoic, p-toluenesulphonic, methanesulphonic, naphthalenesulphonic acid or tartaric acids. In one aspect of the invention, the salt of a compound of Formula (I) is the hydrochloride salt. In another aspect, the salt of a compound of Formula (I) is the dihydrochloride salt. Compounds of Formula (I) may also be prepared as the N-oxide. The invention extends to all such salts, solvates and/or N-oxides.

### Compound Preparation

The general procedures used to synthesise the compounds of Formula (I), are described in reaction Schemes 1-11 and are illustrated in the Examples.

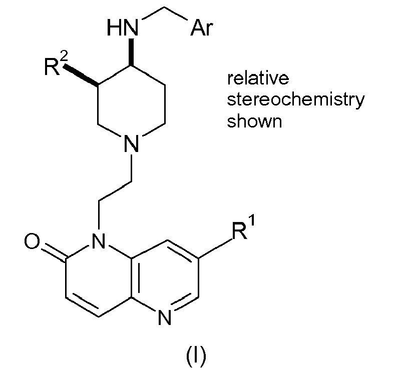

Compounds of Formula (I), wherein R¹, R² and Ar are as defined for Formula (I), may be prepared by a reductive amination reaction between a compound of Formula (II), wherein R¹ and R² are as defined for Formula (I), or an acid salt of a compound of Formula (II) such as a hydrochloride salt, and an aldehyde of Formula (III), wherein Ar is as defined for Formula (I), according to Scheme 1. Compounds (II) are reacted with compounds (III) in the presence of a hydride donor such as NaBH(AcO)₃ or polymer-supported NaBH₃CN, optionally in the presence of a catalytic acid such as acetic acid or a base such as triethylamine, in the presence of a suitable solvent such as 1,2-dicloroethane, or THF, or a mixture of DCM and MeOH, to give compounds (I).

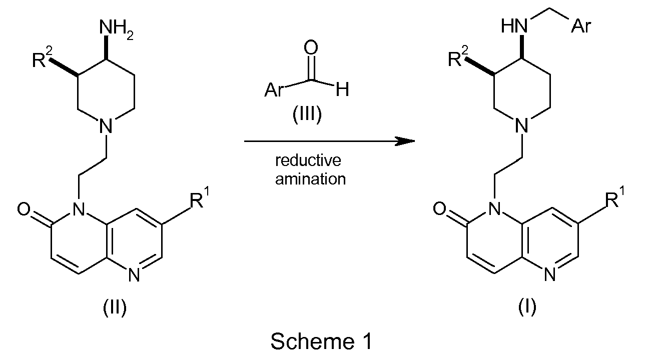

Alternatively, compounds of Formula (I), wherein R¹ and Ar are as defined for Formula (I) and R² is hydrogen, may be prepared by an alkylation reaction between a compound of Formula (II), wherein R¹ is as defined for Formula (I) and R² is hydrogen, and an alkylating agent of Formula (IV), wherein hal is a halo group, for example bromo, according to Scheme 2. Compounds (II) are reacted with compounds (IV) in the presence of a suitable base such as K₂CO₃ in a suitable solvent such as acetonitrile, to give compounds (I).

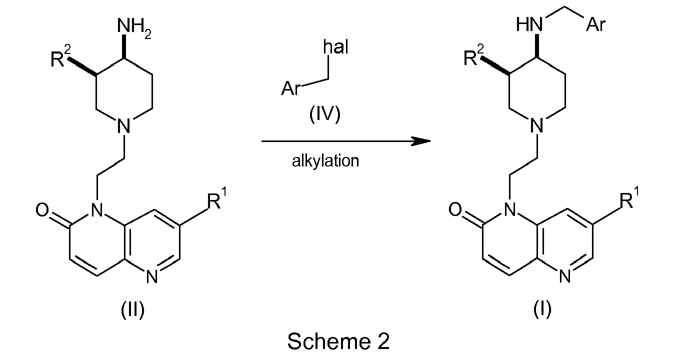

The acid salts of compounds of Formula (I), wherein R¹, R² and Ar are as defined for Formula (I), may be prepared by treating a solution of a compound of Formula (I) in a suitable solvent such as DCM with a suitable acid. For example, to make a hydrochloride salt of a compound of Formula (I), a solution of HCl in 1,4-dioxane or in MeOH may be employed. If a monohydrochloride salt of a compound of Formula (I) is required, for example one equivalent of HCl may be used. If a dihydrochloride salt of a compound of Formula (I) is required, an excess of HCl may be used.

Compounds of Formula (II), wherein R² is as defined for Formula (I) and R¹ is hydrogen; halo; or C₁₋₃alkoxy-, may be prepared by a reductive amination reaction between compounds of Formula (V), wherein R¹ is hydrogen; halo; or C₁₋₃alkoxy-, and compounds of Formula (VI), wherein R² is as defined for Formula (I) and P is a nitrogen protecting group such as BOC, followed by a deprotection reaction, according to Scheme 3. Compounds (V) are reacted with compounds (VI) in the presence of a hydride donor such as NaBH(AcO)₃, in a suitable solvent such as CHCl₃ or DCM, or a mixture of either of these solvents with MeOH, followed by a deprotection reaction to remove the protecting group P. For Example, when the protecting group is BOC, this may be removed by treatment with a suitable acid such as HCl (for example a solution in 1,4-dioxane) in a suitable solvent such as DCM, to give the hydrochloride salt of compounds (II). If the free base of compound (II) is required, this may be followed by treatment with a suitable base such as aqueous NaOH with cooling, to give compounds (II).

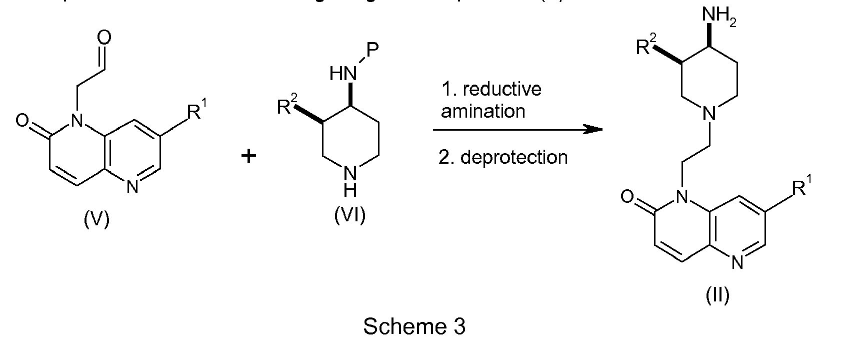

Compounds of Formula (II), wherein R² is hydrogen and R¹ is C₁₋₃alkoxy-, may be prepared from a compound of Formula (II), wherein R² is hydrogen and R¹ is fluoro, as shown in Scheme 4. The fluoro compound of Formula (II) may be treated with a suitable base such as NaH in a suitable solvent such as a mixture of 1,4-dioxane and the appropriate alcohol C₁₋₃alkylOH, in the presence of heat, for example in a microwave oven.

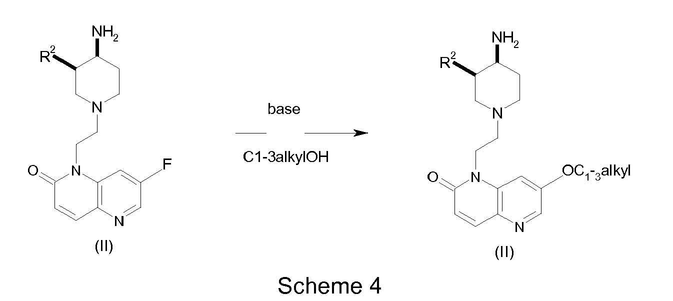

Compounds of Formula (VI), wherein R² is hydrogen are commercially available (for example from Aldrich). Compounds of Formula (VI), wherein R² is hydroxy and protecting group P is for example BOC, may be prepared according to the procedure given in W02004058144, Example 5(c), cis-(3-hydroxy-piperidin-4-yl)-carbamic acid *tert*-butyl ester Enantiomer 1.

Compounds of Formula (V), wherein R¹ is hydrogen; halo; or C₁₋₃alkoxy-, may be prepared by oxidation of a compound of Formula (VII), wherein R¹ is hydrogen; halo; or C₁₋₃alkoxy-, according to Scheme 5. Compounds (VII) are treated with a suitable oxidising agent such as a mixture of sodium periodate and osmium tetroxide, in a suitable solvent such as a mixture of 1,4-dioxane and water, to give compounds (V).

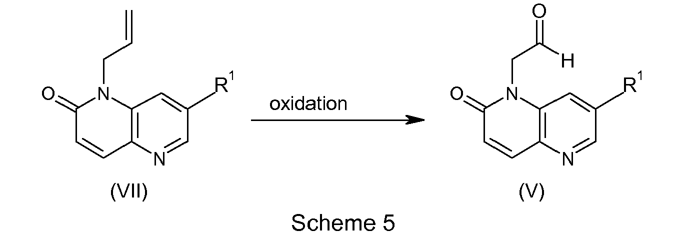

Compounds of Formula (VII), wherein R¹ is hydrogen; halo; or C₁₋₃alkoxy-, may be prepared by N-alkylation of compounds of Formula (VIII), wherein R¹ is hydrogen; halo; or C₁₋₃alkoxy-, according to Scheme 6. Compounds (VIII) are treated with a compound of Formula CH₂=CH-CH₂-hal, wherein hal is a halo group, for example with allyl bromide, in the presence of a suitable base such as NaH and optionally a catalyst such as LiBr, in a suitable solvent such as a mixture of DME and DMF, at elevated temperature, for example from 50-75°C, to give compounds (VIII).

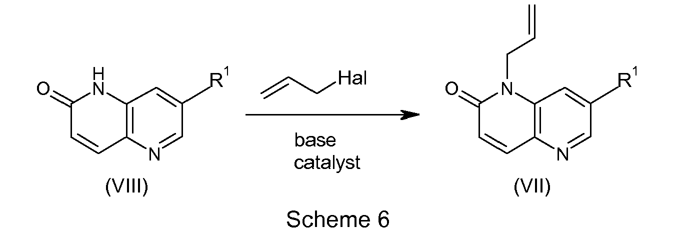

Compounds of Formula (VIII), wherein R¹ is hydrogen; halo; or C₁₋₃alkoxy-, may be prepared by a hydrolysis reaction of compounds of Formula (IX), wherein R¹ is hydrogen; halo; or C₁₋₃alkoxy-, according to Scheme 7. Compounds (IX) may be treated with an acid, such as aqueous HCl, at elevated temperature, for example 90-110°C, to give compounds (VIII).

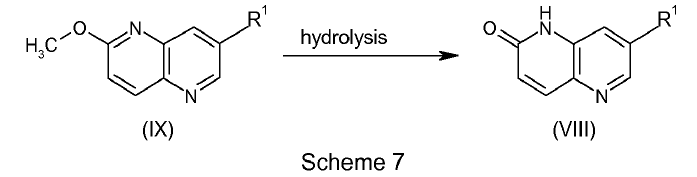

Compounds of Formula (IX), wherein R¹ is hydrogen; halo; or C₁₋₃alkoxy-, may be prepared from compounds of Formula (X), wherein R¹ is hydrogen; halo; or C₁₋₃alkoxy-, and halo is chloro or bromo, for example bromo, according to Scheme 8. Compounds (X) are subjected to hydrogenation in the presence of a suitable catalyst such as palladium on charcoal, in the presence of a suitable base such as NaHCO₃, to give compounds (IX).

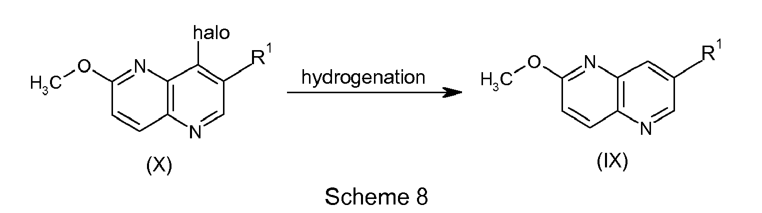

Compounds of Formula (X), wherein R¹ is hydrogen or halo, may be prepared from compounds of Formula (XI), wherein R¹ is hydrogen or halo, according to Scheme 9. Compounds (XI) are treated with a source of chloride or bromide, such as PBr₃, in a suitable solvent such as DMF, to give compounds (X).

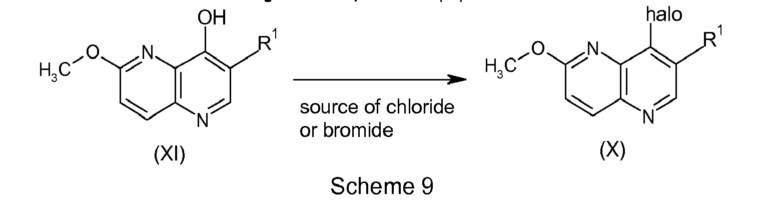

The compound of Formula (XI), wherein R¹ is hydrogen, may be prepared according to the procedure provided in W02007016610, preparation 2 (a).

Compounds of Formula (XI), wherein R¹ is halo, for example fluoro, may be prepared according to the synthesis in Scheme 10.

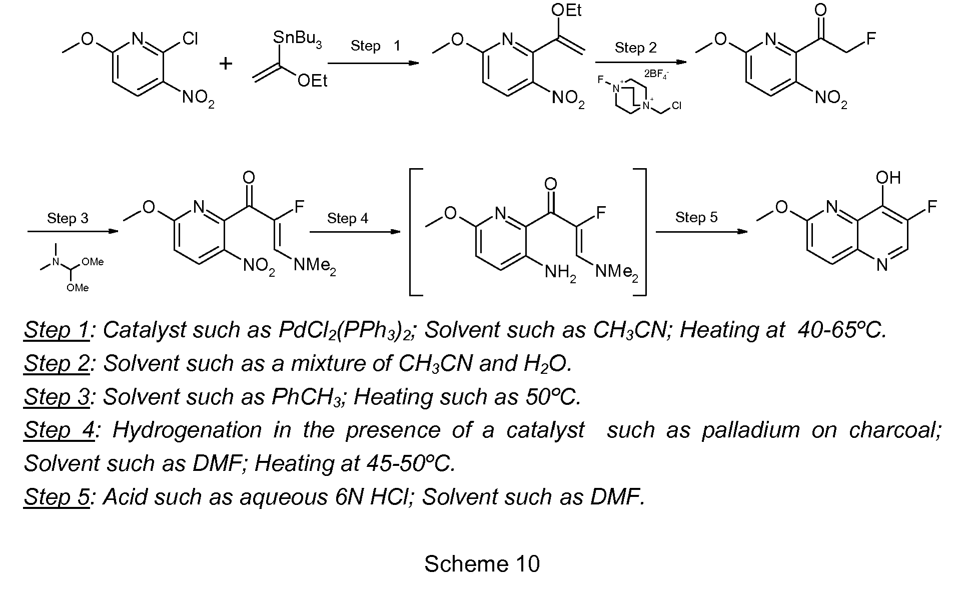

Compounds of Formula (X), wherein R¹ is C₁₋₃alkoxy-, may be prepared from compounds of Formula (X), wherein R¹ is halo, for example fluoro, according to Scheme 11. Compounds (X), wherein R¹ is fluoro, are treated with an appropriate base such as NaOC₁₋₃alkyl, for example NaOMe, in the appropriate alcohol solvent (C₁₋₃alkylOH), for example methanol, at elevated temperature, for example 40-65°C.

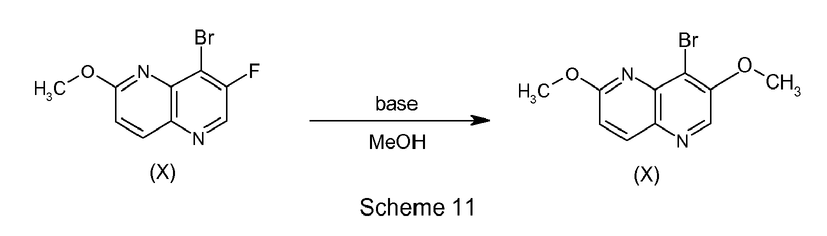

Those skilled in the art will appreciate that in the preparation of the compound of Formula (I), it may be necessary and/or desirable to protect one or more sensitive groups in the molecule or the appropriate intermediate to prevent undesirable side reactions. Suitable protecting groups for use according to the present invention are well known to those skilled in the art and may be used in a conventional manner. See, for example, "Protective groups in organic synthesis" by T.W. Greene and P.G.M. Wuts (John Wiley & sons 1991) or "Protecting Groups" by P.J. Kocienski (Georg Thieme Verlag 1994). Examples of suitable amino protecting groups include acyl type protecting groups (e.g. formyl, trifluoroacetyl, acetyl), aromatic urethane type protecting groups (e.g. benzyloxycarbonyl (Cbz) and substituted Cbz), aliphatic urethane protecting groups (e.g. 9-fluorenylmethoxycarbonyl (Fmoc), t-butyloxycarbonyl (Boc), isopropyloxycarbonyl, cyclohexyloxycarbonyl) and alkyl or aralkyl type protecting groups (e.g. benzyl, trityl, chlorotrityl). Examples of suitable oxygen protecting groups may include for example alky silyl groups, such as trimethylsilyl or tert-butyldimethylsilyl; alkyl ethers such as tetrahydropyranyl or tert-butyl; or esters such as acetate.

It will be readily apparent to those skilled in the art that other compounds of Formula (I) may be prepared using methods analogous to those outlined above, or by reference to the experimental procedures detailed in the Examples provided herein. Further details for the preparation of compounds of Formula (I) are found in the Examples.

### Compositions and formulations

The compounds of the invention may be formulated for administration in any convenient way for use in human or veterinary medicine, by analogy with formulation of antibacterials, or formulation of other antitubercular agents.

The compounds of the invention will normally, but not necessarily, be formulated into pharmaceutical compositions prior to administration to a patient. In one aspect, the invention is directed to a pharmaceutical composition comprising a compound of Formula (I), or a pharmaceutically acceptable salt, solvate or N-oxide thereof. In another aspect the invention is directed to a pharmaceutical composition comprising a compound of Formula (I), or a pharmaceutically acceptable salt, solvate or N-oxide thereof, and one or more pharmaceutically acceptable carriers, excipients or diluents. The carrier, excipient or diluent must be "acceptable" in the sense of being compatible with the other ingredients of the formulation and not deleterious to the recipient thereof.

The pharmaceutical compositions of the invention include those in a form adapted for oral, or parenteral use and may be used for the treatment of tuberculosis in mammals including humans.

The pharmaceutical compositions of the invention include those in a form adapted for oral, topical or parenteral use and may be used for the treatment of bacterial infections in mammals including humans.

The composition may be formulated for administration by any convenient route. For the treatment of tuberculosis, the compositions may be in the form of tablets, capsules, powders, granules, lozenges, aerosols or liquid preparations, such as oral or sterile parenteral solutions or suspensions. For the treatment of bacterial infections the compositions may be in the form of tablets, capsules, powders, granules, lozenges, creams, aerosols or liquid preparations, such as oral or sterile parenteral solutions or suspensions.

For the treatment of bacterial infections the topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

In one aspect of the invention, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-1000 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 30 mg/kg per day.

The compound of Formula (I), or a pharmaceutically acceptable pharmaceutically acceptable salt, solvate or N-oxide thereof, may be the sole therapeutic agent in the compositions of the invention, or it may be present in the formulation in combination with one or more additional therapeutic agents. The invention thus provides, in a further aspect, a combination comprising a compound of Formula (I), or a pharmaceutically acceptable salt, solvate or N-oxide thereof together with one or more additional therapeutic agents.

The one or more additional therapeutic agent is, for example, an agent useful for the treatment of tuberculosis in a mammal. Examples of such therapeutic agents include isoniazid, ethambutol, rifampin, pirazinamide, streptomycin, capreomycin, ciprofloxacin and clofazimine.

When a compound of Formula (I), or a pharmaceutically acceptable pharmaceutically acceptable salt, solvate or N-oxide thereof is used in combination with one or more additional therapeutic agents, the dose of the compound or agent may differ from that when the compound or agent is used alone. Appropriate doses will be readily appreciated by those skilled in the art. It will be appreciated that the amount of a compound of the invention and the one or more additional therapeutic agents required for use in treatment will vary with the nature of the condition being treated and the age and the condition of the patient and will be ultimately at the discretion of the attendant physician or veterinarian.

The combinations may conveniently be presented for use in the form of a pharmaceutical formulation. In a further aspect of the present invention there is provided a pharmaceutical combination comprising a compound of Formula (I), or a pharmaceutically acceptable salt, solvate or N-oxide thereof, together with one or more additional therapeutic agents, and one or more pharmaceutically acceptable carriers, excipients or diluents. The individual components of such combinations may be administered either sequentially or simultaneously in separate or combined pharmaceutical formulations by any convenient route.

When administration is sequential, either the compound of the present invention or one or more additional therapeutic agent may be administered first. When administration is simultaneous, the combination may be administered either in the same or different pharmaceutical composition. When combined in the same formulation it will be appreciated that the compound and agents must be stable and compatible with each other and the other components of the formulation. When formulated separately they may be provided in any convenient formulation, conveniently in such manner as are known for such compounds in the art.

### Abbreviations

In describing the invention, chemical elements are identified in accordance with the Periodic Table of the Elements. Abbreviations and symbols utilized herein are in accordance with the common usage of such abbreviations and symbols by those skilled in the chemical arts. The following abbreviations are used herein:
- EtOAc: ethyl acetate
- AcOH: acetic acid
- Ac20: acetic anhydride
- BOC: N-tert-butoxycarbonyl
- BOC anhydride: di-*tert*-butyl dicarbonate
- Celite®: a filter aid composed of acid-washed diatomaceous silica, (a trademark of Manville Corp., Denver, Colorado)
- DME: dimethoxyethane
- DCM: dichloromethane
- DIBAL-H: diisobutyl aluminium hydride
- DMF: dimethylformamide
- DMSO-d6: deuterated dimethylsulfoxide
- DMSO: dimethylsulfoxide
- ES MS: Electrospray mass spectrometry
- EtOH: ethanol
- h: hours
- HPLC: high performance liquid chromatography
- LCMS: Liquid chromatography mass spectroscopy
- MeOH: methanol
- NaBH(OAc)₃: sodium triacetoxyborohydride
- NMR: Nuclear Magnetic Resonance spectroscopy
- t-BuOMe: methyl t-butyl ether
- TFA: trifluoroacetic acid
- THF: tetrahydrofuran
- uv: ultraviolet

### Examples

The following Examples illustrate the invention. These Examples are not intended to limit the scope of the invention, but rather to provide guidance to the skilled artisan to prepare and use the compounds, compositions, and methods of the invention. While particular embodiments of the invention are described, the skilled artisan will appreciate that various changes and modifications can be made. References to preparations carried out in a similar manner to, or by the general method of, other preparations, may encompass variations in routine parameters such as time, temperature, workup conditions, minor changes in reagent amounts etc.

Proton nuclear magnetic resonance (¹H NMR) spectra were recorded, and chemical shifts are reported in parts per million (δ) downfield from the internal standard tetramethylsilane (TMS). Abbreviations for NMR data are as follows: s = singlet, d = doublet, t = triplet, q = quartet, m = multiplet, dd = doublet of doublets, dt = doublet of triplets, app = apparent, br = broad. Mass spectra were obtained using electrospray (ES) ionization techniques. All temperatures are reported in degrees centigrade.

Reactions involving metal hydrides including lithium hydride, lithium aluminium hydride, diisobutylaluminium hydride, sodium hydride, sodium borohydride and sodium triacetoxyborohydride are carried out under argon unless otherwise specified.

### Intermediates

### Intermediate 1

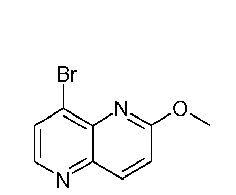

### 8-Bromo-2-(methyloxy)-1,5-naphthyridine

6-(Methyloxy)-1,5-naphthyridin-4-ol (21.5 g) (for a synthesis see W02007016610 Preparation 2 (a)) was stirred in DMF (150 ml) at 0°C under N₂, and phosphorous tribromide (13.5 ml) was added slowly. The mixture was allowed to warm to room temperature and stirred for 90 minutes. H₂O (375 ml) was added and the pH was adjusted to pH 7 by addition of solid Na₂CO₃. The solid was isolated by filtration with suction, dried on the sinter with suction for 2h then dried under vacuum at 45°C to give the desired compound (26.0 g, 90%). ¹H-NMR δ, ppm, DMSO-d₆): 8.59 (d, 1 H), 8.30 (d, 1 H), 8.08 (d, 1H), 7.33 (d, 1 H), 4.06 (s, 3H).

### Intermediate 2

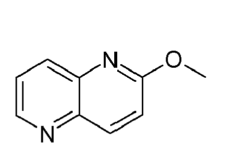

### 2-(Methyloxy)-1,5-naphthyridine

To a mixture of Intermediate 1 (25.5 g) in CH₂Cl₂ (200 ml) and EtOH (200 ml) was added NaHCO₃ (20 g) and 5% wet palladium on carbon (4 g). The resulting suspension was hydrogenated at 1.5 bar for 21 h. The mixture was filtered with suction through celite and the solids were washed with CH₂Cl₂/EtOH 1:1 (2000 ml). The combined filtrate plus washings were concentrated under reduced pressure and then treated with CH₂Cl₂/H₂O 2:1 (1600 ml). The organic phase was separated, dried over MgSO₄, filtered and evaporated under reduced pressure to give the desired compound (15.8 g, 92%). [ES MS] m/z 161 (MH⁺).

### Intermediate 3

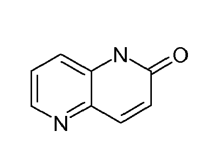

### 1,5-Naphthyridin-2(1H)-one

Intermediate 2 (15.8 g) was stirred in 6N HCl (100 ml) at 110°C for 2h. The mixture was cooled at 0°C and the pH was adjusted to 6-7 with solid NaOH. The precipitated solid was isolated by filtration with suction, dried on the sinter with suction for 2h, and dried in a vacuum at 45°C to give the desired (14.4 g, 98%). [ES MS] m/z 147 (MH⁺).

### Intermediate 4

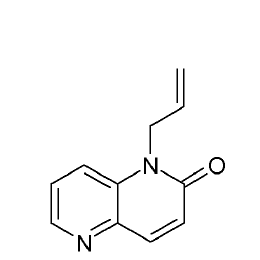

### 1-(2-Propen-1-yl)-1,5-naphthyridin-2(1H)-one

To a suspension of Intermediate 3 (5.9 g) in dry DME (180 ml) and dry DMF (45 ml) at 0°C under argon was added in portions NaH (60% w:w dispersion in mineral oil, 3.2 g). After stirring for 45 minutes, the mixture was treated with lithium bromide (8.8 g) and the suspension was allowed to warm to room temperature. After stirring for 45 minutes, the mixture was treated with allyl bromide (7 ml) and then stirred at 65°C for 3 h. The mixture was cooled to room temperature and concentrated under reduced pressure, then t-BuOMe (300 ml) was added and the mixture was then washed with 1 N NH₄Cl (200 ml). The combined aqueous phases were extracted with *t*-BuOMe (2 x 100 ml). The organic phases were combined, washed with brine (200 ml), dried over Na₂SO₄, filtered and evaporated under reduced pressure. The residue was purified by column chromatography on silica gel using a EtOAc and hexane gradient (50-75%) to give the desired product (4.29 g, 57%). To obtain an additional amount of the desired compound, the combined aqueous phases were extracted exhaustively with CH₂Cl₂. Then, the organic extracts were combined, dried over MgSO₄, filtered and evaporated under reduced pressure. The residue was purified by column chromatography on silica gel using a EtOAc and hexane gradient (50-75%) to give the desired product (1.5 g, 20%). [ES MS] m/z 187 (MH⁺).

### Intermediate 5

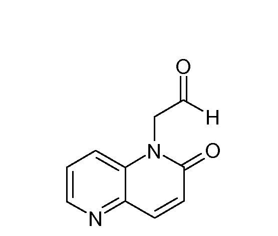

### (2-Oxo-1,5-naphthyridin-1(2H)-yl)acetaldehyde (mixture with the methyl hemiacetal)

To a solution of Intermediate 4 (5.2 g) in 1,4-dioxane (100 ml) and H₂O (50 ml) was added consecutively sodium periodate (13.8 g) and osmium tetroxide (4 g of Supported OsO₄). The mixture was stirred at room temperature for 24h. Additional amount of sodium periodate (1.4 g) and osmium tetroxide (500 mg) was added and the mixture was stirred another 72h. The mixture was filtered and the solid washed with H₂O (250 ml) and THF (125 ml). The combined filtrate plus washings were extracted with CH₂Cl₂/MeOH (525/125; 375/125 and 375/125 ml). The organic extracts were combined, dried over Na₂SO₄, filtered and evaporated under reduced pressure to give the desired product (5.4 g, 90%). ¹H-NMR (δ, ppm, CDCl₃): 9.75 (s, 1/2H), 8.59-8.55 (m, 1 H), 8.03-7.85 (m, 2H), 7.50-7.30 (m, 2H), 6.98 (d, 1 H), 5.15-4.25 (m, 5H), 3.70 (s, 3H), 3.49 (s, 1/2H), 3.42-3.30 (m, 1 H).

### Intermediate 6

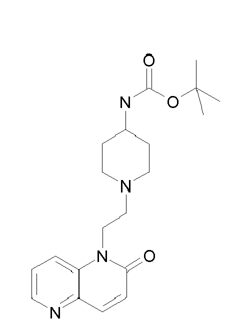

### 1,1-Dimethylethyl {1-[2-(2-oxo-1,5-naphthyridin-1(2H)-yl)ethyl]-4-piperidinyl}carbamate

To a mixture of Intermediate 5 (10.2 g) in CH₂Cl₂ (350 ml) and MeOH (20 ml) was added 1,1-dimethylethyl-4-piperidinylcarbamate (10.8 g, from Aldrich). After stirring for 1h, sodium triacetoxyborohydride (34.4 g) was added. The reaction was stirred for 3h before addition of H₂O (200 ml) and saturated NaHCO₃ (400 ml). The reaction was extracted with CH₂Cl₂/MeOH (500/75, 450/50 and 450/50 ml). The combined organic phases were dried over Na₂SO₄, evaporated and the residue was purified by chromatography on silica gel using a CH₂Cl₂ and MeOH gradient to provide the desired compound (12.7 g, 63%). [ES MS] m/z 373 (MH⁺).

### Intermediate 7

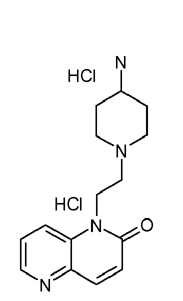

### 1-[2-(4-Amino-1-piperidinyl)ethyl]-1,5-naphthyridin-2(1H)-one dihydrochloride

To a solution of Intermediate 6 (7.2 g) in CH₂Cl₂ (90 ml) was added HCl (4M solution in 1,4-dioxan, 35 ml). After stirring for 20h, the obtained solid was filtered, washed with CH₂Cl₂ and dried to give 6.8 g of the title compound. ¹H-NMR (δ, ppm, D₂O): 8.52 (d, 1 H), 8.22 (d, 1 H), 7.99 (d, 1 H), 7.82-7.78 (m,1 H), 6.99 (d, 1 H), 4,66-4.60 (m), 3.79-3.72 (m, 2H), 3.48-3.42 (m, 3H), 3.14-3.03 (m, 2H), 2.23-2.15 (m, 2H), 1.87-1.76 (m, 2H).

### Intermediate 8

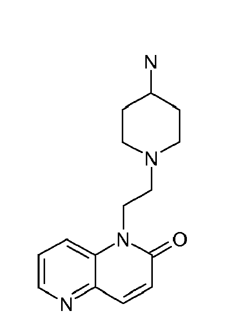

### 1-[2-(4-Amino-1-piperidinyl)ethyl]-1,5-naphthyridin-2(1H)-one

To a solution of Intermediate 7 (2 g) in H₂O (20 ml) at 0°C was added aqueous 1 N NaOH until pH 11. The reaction was then extracted with CH₂Cl₂/MeOH 95:5 (50 ml). The organic phase was dried over Na₂SO₄ and evaporated to give the desired compound (387 mg). The aqueous phase was evaporated and the residue was treated with CH₂Cl₂ (25 ml) and the mixture was stirred for 1 h. Then it was filtered and the solvent was evaporated to give an additional amount of the title compound (1.0 g). ¹H-NMR (δ, ppm, CDCl₃): 8.55 (d, 1 H), 7.90 (d, 1 H), 7.77 (d, 1 H), 7.48-7.44 (m, 1 H), 6.91 (d, 1 H), 4.38 (t, 2H), 2.99-2.94 (m, 2H), 2.73-2.62 (m, 3H), 2.18 (dt, 2H), 1.87-1.76 (m, 2H); 1.43-1.30 (m, 2H), 1.25-1.13 (m, 2H).

### Intermediate 9

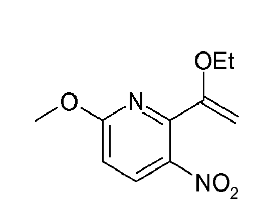

### 2-[1-(Ethyloxy)ethenyl]-6-(methyloxy)-3-nitropyridine

To a suspension of 2-chloro-6-methoxy-3-nitropyridine (600 g) and PdCl₂(PPh₃)₂ (33.5 g) in acetonitrile (4200 ml) at 65°C under N₂ was added dropwise over 2h (1-ethoxyvinyl)-tributyl-stannane (1182 ml). The resulting suspension was stirred at 65 °C for 4h then left to cool to room temperature overnight. The reaction mixture was quenched with 10% KF aqueous solution (3600 ml) with vigorous stirring and stirred for 1 h. The resulting solid was removed by vacuum filtration and washed with acetonitrile (7 x 1000 ml). The layers were separated and the organic layer was evaporated to 3000 ml. This was filtered through Whatman, GF/B glass microfibre filter paper and the small amount of brown solid removed was washed with MeCN (1800 ml). EtOAc (3600 ml) was added and the volume reduced to 1800 ml. Cyclohexane (3600 ml) was added and the volume reduced to 3000 ml. Cyclohexane (2400 ml) and silica gel (600 g, 1 wt) were added and allowed to stir at room temperature for 1.5h. The solid was removed by vacuum filtration and washed with EtOAc/cyclohexane, 1:8 (4200 ml). The filtrate was reduced to 1800 ml. Cyclohexane (2400 ml) was added and the volume reduced to 1800 ml. Cyclohexane (3600 ml) and EtOAc (600 ml) and silica gel (600 g, 1 wt) were added and stirred for 1.5h. The solid was removed by vacuum filtration and washed with EtOAc/cyclohexane 1:8 (4200 ml). The solvents were evaporated to dryness. MeCN (2000 ml) was added and evaporated to give an orange coloured oil.

### Intermediate 10

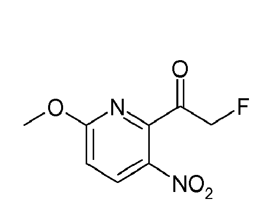

### 2-Fluoro-1-[6-(methyloxy)-3-nitro-2-pyridinyl]ethanone

To a suspension of Selectfluor (1286.4 g) in acetonitrile (2060 ml) and H₂O (820 ml) was added dropwise over 1.5h Intermediate 9 in acetonitrile (1416 ml), maintaining the temperature <15°C using an ice/water bath. The resulting solution was stirred at room temperature overnight. The reaction mixture was quenched with saturated aqueous NaHCO₃ (2140 ml) and stirred for 30 minutes. The volume was reduced by rotary evaporation to 3250 ml. To the resulting yellow suspension was added EtOAc (4400 ml) and H₂O (720 ml) and allowed to stir for 15 minutes. The layers were separated and the aqueous extracted with EtOAc (2 x 1000 ml). The organic layers were combined and washed with H₂O (1000 ml) and saturated NaCl (1000 ml). The organic layer was dried over MgSO₄ (400 g), filtered and evaporated. Acetonitrile (1000 ml) was added and evaporated to give an orange oil which slowly solidified on standing.

### Intermediate 11

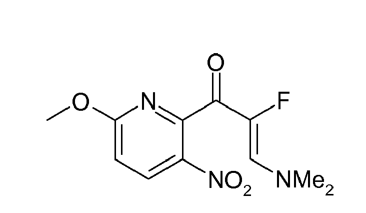

### (2Z)-3-(Dimethylamino)-2-fluoro-1-[6-(methyloxy)-3-nitro-2-pyridinyl]-2-propen-1-one

To a solution of Intermediate 10 (657.0 g) in toluene (2700 ml) under N₂ was added *N*,*N-*dimethylformamide dimethylacetal (1550 ml). The reaction mixture was heated at 50 °C for 4h. Cyclohexane (2000 ml) was added and the reaction mixture was cooled slowly over 1 h, then to <5°C using an ice/water bath. The precipitated solid was collected by vacuum filtration and washed with EtOAc/cyclohexane, 1:1 (3 x 1000 ml). The yellow solid was dried in the oven, under vacuum at 40°C overnight.

### Intermediate 12

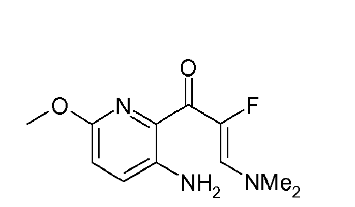

### (2Z)-1-[3-Amino-6-(methyloxy)-2-pyridinyl]-3-(dimethylamino)-2-fluoro-2-propen-1-one

To a mixture of Intermediate 11 (1146.7 g) in DMF (10500 ml) was added 5% wet palladium on carbon (274.4 g) in DMF (1000 ml). The resulting suspension was hydrogenated at 1.0 bar for 3h, maintaining the temperature between 45-50 °C. The reaction mixture was warmed to 60 °C. DMF (1800 ml) was warmed to 50 °C and charged to the pressurised filter. The hot reaction mixture was nitrogen transferred through the pressurised filter, at 1.0 bar, to remove the catalyst. The vessel was rinsed out with hot DMF (2 x 1500 ml). The product was not isolated and used directly in the next step.

### Intermediate 13

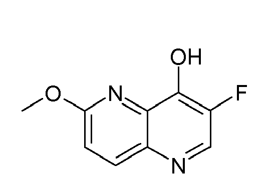

### 3-Fluoro-6-(methyloxy)-1,5-naphthyridin-4-ol

To a solution of Intermediate 12 in DMF at 0°C was added dropwise aqueous 6N HCl (184 ml). The reaction was allowed to warm to room temperature and stirred overnight. The volume of the reaction mixture was reduced to -2000 ml by rotary evaporation at 50 °C and the yellow suspension was cooled to 10 °C using an ice/water bath. H₂O (4000 ml) was added slowly over 30 minutes. The reaction mixture was stirred vigorously for 1 h. The precipitated solid was collected by vacuum filtration and washed with H₂O (3000 ml) then EtOAc/cyclohexane, 1:1 (3 x 2000 ml). The pale brown solid was dried in the oven, under vacuum at 50 °C for 4 days.

### Intermediate 14

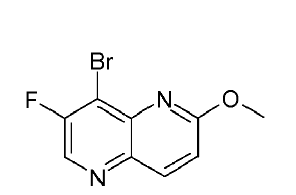

### 8-Bromo-7-fluoro-2-(methyloxy)-1,5-naphthyridine

This was prepared from Intermediate 13 (15.8 g) using a procedure analogous to that described for Intermediate 1 to give 19.6 g (93%) of the title compound. ¹H-NMR (δ, ppm, DMSO-d₆): 8.86 (s, 1 H), 8.34 (d, 1 H), 7.31 (d, 1 H), 4.08 (s, 3H).

### Intermediate 15

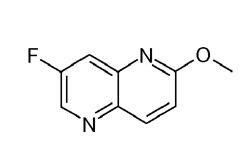

### 7-Fluoro-2-(methyloxy)-1,5-naphthyridine

This was prepared from Intermediate 14 (12.7 g) using a procedure analogous to that procedure described for Intermediate 2 to give 8.3 g (94%) of the title compound. ¹H-NMR (δ, ppm, CDCl₃): 8.67 (d, 1 H), 8.18 (d, 1 H), 7.81-7.76 (m, 1 H), 7.07 (d, 1 H), 4.08 (s, 3H).

### Intermediate 16

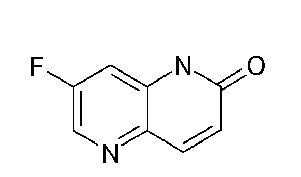

### 7-Fluoro-1,5-naphthyridin-2(1H)-one

This was prepared from Intermediate 15 (6.4 g) using a procedure analogous to that described for Intermediate 3 to give 5.7 g (95%) of the title compound. [ES MS] m/z 279 (MH⁺).

### Intermediate 17

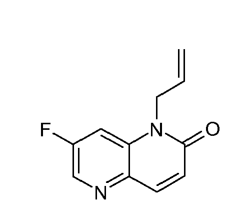

### 7-Fluoro-1-(2-propen-1-yl)-1,5-naphthyridin-2(1H)-one

This was prepared from Intermediate 16 (5.0 g) using a procedure analogous to that described for Intermediate 4 to give 5.4 g (88%) of the title compound. [ES MS] m/z 205 (MH⁺).

### Intermediate 18

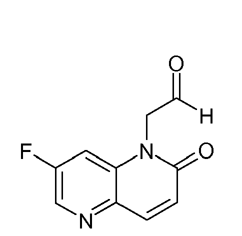

### 7-Fluoro-(2-oxo-1,5-naphthyridin-1(2H)-yl)acetaldehyde (mixture with the methyl hemiacetal)

This was prepared from Intermediate 17 (5.4 g) using a procedure analogous to that described for Intermediate 5 to give 5.8 g of the title compound. ¹H-NMR (δ, ppm, CDCl₃): 9.77 (s, 1/2H), 8.45 (bs, 1 H), 7.97 (d, 1 H), 7.59 (d, 1/2H), 7.06 (d, 1 H), 6.92 (d, 1 H), 5.05-4.95 (m, 2H), 4.45-4.30 (m, 1 H), 3.49 (s, 5/2H), 3.42 (s, 5/2H).

### Intermediate 19

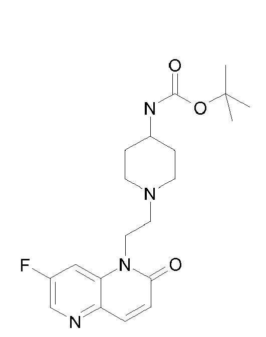

### 1,1-Dimethylethyl {1-[2-(7-fluoro-2-oxo-1,5-naphthyridin-1(2H)-yl)ethyl]-4-piperidinyl}carbamate

This was prepared from Intermediate 18 (5.8 g) using a procedure analogous to that described for Intermediate 6 to give 7.2 g (66%) of the title compound. [ES MS] m/z 391 (MH⁺).

### Intermediate 20

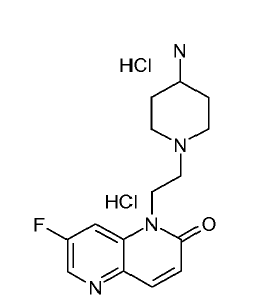

### 1-[2-(4-amino-1-piperidinyl)ethyl]-7-fluoro-1,5-naphthyridin-2(1H)-one dihydrochloride

This was prepared from Intermediate 19 (12.7 g) using a procedure analogous to that described for Intermediate 7 to give 13.5 g of the title compound. ¹H-NMR (δ, ppm, D₂O): 8.41 (s, 1H), 7.94 (d, 1H), 7.80 (d, 1H), 6.83 (d, 1H), 4.70-4.55 (m), 3.85-3.75 (m, 2H), 3.46 (t, 2H), 3.15-3.05 (m, 2H), 2.25 (bd, 2H), 1.90-1.75 (m, 2H).

### Intermediate 21

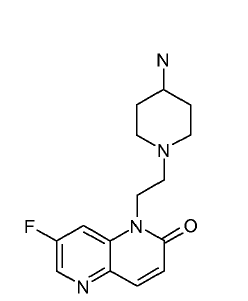

### 1-[2-(4-Amino-1-piperidinyl)ethyl]-7-fluoro-1,5-naphthyridin-2(1H)-one

To a solution of Intermediate 20 (13.5 g) in H₂O (110 ml) at 0°C was added aqueous 2N NaOH until pH 11. The reaction was then extracted with CH₂Cl₂/MeOH 95:5 (120 ml). The organic phase was dried over Na₂SO₄ and evaporated to give the desired compound (760 mg). The aqueous phase was evaporated and the residue was treated with CH₂Cl₂/MeOH 95:5 (120 ml) and the mixture was stirred for 1 h. Then it was filtered and the solvent was evaporated to give an additional amount of the title compound (8.0 g). ¹H-NMR (δ, ppm, DMSO-d₆): 8.54 (d, 1 H), 8.01 (d, 1 H), 7.92 (d, 1 H), 4.29 (t, 2H), 3.65-3.15 (m, 4H), 3.35 (d, 2H), 2.80-2.70 (m, 1 H), 2.53-2.48 (m), 2.01 (t, 2H), 1.72 (d, 2H), 1.35-1.20 (m, 2H).

### Intermediate 22

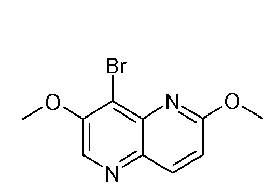

### 8-Bromo-2,7-bis(methyloxy)-1,5-naphthyridine

To a solution of Intermediate 14 (62.5 g) in dry MeOH (600 ml) under N₂ was added NaMeO (25%wt solution in MeOH, 525 ml). The mixture was stirred at 60°C for 2h and then, was cooled to room temperature. Brine (800 ml), H₂O (800 ml) and CH₂Cl₂ (1 L) were added. The mixture was stirred and filtered with suction. The organic phase was separated and the aqueous phase was extracted with more CH₂Cl₂ (2 x 500 ml). The combined organic phases were dried over Na₂SO₄ and concentrated under reduced pressure to give the desired compound (60.1 g, 92%). ¹H-NMR (δ, ppm, CDCl₃): 8.54 (s, 1 H), 8.15 (d, 1 H), 7.03 (d, 1 H), 4.15, 4.16 (s, 6H).

### Intermediate 23

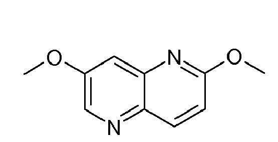

### 2,7-Bis(methyloxy)-1,5-naphthyridine

This was prepared from Intermediate 22 (15.0 g) using a procedure analogous to that described for Intermediate 2 to give 10.4 g (98%) of the title compound. ¹H-NMR (δ, ppm, CDCl₃): 8.52 (d, 1 H), 8.14 (d, 1 H), 7.46 (d, 1 H), 6.96 (d, 1 H), 4.07 (s, 3H), 3.96 (s, 3H).

### Intermediate 24

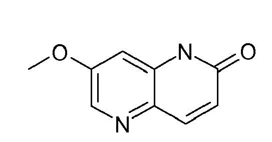

### 7-(Methyloxy)-1,5-naphthyridin-2(1H)-one

This was prepared from Intermediate 23 (39.7 g) using a procedure analogous to that described for Intermediate 3 to give 36.2 (98%) of the title compound. ¹H-NMR (δ, ppm, DMSO-d₆): 11.78 (bs, 1H), 8.19 (d, 1H), 7.84 (d, 1H), 7.12 (d, 1H), 6.52 (d, 1H), 3.86 (s, 3H).

### Intermediate 25

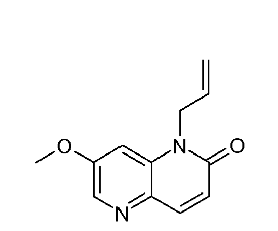

### 7-Methyloxy-1-(2-propen-1-yl)-1,5-naphthyridin-2(1H)-one

This was prepared from Intermediate 24 (15.0 g) using a procedure analogous to that described for Intermediate 4 to give 14.5 g (79%) of the title compound. ¹H-NMR (δ, ppm, CDCl₃): 8.27 (d, 1 H), 7.87 (dd, 1 H), 7.02 (d, 1 H), 6.78 (d, 1 H), 6.00-5.88 (m, 1 H), 5.26 (d, 1 H), 5.10 (d, 1 H), 4.92-4.89 (m, 2H), 3.94 (s, 3H).

### Intermediate 26

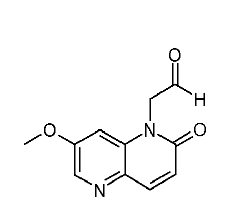

### [7-(Methyloxy)-2-oxo-1,5-naphthyridin-1(2H)-yl]acetaldehyde

To a solution of Intermediate 25 (15.4 g) in 1,4-dioxane (250 ml) and H₂O (120 ml) was added consecutively sodium periodate (35 g) and osmium tetroxide (11.3 g of Supported OsO₄). The mixture was stirred at room temperature for 24h. Additional amount of sodium periodate (3.5 g) and osmium tetroxide (1.2 g) was added and the mixture was stirred another 72h. The mixture was filtered and the filtrate was extracted with CH₂Cl₂/MeOH 9:1 (300, 200 and 100 ml). The organic phases were combined, dried over Na₂SO₄, filtered and evaporated under reduced pressure to give the desired product (14.9 g, 95%). ¹H-NMR (δ, ppm, CDCl₃): 9.72 (s, 1 H), 8.30 (d, 1 H), 7.94 (d, 1 H), 6.81 (d, 1 H), 6.72 (bd, 1 H), 5.11 (s, 2H), 3.93 (s, 3H).

### Intermediate 27

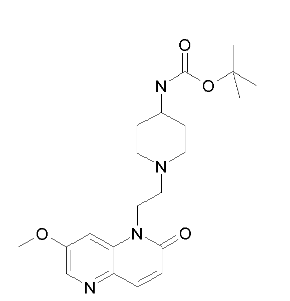

### 1,1-Dimethylethyl (1-{2-[7-(methyloxy)-2-oxo-1,5-naphthyridin-1(2H)-yl]ethyl}-4-piperidinyl)carbamate

This was prepared from Intermediate 26 (12.8 g) using a procedure analogous to that described for Intermediate 6 to give 15.3 g (65%) of the title compound. ¹H-NMR (δ, ppm, CDCl₃): 8.27 (d, 1 H), 7.83 (d, 1 H), 7.17 (bs, 1 H), 6.73 (d, 1 H), 4.44 (bs, 1 H), 4.35 (t, 2H), 3.97 (s, 3H), 3.47 (bs, 1 H), 2.94 (bd, 2H), 2.64 (t, 2H), 2.26 (t, 2H), 1.95 (bd, 2H), 1.70 (bs, 2H), 1.44 (s, 9H).

### Intermediate 28

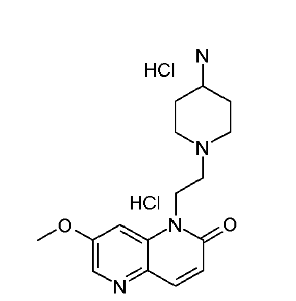

### 1-[2-(4-amino-1-piperidinyl)ethyl]-7-(methyloxy)-1,5-naphthyridin-2(1H)-one dihydrochloride

This was prepared from Intermediate 27 (15.4 g) using a procedure analogous to that described for Intermediate 7.

### Intermediate 29

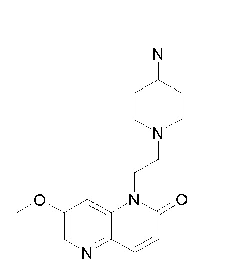

### 1-[2-(4-Amino-1-piperidinyl)ethyl]-7-methyloxy-1,5-naphthyridin-2(1H)-one as free base and dihydrochloride

This was prepared from Intermediate 28 by the procedure described in Intermediate 21 to give 11.5 g (99%) of the title compound. ¹H-NMR (δ, ppm, DMSO-d₆): 8.26 (d, 1 H), 7.85 (d, 1 H), 7.39 (d, 1 H), 6.64 (d, 1 H), 4.34 (d, 2H), 3.97 (s, 3H), 3.72 (bs, 2H), 2.92 (bd, 2H), 2.74-2.64 (m, 1 H), 2.54-2.48 (m), 2.03 (t, 2H), 1.72 (bd, 2H), 1.35-1.24 (m, 2H).

### Intermediate 30

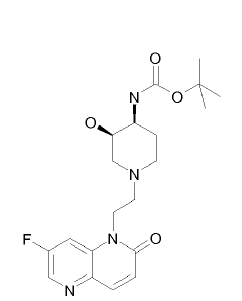

### 1,1-Dimethylethyl {(3R,4S)-1-[2-(7-fluoro-2-oxo-1,5-naphthyridin-1(2H)-yl)ethyl]-3-hydroxy-4-piperidinyl}carbamate

Intermediate 18 (200 mg) and 1,1-dimethylethyl[(3*R*,4*S*)-3-hydroxy-4-piperidinyl]carbamate (182 mg) (for a synthesis see W02004058144, Example 5(c), cis-(3-hydroxy-piperidin-4-yl)-carbamic acid *tert*-butyl ester Enantiomer 1) were stirred in CHCl₃ (10 ml) plus MeOH (0.5 ml) under argon for 2h. Sodium triacetoxyborohydride (534 mg) was added in one portion and the mixture was stirred at room temperature overnight, then quenched by addition of saturated aqueous NaHCO₃ (20 ml) and extracted with 20% v:v MeOH in CH₂Cl₂ (3 x 200 ml). The organic extracts were combined, dried over anhydrous MgSO₄, filtered and evaporated under reduced pressure to give the crude product, which was purified by column chromatography on silica, eluted with 0-20% (2M ammonia in MeOH) in CH₂Cl₂. Appropriate fractions were combined and evaporated under reduced pressure to give title compound (247 mg) as an off-white foam. [ES MS] m/z 407 (MH⁺).

### Intermediate 31

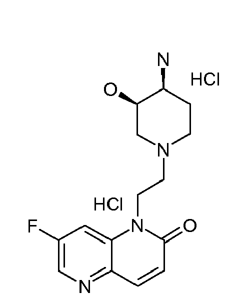

### 1-{2-[(3R,4S)-4-Amino-3-hydroxy-1-piperidinyl]ethyl}-7-fluoro-1,5-naphthyridin-2(1H)-one dihydrochloride

Intermediate 30 (240 mg) was dissolved in CH₂Cl₂ (10 ml) and the solution was treated with 4M HCl in 1,4-dioxane (2 ml). Effervescence and formation of a precipitate was observed. After 2h, the solvents were removed under reduced pressure and the residue was dried under reduced pressure overnight, to give 220 mg of the title compound as an off-white solid. [ES MS] m/z 307 (MH⁺).

### Intermediate 32

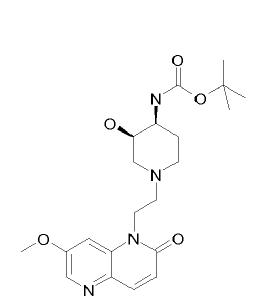

### 1,1-Dimethylethyl ((3R,4S)-3-hydroxy-1-{2-[7-(methyloxy)-2-oxo-1,5-naphthyridin-1(2H)-yl]ethyl}-4-piperidinyl)carbamate

This was prepared from Intermediate 26 (200 mg) and 1,1-dimethylethyl[(3R,4S)-3-hydroxy-4-piperidinyl] carbamate (173 mg) (for a synthesis see W02004058144, Example 5(c), cis-(3-hydroxy-piperidin-4-yl)-carbamic acid tert-butyl ester Enantiomer 1) using a procedure analogous to that described for Intermediate 30 to give 263 mg of the title compound. [ES MS] m/z 419 (MH⁺).

### Intermediate 33

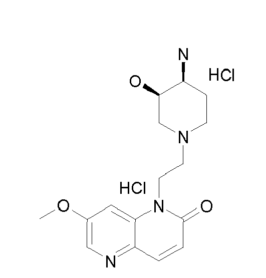

### 1-{2-[(3R,4S)-4-Amino-3-hydroxy-1-piperidinyl]ethyl}-7-(methyloxy)-1,5-naphthyridin-2(1H)-one dihydrochloride

This was prepared from Intermediate 32 (258 mg) using a procedure analogous to that described for Intermediate 31 to give 223 mg of the title compound.
[ES MS] m/z 319 (MH⁺).

### Intermediate 34

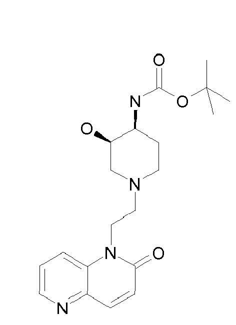

### 1,1-Dimethylethyl {(3R,4S)-3-hydroxy-1-[2-(2-oxo-1,5-naphthyridin-1(2H)-yl)ethyl]-4-piperidinyl}carbamate

This was prepared from Intermediate 5 (639 mg) and 1,1-dimethylethyl[(3R,4S)-3-hydroxy-4-piperidinyl] carbamate (250 mg) (for a synthesis see W02004058144, Example 5(c), cis-(3-hydroxy-piperidin-4-yl)-carbamic acid tert-butyl ester Enantiomer 1) using a procedure analogous to that described for Intermediate 30 to give 250 mg of the title compound.

### Intermediate 35

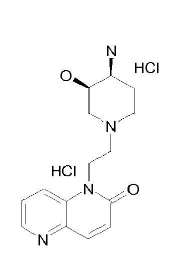

### 1-{2-[(3R,4S)-4-Amino-3-hydroxy-1-piperidinyl]ethyl}-1,5-naphthyridin-2(1H)-one dihydrochloride

This was prepared from Intermediate 34 (250 mg) using a procedure analogous to that described for Intermediate 31 to give 250 mg of the title compound.

### Intermediate 35b

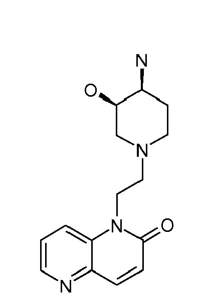

### 1-{2-[(3R,4S)-4-Amino-3-hydroxy-1-piperidinyl]ethyl}-1,5-naphthyridin-2(1H)-one

To a solution of Intermediate 35 in H₂O was added solid NaHCO₃ until pH 9. The reaction was then extracted with CH₂Cl₂/MeOH 9:1. The organic phase was dried over Na₂SO₄ and evaporated to give 28 mg of a yellow solid, which ¹H-NMR was not consistent with the desired compound. The aqueous phase was evaporated and the residue was treated with CH₂Cl₂ and the mixture was stirred at room temperature. Then it was filtered and the solvent was evaporated to provide the title compound (free base) (45 mg). ¹H-NMR (δ, ppm,CDCl₃): 8.56 (d, 1 H), 7.92 (d, 1 H), 7.71 (d, 1 H), 7.47 (dd, 1 H), 6.93 (d, 1 H), 4.53-4.44 (m, 1 H), 4.34-4.25 (m, 1 H), 3.66 (bs, 1 H), 3.09-3.06 (m, 1 H), 2.85-2.81 (m, 1 H), 2.72-2.65 (m, 3H), 2.34 (d, 1 H), 2.26-2.17 (m, 1 H), 1.64-1.57 (m, 5H).

### Intermediate 36

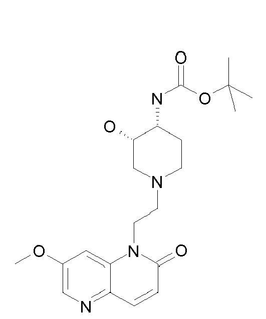

### 1,1-Dimethylethyl ((3S,4R)-3-hydroxy-1-{2-[7-(methyloxy)-2-oxo-1,5-naphthyridin-1(2H)-yl]ethyl}-4-piperidinyl)carbamate

This was prepared from Intermediate 26 (200 mg) and 1,1-dimethylethyl[(3S,4R)-3-hydroxy-4-piperidinyl] carbamate (182 mg) (for a synthesis see W02004058144, Example 5(c), cis-(3-hydroxy-piperidin-4-yl)-carbamic acid tert-butyl ester Enantiomer 2) using a procedure analogous to that described for Intermediate 30 to give 226 mg of the title compound. [ES MS] m/z 419 (MH⁺).

### Intermediate 37

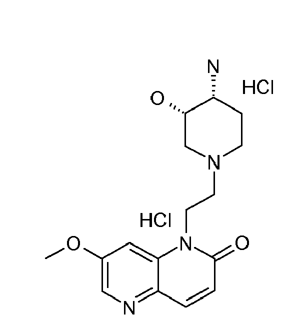

### 1-{2-[(3S,4R)-4-Amino-3-hydroxy-1-piperidinyl]ethyl}-7-(methyloxy)-1,5-naphthyridin-2(1H)-one dihydrochloride

This was prepared from Intermediate 36 (223 mg) using a procedure analogous to that described for Intermediate 31 to give 209 mg of the title compound. [ES MS] m/z 319 (MH⁺).

### Intermediate 38

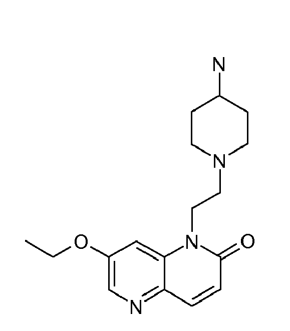

### 1-[2-(4-Amino-1-piperidinyl)ethyl]-7-(ethyloxy)-1,5-naphthyridin-2(1H)-one

Sodium hydride (124 mg) was added to a mixture of 1,4-dioxane (2 ml) and EtOH (2ml) and it was stirred at room temperature for 5 minutes. Intermediate 21 (300 mg) was added and the mixture was microwave-heated to 160°C for 10 minutes. H₂O was added and it was acidified with 2N HCl. The aqueous phase was washed with CH₂Cl₂/MeOH 10%, pH was adjusted to 11 with aqueous 2N NaOH. The aqueous layer was extracted with a mixture of CH₂Cl₂/MeOH 9:1, dried over Na₂SO₄ and concentrated under vacuum to give 255 mg of the title compound as a yellow oil. ¹H-NMR (δ, ppm, DMSO-*d₆*): 8.25 (d, 1 H), 7.84 (d, 1 H), 7.38 (d, 1 H), 6.63 (d, 1 H), 4.34-4.22 (m, 4H), 2.88-2.84 (m, 2H), 2.05-1.97 (m, 2H), 1.65-1.61 (m, 2H), 1.40 (t, 3H), 1.21-1.09 (m, 2H). [ES MS] m/z 317 (MH⁺).

### Intermediate 39

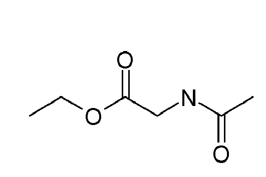

### Ethyl N-acetylglycinate

To a solution of N-acetylglycine (3 g) in EtOH (40 ml) under Ar was added p-toluenesulfonic acid (441 mg). The mixture was heated at 90°C overnight. The cooled mixture was concentrated under vacuum and the residue was partitioned between CH₂Cl₂ and saturated NaHCO₃. The organic phase was concentrated under vacuum to give 2.5 g (69%) of the title compound. ¹H-NMR (δ, ppm, CDCl₃): 6.00 (bs, 1 H), 4.23 (q, 2H), 4.03 (d, 2H), 2.05 (s, 3H), 1.30 (t, 3H).

### Intermediate 40

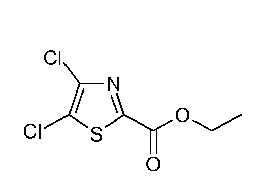

### Ethyl 4,5-dichloro-1,3-thiazole-2-carboxylate

A solution of Intermediate 39 (1.2 g) and thionyl chloride (3.2 ml) in anhydrous benzene (10 ml) was heated under reflux for 1 h. The cooled mixture was concentrated under vacuum. The residue obtained was purified by column chromatography on silica gel using hexane/EtOAc 9:1 as eluent to give 500 mg (26%) of the title compound.
¹H-NMR (δ, ppm, CDCl₃): 4.48 (q, 2H), 1.43 (t, 3H). [ES MS] m/z 226 (MH+).

### Intermediate 41

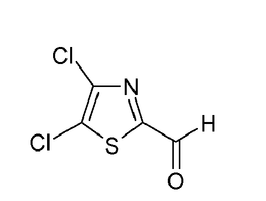

### 4,5-Dichloro-1,3-thiazole-2-carbaldehyde

To a solution of Intermediate 40 (500 mg) in CH₂Cl₂ (7 ml) at -78°C under N₂ was added dropwise di-isobutyl aluminium hydride (1.5 M solution in toluene, 1.6 ml). The solution was stirred at -78°C for 45 minutes. Then, more DIBAL-H (1.5 M solution in toluene, 1.0 ml) was added. The reaction was stirred at -78°C for 30 minutes. To this solution was added slowly a mixture of MeOH/acetic acid 2:1 (6 ml) followed by H₂O/CH₂Cl₂. The combined organic phases were washed with brine, and concentrated in vacuo to give 76.3 mg of the title compound.
¹H-NMR (δ, ppm, CDCl₃): 9.73 (s, 1 H).

### Intermediate 42

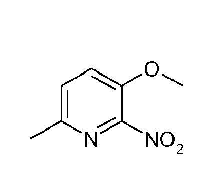

### 6-Methyl-3-(methyloxy)-2-nitropyridine

To a solution of 6-methyl-2-nitro-3-pyridinol (2.5 g) in DMF (40 ml) under N₂ was added a solution of NaH (60% w:w dispersion in mineral oil, 714 mg) in DMF (10 ml). The reaction was heated under reflux for 1.5h and then cooled to room temperature. To this solution iodomethane (1.05 ml) was added. The mixture was stirred at room temperature overnight. Then, more iodomethane (0.28 ml) was added. After stirring for 1.5h, isopropanol (10 ml) was added and the mixture was stirred for 30 minutes. The mixture was concentrated under vacuum and the residue was partitioned between H₂O and EtOAc. The combined organic phases were dried over Na₂SO₄, filtered, and concentrated under vacuum. The residue obtained was purified by column chromatography on silica gel using hexane/EtOAc as eluent to give the title compound together with starting material. The obtained solid was partitioned between CH₂Cl₂/Na₂CO₃ 9:1. The combined organic phases were dried over Na₂SO₄, filtered, and concentrated under vacuum to give 2.3 g of the title compound as a yellow solid. ¹H-NMR (δ, ppm, CDCl₃): 7.39 (q, 2H), 3.94 (s, 3H), 2.53 (s, 3H).

### Intermediate 43

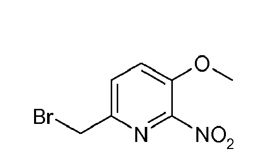

### 6-(Bromomethyl)-3-(methyloxy)-2-nitropyridine

To a solution of Intermediate 42 (500 mg) in CCl₄ (10 ml) was added N-bromosuccinimide (525 mg) and benzoyl peroxide (35.8 mg). The mixture was heated under reflux overnight. Then, more benzoyl peroxide (35.8 mg) was added. After refluxing for 24h, the solvent was evaporated under vacuum and the residue obtained was purified by column chromatography on silica gel using hexane/EtOAc as eluent to give 109 mg of the title compound. ¹H-NMR (δ, ppm, CDCl₃): 7.70 (d, 1 H), 7.51 (d, 1 H), 4.52 (s, 2H), 3.98 (s, 3H).

### Intermediate 44

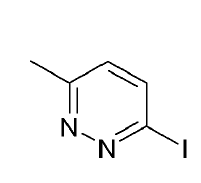

### 3-lodo-6-methylpyridazine

To a solution of 3-chloro-6-methylpyridazine (500 mg) in HI (3 ml) was added Nal (782 mg). The reaction was heated at 40 °C for 4 hours and at 70 °C overnight. The yellow precipitate was filtered. The mother liquors were basified using solid NaOH, extracted with CH₂Cl₂, dried over MgSO₄, filtered, concentrated, and purified by chromatography on silica gel using EtOAc/hexane as eluent to give 171.7 mg (20%) of the title compound as a white solid almost pure by HPLC. The yellow precipitate was basified using the same aqueous phase, extracted with CH₂Cl₂, dried over MgSO₄, filtered and concentrated, to give 579.1 mg (68%) of the pure title compound as a white solid. ¹H-NMR (δ, ppm, CDCl₃): 7.74 (1 H, d), 7.02 (1 H, d), 2.66 (3H, s). [ES MS] m/z 221 (MH⁺).

### Intermediate 45

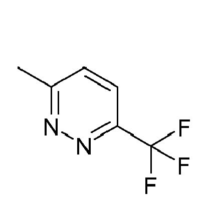

### 3-Methyl-6-(trifluoromethyl)pyridazine

A mixture of Cul (263 mg) and KF (80 mg) was heated under vacuum for 30 minutes until a greenish colour was observed. The system was filled with N₂ and a solution of Intermediate 44 (300 mg) in DMF (1.25 ml) and N-methylpirrolidinone (1.25 ml) followed by trimethyl(trifluoromethyl)silane (185 µl) were subsequently added. A dark brown colour was observed. After stirring at room temperature for 5 days, the reaction was not complete. Cul (263 mg), KF (80 mg), and CF₃TMS (185 µl) were added and stirring was continued overnight. Additional Cul (135 mg), KF (40 mg), and CF₃TMS (90 µl) were added and after one more day at room temperature, almost no starting material was observed. Aqueous ammonia and *t*-BuOMe were added and the phases were separated. The aqueous one was washed with *t*-BuOMe. The organic extracts were combined and subsequently washed with aqueous ammonia, 1 M HCl, saturated NaHCO₃, and brine, dried over Na₂SO₄, filtered and concentrated to give 70 mg (35%) of the title compound. ¹H-NMR (δ, ppm, CDCl₃): 7.72 (1H, d), 7.53 (1H, d), 2.85 (3H, s). [ES MS] m/z 163 (MH⁺).

### Intermediate 46

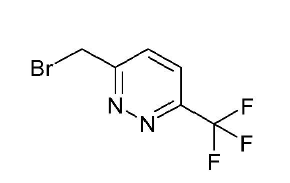

### 3-(Bromomethyl)-6-(trifluoromethyl)pyridazine

A mixture of Intermediate 45 (70 mg), N-bromosuccinimide (84 mg), azobisisobutyronitrile (14.1 mg), and CCl₄ (3 ml) was heated under reflux for 2 days. The solvent was evaporated to dryness under vacuum. The residue obtained was purified by chromatography column on silica gel (AIT, Flashsmart BP-SUP, 20-40µm) using a mixture of EtOAc/hexane as eluent to give 14 mg (14%) of the title compound along with 15.3 mg (11 %) of the dibrominated derivative. ¹H-NMR (δ, ppm, CDCl₃): 7.90 (1 H, d), 7.86 (1 H, d), 4.83 (2H, s). [ES MS] m/z 241 and 243 (MH⁺).

### Intermediate 47

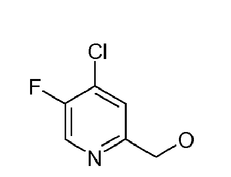

### (4-Chloro-5-fluoro-2-pyridinyl)methanol

4-Chloro-5-fluoro-2-methylpyridine 1-oxide (840 mg) (for a synthesis see *J*. *Med. Chem*., **1989,** *32*, 1970-1977) was refluxed in Ac₂O (6 ml) for 1 h. After adding H₂O, the pH was adjusted to 9 with aqueous 2N NaOH. The aqueous phase was extracted with CH₂Cl₂, dried over Na₂SO₄, filtered and concentrated under vacuum to give 700 mg of a mixture of the (4-chloro-5-fluoro-2-pyridinyl)methyl acetate (majority) and (4-chloro-5-fluoro-2-pyridinyl)methanol. The mixture was dissolved in aqueous 35% HCl (4 ml) and H₂O (4 ml) and refluxed for 30 minutes. After adding H₂O, the pH was adjusted to 9-14 with aqueous 2N NaOH. The aqueous phase was extracted with CH₂Cl₂, dried over Na₂SO₄, filtered and concentrated under vacuum to give 475 mg of the title compound as a colourless oil. ¹H-NMR (δ, ppm, CDCl₃): 8.43 (s, 1 H), 7.41 (d, 1 H), 4.74 (d, 2H).

### Intermediate 48

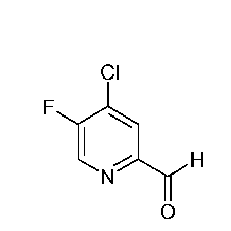

### 4-Chloro-5-fluoro-2-pyridinecarbaldehyde

To a solution of Intermediate 47 (200 mg) in CH₂Cl₂ (5 ml) was added manganese oxide (861 mg). The suspension was stirred overnight and filtered eluting with CH₂Cl₂. The filtrate was concentrated in vacuo to afford 130 mg (66%) of the title compound as a yellow oil. ¹H-NMR (δ, ppm, CDCl₃): 10.00 (s, 1 H), 8.64 (s, 1 H), 8.06 (d, 1 H).

### Intermediate 49

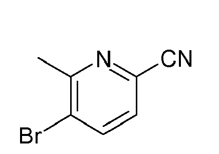

### 5-Bromo-6-methyl-2-pyridinecarbonitrile

A mixture of 3,6-dibromo-2-methylpyridine (200 mg), zinc cyanide (140 mg) and tetrakis(triphenylphosphine)palladium (0) (92.4 mg) in DMF (4 ml) was heated at 100 °C for 10h. Then, an excess of tetrakis(triphenylphosphine)palladium (0) (93 mg) was added. The mixture was heated at 140 °C for 20h. After cooling, the reaction was filtered through a pad of celite, diluted with EtOAc and washed with H₂O (x 3). The combined aqueous layers were then washed with EtOAc (x 3). The combined organic layers were washed with brine, dried over MgSO₄, filtered and concentrated under vacuum to give 314 mg of a brown sticky solid. This was purified on Biotage using hexane/EtOAc 8:2 as eluent to give 89 mg of the title compound as a white solid. [ES MS] m/z 197 (MH+).

### Intermediate 50

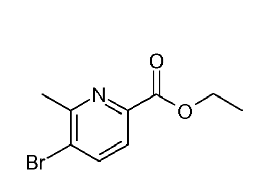

### Ethyl 5-bromo-6-methyl-2-pyridinecarboxylate

A mixture of Intermediate 49 (89 mg) and borane-tetrahydrofuran complex (0.23 ml) in EtOH (2 ml) was refluxed under argon overnight. More borane-tetrahydrofuran complex in EtOH was added until starting material was not detected by LCMS. The solvent was removed in vacuo, the residue was partitioned between CH₂Cl₂ and H₂O and passed through a phase separator with a Na₂SO₄ cartridge attached. The solvent was removed in vacuo to give 87 mg of the title compound as a yellow solid. [ES MS] m/z 266 (M+Na).

### Intermediate 51

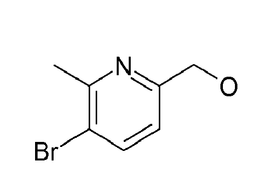

### (5-Bromo-6-methyl-2-pyridinyl)methanol

To a suspension of Intermediate 50 (200 mg) in CH₂Cl₂ (2.5 ml) at 0°C was added di-isobutyl aluminium hydride (1.5M solution in toluene, 1.1 ml). The solution was stirred at 0°C for 8h. Then, more DIBAL-H (1.5 M solution in toluene, 1.1 ml) was added. The reaction was stirred at room temperature overnight. To this solution was added saturated, aqueous NaK-tartrate solution followed by CH₂Cl₂. This emulsion was vigorously stirred. The phases were separated and the aqueous phase was extracted with CH₂Cl₂. The combined organic phases were washed with brine, dried over MgSO₄, filtered and concentrated in vacuo to afford the desired product (155 mg, 96%). ¹H-NMR (δ, ppm, CDCl₃): 7.78 (d, 1 H), 6.96 (d, 1 H), 4.68 (d, 2H), 3.55 (t, 1 H), 2.67 (s, 3H). [ES MS] m/z 202 (MH+).

### Intermediate 52

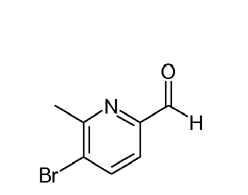

### 5-Bromo-6-methyl-2-pyridinecarbaldehyde

This was prepared from Intermediate 51 (155 mg) using a procedure analogous to that described for Intermediate 48 to give 81 mg (53%) of the title compound. ¹H-NMR (δ, ppm, CDCl₃): 10.01 (s, 1 H), 8.00 (d, 1 H), 7.66 (d, 1 H), 2.78 (s, 3H).

### Intermediate 53

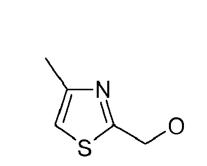

### (4-Methyl-1,3-thiazol-2-yl)methanol

To a solution of 4-methyl-1,3-thiazole-2-carbaldehyde (300 mg) in THF (3 ml) and EtOH (3 ml) at 0°C was added sodiumborohydride (267 mg). The mixture was stirred at room temperature overnight. The solvent was evaporated under vacuum and the residue was suspended in CH₂Cl₂. The suspension was stirred for 2h and the obtained salts were filtered off. The filtrate was concentrated under vacuum to give 252 mg of the title compound as a yellow solid. ¹H-NMR (δ, ppm, CDCl₃): 7.43 (d, 1 H), 7.37(d, 1 H), 3.94 (s, 3H), 2.53(s, 3H).

### Intermediate 54

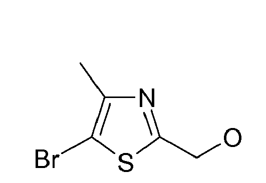

### (5-Bromo-4-methyl-1,3-thiazol-2-yl)methanol

To a solution of Intermediate 53 (252 mg) in DMF was added N-bromosuccinimide (385 mg). The mixture was heated under reflux overnight. The solvent was evaporated under vacuum and the residue obtained was purified by column chromatography on silica gel using hexane/EtOAc as eluent to give 95 mg of the title compound as a brown solid. ¹H-NMR (δ, ppm, CDCl₃): 4.86 (s, 2H), 2.38 (s, 3H).

### Intermediate 55

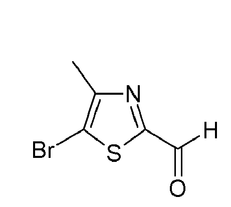

### 5-Bromo-4-methyl-1,3-thiazole-2-carbaldehyde

To a solution of Intermediate 54 (95 mg) in CH₂Cl₂ (10 ml) was added manganese oxide (118 mg). The mixture was heated under reflux overnight. Then, more MnO₂ (79 mg) was added. After refluxing overnight, the cooled solution was filtered through celite and the corresponding filtrate was concentrated under vacuum. The residue obtained was purified by column chromatography on silica gel using hexane/EtOAc as eluent to give 50 mg of the title compound as a yellow oil. ¹H-NMR (δ, ppm, CDCl₃): 9.82 (s, 1 H), 2.52 (s, 3H).

### Intermediate 56

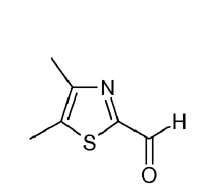

### 4,5-Dimethyl-1,3-thiazole-2-carbaldehyde

To a solution of butyllithium (1.6M solution in hexanes, 2.2 ml) in THF (10 ml) at -78°C was added dropwise 4,5-dimethyl-1,3-thiazole (360 µl). After stirring at -78°C for 1 h, DMF (286 µl) was added and the reaction was allowed to warm to room temperature. The solvent was evaporated under vacuum. H₂O and saturated NaCl were added and aqueous phase was washed with CH₂Cl₂ several times. The combined organic layers were dried over NaSO₄, filtered, and concentrated under vacuum to give 310 mg of the title compound as a yellow oil. ¹H-NMR (δ, ppm, CDCl₃): 9.84 (s, 1H), 2.47(s, 3H), 2.44 (s, 3H).

### Intermediate 57

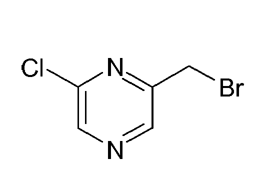

### 2-(Bromomethyl)-6-chloropyrazine

This was prepared from 2-chloro-6-methylpyrazine (200 mg) by the procedure described in Intermediate 46. The residue obtained was purified by column chromatography on silica gel using CH₂Cl₂/hexane as eluent to give 55 mg (17%) of the title compound along with 15.3 mg (11%) of the dibrominated derivative. ¹H-NMR (δ, ppm, CDCl₃): 8.63 (s, 1H) 8.54 (s, 1 H), 4.52 (s, 2H).

### Intermediate 58

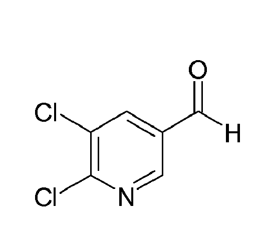

### 5,6-Dichloro-3-pyridinecarbaldehyde

To a solution of (5,6-dichloro-3-pyridinyl)methanol (200 mg) in CH₂Cl₂, molecular sieves (3A) (55 mg) and 4-methy-morpholine-N-oxide (198 mg) was added and stirred at room temperature for 10 minutes. Then tetra-n-propylammonium perruthenate(VII) (38 mg) was added and stirred overnight. The reaction mixture was filtered and the solvent was evaporated to dryness under vacuum. The residue obtained was purified by chromatography column on silica gel using a mixture of EtOAc/hexane as eluent to give 44 mg (22%) of the title compound. ¹H-NMR (δ, ppm, CDCl₃): 10.09 (s, 1 H), 8.77 (s, 1 H), 8.25 (s, 1 H).

### Intermediate 59

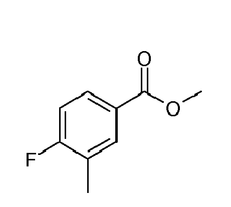

### Methyl 4-fluoro-3-methylbenzoate

To a suspension of 4-fluoro-3-methylbenzoic acid (300 mg) in MeOH (4.5 ml) was added dropwise thionyl chloride (142 µl). The solution was stirred at room temperature overnight and concentrated in vacuo to give 297 mg of the title compound. ¹H-NMR (δ, ppm, CDCl₃): 7.92-7.84 (m, 2H), 7.05 (t, 1 H), 3.91 (s, 3H), 2.32 (d, 3H).

### Intermediate 60

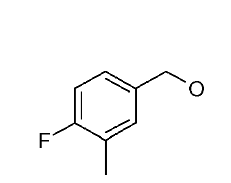

### (4-Fluoro-3-methylphenyl)methanol

This was prepared from 59 (297 mg) using a procedure analogous to that described for Intermediate 51 to give 240 mg (97%) of the title compound. ¹H-NMR (δ, ppm, CDCl₃): 7.24-7.16 (m, 2H), 6.98 (t, 1 H), 4.63 (d, 2H), 2.28 (s, 3H).

### Intermediate 61

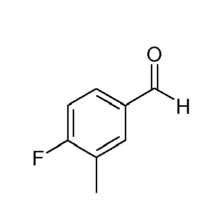

### 4-Fluoro-3-methylbenzaldehyde

This was prepared from Intermediate 60 (240 mg) using a procedure analogous to that described for Intermediate 48 to give 150 mg (61%) of the title compound. ¹H-NMR (δ, ppm, CDCl₃): 9.94 (s, 1 H), 7.77-7.70 (m, 2H), 7.16 (t, 1 H), 2.37 (d, 3H).

### Intermediate 62

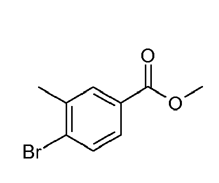

### 4-Bromo-3-methylbenzoic acid

This was prepared from 4-bromo-3-methylbenzoic acid (300 mg) using a procedure analogous to that described for Intermediate 59 to give 315 mg (98%) of the title compound. ¹H-NMR (δ, ppm, CDCl₃): 7.91 (d, 1 H), 7.71 (dd, 1 H), 7.61 (d, 1 H), 3.92 (s, 3H), 2.45 (s, 3H).

### Intermediate 63

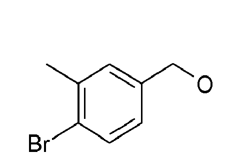

### (4-Bromo-3-methylphenyl)methanol

This was prepared from Intermediate 62 (300 mg) using a procedure analogous to that described for Intermediate 51 to give 212 mg (81%) of the title compound. ¹H-NMR (δ, ppm, CDCl₃): 7.52 (d, 1 H), 7.25 (d, 1 H), 7.05 (dd, 1 H), 4.64 (d, 2H), 2.41 (s, 3H).

### Intermediate 64

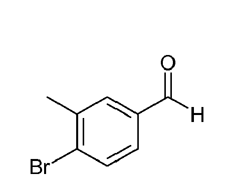

### 4-Bromo-3-methylbenzaldehyde

This was prepared from Intermediate 63 (205 mg) using a procedure analogous to that described for Intermediate 58 to give 62 mg (31%) of the title compound. ¹H-NMR (δ, ppm, CDCl₃): 9.96 (s, 1 H), 7.73 (d, 1 H), 7.56 (dd, 1 H), 2.49 (s, 3H).

### Examples

### Example 1

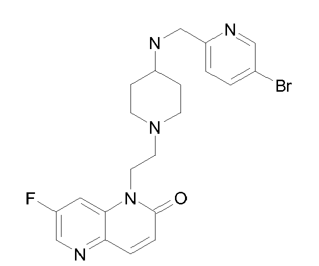

### 1-[2-(4-{[(5-Bromo-2-pyridinyl)methyl]amino}-1-piperidinyl)ethyl]-7-fluoro-1,5-naphthyridin-2(1H)-one

To a solution of Intermediate 21 (662 mg) in 1,2-dichloroethane (20 ml) was added 5-bromo-2-pyridinecarbaldehyde (404 mg, from Alfa Aesar). After stirring for a few minutes, NaBH(OAc)₃ (sodium triacetoxyborohydride) (1.45 g) was added. The reaction was stirred at room temperature overnight and more NaBH(OAc)₃ was added in several portions until starting material was not detected by LCMS. The solvent was removed to dryness under vacuum and the residue was dissolved in CH₂Cl₂. The mixture was washed with saturated NaHCO₃, brine, dried over Na₂SO₄, and concentrated under vacuum. The residue obtained was purified by column chromatography on silica gel using CH₂Cl₂/MeOH 9:1 as eluent to give 490 mg (49%) of the title compound. ¹H-NMR (δ, ppm, CDCl₃): 8.60 (d, 1 H), 8.42 (d, 1 H), 7.88 (d, 1 H), 7.76 (dd, 1 H), 7.56 (dd, 1 H), 7.24 (d, 1 H), 6.85 (d, 1 H), 4.32 (t, 2H), 3.89 (s, 2H), 2.97 (bd, 2H), 2.64 (t, 2H), 2.57-2.46 (m, 1 H), 2.18 (bt, 2H), 1.92 (bd, 2H), 1.51-1.34 (m, 2H). [ES MS] m/z 460 (MH+).

### Example 1b

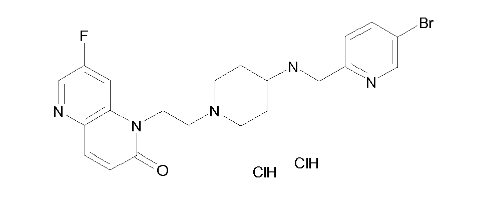

### 1-[2-(4-{[(5-Bromo-2-pyridinyl)methyl]amino}-1-piperidinyl)ethyl]-7-fluoro-1,5-naphthyridin-2(1H)-one dihydrochloride

To a solution of Example 1 (24 mg) in CH₂Cl₂ (1.5 ml) was added dropwise a solution of HCl (4M solution in 1,4-dioxane, 26 µl). The mixture was stirred at room temperature for 10 minutes and the solvent was concentrated under vacuum to give the desired product (28 mg). ¹H-NMR (δ, ppm, DMSO-d₆): 10.94-10.64 (m, 1 H), 9.81-9.64 (m, 1 H), 8.78 (s, 1 H), 8.59 (s, 1 H), 8.37-8.27 (m, 1 H), 8.19-8.16 (m, 1 H), 7.97 (d, 1 H), 7.58 (d, 1 H), 6.85 (d, 1 H), 4.64-4.45 (m, 2H), 4.33 (s, 2H), 3.85-3.63 (m, 1 H), 3.11-2.93 (m, 2H), 2.26-1.90 (m, 4H). [ES MS] m/z 460 (MH+).

### Example 2

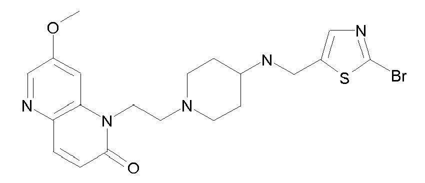

### 1-[2-(4-{[(2-Bromo-1,3-thiazol-5-yl)methyl]amino}-1-piperidinyl)ethyl]-7-(methyloxy)-1,5-naphthyridin-2(1H)-one

To a solution of Intermediate 29 (210 mg) in 1,2-dichloroethane (8 ml) was added 2-bromo-1,3-thiazole-5-carbaldehyde (80 mg, from COMBI-BLOCKS). After stirring for a few minutes, sodium triacetoxyborohydride (278 mg) was added. The reaction was stirred at room temperature overnight and more NaBH(OAc)₃ was added in several portions until starting material was not detected by LCMS. The solvent was removed to dryness under vacuum and the residue was dissolved in CH₂Cl₂. The mixture was washed with saturated NaHCO₃, brine, dried over Na₂SO₄, and concentrated under vacuum. The residue obtained was purified by column chromatography on silica gel using CH₂Cl₂/MeOH 9:1 as eluent to give 110 mg (55%) of the title compound. ¹H-NMR (δ, ppm, CDCl₃): 8.28 (d, 1 H), 7.84 (d, 1 H), 7.38 (s, 1 H), 7.20 (s, 1 H), 6.74 (d, 1 H), 4.36 (t, 2H), 3.99 (bd, 2H), 3.98 (s, 3H), 2.97 (bd, 2H), 2.65 (t, 2H), 2.60-2.48 (m, 1 H), 2.19 (bt, 2H), 1.89 (bd, 2H), 1.47-1.30 (m, 2H). [ES MS] m/z 478 (MH+).

### Example 2b

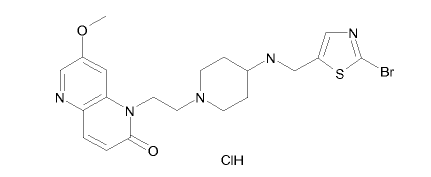

### 1-[2-(4-{[(2-Bromo-1,3-thiazol-5-yl)methyl]amino}-1-piperidinyl)ethyl]-7-(methyloxy)-1,5-naphthyridin-2(1H)-one hydrochloride

To a solution of Example 2 (83 mg) in CH₂Cl₂ (1.5 ml) was added dropwise a solution of HCl (4M solution in 1,4-dioxane, 87 µl). The mixture was stirred at room temperature for 10 minutes and the solvent was concentrated under vacuum to give the desired product (87.5 mg). ¹H-NMR (δ, ppm, DMSO-d₆): 8.30-8.29 (m,1H), 7.88 (d, 1H), 7.75-7.72 (m, 1H), 7.47-7.46 (m, 1 H), 6.67 (d, 1 H), 4.51-4.41 (m, 2H), 4.29-4.22 (m, 2H), 4.00 (s, 3H), 3.25-3.18 (m, 2H), 2.89-2.72 (m, 2H), 2.35-2.26 (m, 1 H), 2.06-1.93 (m, 2H), 1.57-1.44 (m, 2H). [ES MS] m/z 478 (MH+).

### Example 3

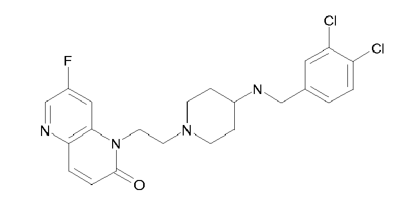

### 1-[2-(4-{[(3,4-Dichiorophenyl)methyl]amino}-1-piperidinyl)ethyl]-7-fluoro-1,5-naphthyridin-2(1H-one

To a solution of Intermediate 21 (700 mg) in 1,2-dichloroethane (30 ml) was added 3,4-dichlorobenzaldehyde (401 mg, from Fluka). After stirring for a few minutes, sodium triacetoxyborohydride (1.5 g) was added. The reaction was stirred at room temperature overnight. The solvent was removed to dryness under vacuum and the residue was dissolved in CH₂Cl₂. The mixture was washed with saturated NaHCO₃, brine, dried over Na₂SO₄, and concentrated under vacuum. The residue obtained was purified by column chromatography on silica gel using CH₂Cl₂/MeOH 9:1 as eluent to give 495 mg of the title compound. ¹H-NMR (δ, ppm, DMSO-d₆): 8.55 (d, 1 H), 8.00-7.91 (m, 2H), 7.58-7.52 (m, 2H), 7.32-7.29 (m, 1 H), 6.82 (d, 1 H), 4.29 (t, 2H), 3.68 (s, 2H), 2.88-2.84 (m, 2H), 2.34-2.24 (m, 1 H), 2.02-1.95 (m, 2H), 1.74-1.70 (m, 2H), 1.22-1.09 (m, 2H). [ES MS] m/z 449 (MH+).

### Example 3b

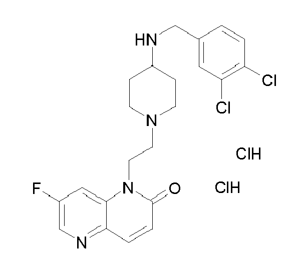

### 1-[2-(4-{[(3,4-Dichiorophenyl)methyl]amino}-1-piperidinyl)ethyl]-7-fluoro-1,5-naphthyridin-2(1H)-one dihydrochloride

To a solution of Example 3 (468 mg) in CH₂Cl₂ (10 ml) was added dropwise a solution of HCl (4M solution in 1,4-dioxane, 520 µl). The mixture was stirred at room temperature for 10 minutes and the solvent was concentrated under vacuum to give the desired product (543 mg). ¹H-NMR (δ, ppm, DMSO-d₆): 10.90-10.78 (m, 1 H), 9.76-9.66 (m, 1 H), 8.60 (s, 1 H), 840-8.26 (m, 1 H), 8.00-7.94 (m, 2H), 7.73 (d, 1 H), 7.60-7.57 (m, 1 H), 6.87 (d, 1 H), 4.65-4.55 (m, 2H), 4.24-4.16 (m, 2H), 3.84-3.72 (m, 2H), 3.19-3.03 (m, 2H), 2.43-2.32 (m, 2H), 2.13-1.98 (m, 2H). [ES MS] m/z 449 (MH+).

### Example 4

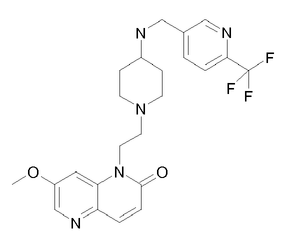

### 7-(Methyloxy)-1-{2-[4-({[6-(trifluoromethyl)-3-pyridinyl]methyl}amino)-1-piperidinyl]ethyl}-1,5-naphthyridin-2(1H)-one

To a solution of Intermediate 28 (100 mg) and Et₃N (0.1 ml) in a mixture of CH₂Cl₂ (3 ml) and MeOH (3 ml) was added 6-(trifluoromethyl)-3-pyridinecarbaldehyde (42 mg, from Aldrich). After stirring for 30 minutes, sodium triacetoxyborohydride (167 mg) was added. The resulting mixture was stirred at room temperature and more NaBH(OAc)₃ (668 mg) was added in several portions until starting material was not detected by LCMS. The solvent was removed to dryness under vacuum and the residue was dissolved in CH₂Cl₂-The mixture was washed with saturated NaHCO₃, dried over Na₂SO₄, and concentrated under vacuum. The residue obtained was purified by column chromatography on silica gel using CH₂Cl₂/MeOH 9:1 as eluent to give 15 mg of the title compound. ¹H-NMR (δ, ppm, DMSO-d₆): 8.69 (s, 1 H), 8.26 (d, 1 H), 8.02 (d, 1 H), 7.87-7.81 (m, 2H), 7.40 (d, 1 H), 6.65 (d, 1 H), 4.34 (t, 2H), 3.96 (s, 3H), 3.82 (s, 2H), 2.92-2.89 (m, 2H), 2.37-2.30 (m, 1 H), 2.05-1.98 (m, 2H), 1.79-1.75 (m, 2H), 1.27-1.15 (m, 2H). [ES MS] m/z 462 (MH+).

### Example 4b

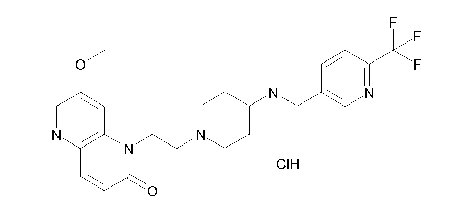

### 7-(Methyloxy)-1-{2-[4-({[H6-(trifluoromethyl)-3-pyridinyl]methyl}amino)-1-piperidinyl]ethyl}-1,5-naphthyridin-2(1H)-one hydrochloride

To a solution of Example 4 (76 mg) in CH₂Cl₂ (1.5 ml) was added dropwise a solution of HCl (4M solution in 1,4-dioxane, 82 µl). The mixture was stirred at room temperature for 10 minutes and the solvent was concentrated under vacuum to give the desired product (78 mg). ¹H-NMR (δ, ppm, DMSO-d₆): 8.30-8.29 (m,1H), 7.88 (d, 1H), 7.75-7.72 (m, 1H), 7.47-7.46 (m, 1H), 6.67 (d, 1H), 4.51-4.41 (m, 2H), 4.29-4.22 (m, 2H), 4.00 (s, 3H), 3.25-3.18 (m, 2H), 2.89-2.72 (m, 2H), 2.35-2.26 (m, 1H), 2.06-1.93 (m, 2H), 1.57-1.44 (m,2H). [ES MS] m/z 478 (MH+).

### Example 5

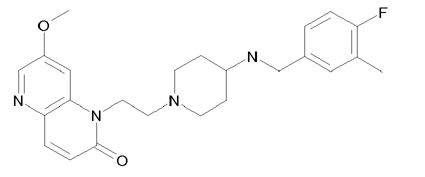

### 1-[2-(4-{[(4-Fluoro-3-methylphenyl)methyl]amino}-1-piperidinyl)ethyl]-7-(methyloxy)-1,5-naphthyridin-2(1H)-one

To a solution of Intermediate 29 (105 mg) in a mixture of CH₂Cl₂ (2.5 ml) and MeOH (2.5 ml) was added Intermediate 61 (48 mg). After stirring for a few minutes, sodium triacetoxyborohydride (223 mg) was added. The mixture was stirred at room temperature overnight and more Intermediate 61 (30 mg), NaBH(OAc)₃ and a few drops of acetic acid were added in several portions until starting material was not detected by LCMS. The resulting mixture was quenched with saturated NaHCO₃, extracted with CH₂Cl₂, dried over Na₂SO₄ and concentrated under vacuum. The residue obtained was purified by preparative HPLC (gradient elution: 10 to 100% CH₃CN/H₂O 0.1% TFA, uv detection 254 nm) to give the title compound as a salt. The obtained compound was dissolved in aqueous 10% Na₂CO₃ and extracted with CH₂Cl₂ to give 20 mg of the title compound. ¹H-NMR (δ, ppm, CDCl₃): 8.28 (d, 1 H), 7.84 (d, 1 H), 7.24 (bd, 1 H), 7.14-7.05 (m, 2H), 6.93 (t, 1 H), 6.74 (d, 1 H), 4.37 (d, 1 H), 3.97 (s, 3H), 3.73 (s, 2H), 2.99 (bd, 2H), 2.64 (t, 2H), 2.58-2.48 (m, 1 H), 2.26 (bd, 3H), 2.19 (bt, 2H), 1.91 (bd, 2H), 1.49-1.36 (m, 2H). [ES MS] m/z 425 (MH+).

### Example 6

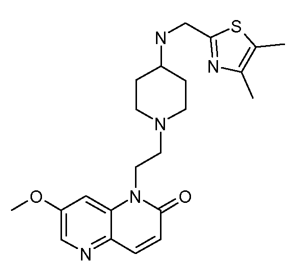

### 1-[2-(4-{[(4,5-Dimethyl-1,3-thiazoi-2-yl)methyl]amino}-1-piperidinyl)ethyl]-7-(methyloxy)-1,5-naphthyridin-2(1H)-one

To a solution of Intermediate 29 (100 mg) in tetrahydrofurane (5 ml) was added Intermediate 56 (57 mg). After stirring for 1 h, sodium triacetoxyborohydride (76 mg) was added. The mixture was stirred at room temperature overnight. LCMS showed that reaction did not take place. The solvent was evaporated to dryness under vacuum, and 5 ml of 1,2-dichloroethane and sodium triacetoxyborohydride (152 mg) were added leaving the reaction under stirring overnight. The solvent was evaporated to dryness under vacuum. H₂O/NaHCO₃ 10% was added and the aqueous layer was extracted with CH₂Cl₂, dried over Na₂SO₄, concentrated under vacuum and purified by column chromatography on silica gel using CH₂Cl₂ /MeOH 80:20 as eluent to give 33 mg of the title compound as a yellow solid. ¹H-NMR (δ, ppm, CDCl₃): 8.27 (s, 1 H), 7.84 (d, 1 H), 6.63 (d, 1 H), 4.37 (t, 2H), 4.02 (s, 2H), 3.98 (s, 3H), 3.04-2.90 (m, 2H), 2.71-2.51 (m, 3H), 2.32 (s, 3H), 2.29 (s, 3H), 2.20 (t, 2H), 2.00-1.85 (m, 2H), 1.53-1.34 (m, 2H). [ES MS ] m/z 428 (MH+).

### Example 7

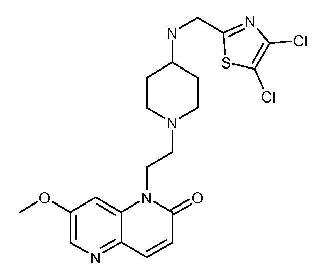

### 1-[2-(4-{[(4,5-Dichloro-1,3-thiazol-2-yl)methyl]amino}-1-piperidinyl)ethyl]-7-(methyloxy)-1,5-naphthyridin-2(1H)-one

To a solution of Intermediate 29 (128 mg) in 1,2-dichloroethane (10 ml) was added Intermediate 41 (76 mg). After stirring for 30 minutes, sodium triacetoxyborohydride (293 mg) was added. The resulting mixture was stirred at room temperature for 2h and 293 mg of NaBH(OAc)₃ were added. The mixture was stirred at room temperature overnight. The solvent was removed to dryness under vacuum, H₂O was added and pH was adjusted to 11 with aqueous NaOH. CH₂Cl₂ was added to the reaction mixture and organic phase was extracted. The residue obtained was purified by preparative HPLC. The obtained compound was dissolved in 0.5N NaOH and extracted with CH₂Cl₂ to give 32 mg of the title compound as a white solid. ¹H-NMR (δ, ppm, CDCl₃): 8.28 (d, 1 H), 7.84 (d, 1 H), 7.19 (d, 2H), 6.74 (d, 1 H), 4.36 (t, 2H), 4.02 (d, 2H), 3.98 (s, 3H), 3.05-2.91 (m, 2H), 2.65 (t, 2H), 2.60-2.49 (m, 1 H), 2.19 (dt, 2H), 1.91 (bd, 2H), 1.49-1.32 (m, 2H). [ES MS] m/z 468 (MH+).

### Example 8

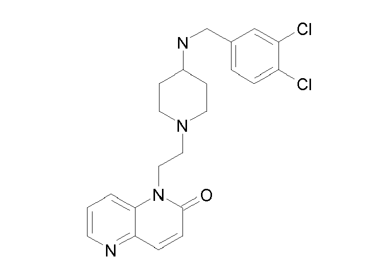

### 1-[2-(4-{[(3,4-Dichlorophenyl)methyl]amino}-1-piperidinyl)ethyl]-1,5-naphthyridin-2(1H)-one

To a solution of Intermediate 8 (1.5 g) in a mixture of CH₂Cl₂ (10 ml) and MeOH (10 ml) was added 3,4-dichlorobenzaldehyde (965 mg, from Fluka). After stirring for 30 minutes, sodium triacetoxyborohydride (3.5 g) was added. The mixture was stirred at room temperature for 3h. The resulting mixture was quenched with saturated NaHCO₃, extracted with CH₂Cl₂, dried over Na₂SO₄ and concentrated under vacuum. The residue obtained was purified by column chromatography on silica gel using a MeOH and CH₂Cl₂ gradient (0-20%) to give the desired product as a white solid (1.58 g, 67%). ¹H-NMR (δ, ppm,CDCl₃): 8.55 (dd, 1 H), 7.91 (d, 1 H), 7.78 (d, 1 H), 7.40 (m, 2H), 7.17 (dd, 1 H), 6.91 (d, 1 H), 4.38 (t, 2H), 3.76 (s, 2H), 2.97 (d, 2H), 2.64 (t, 2H), 2.50 (m, 1 H), 2.18 (t, 2H), 1.87 (d, 2H), 1.42 (m, 4H). [ES MS] m/z 431 (MH+).

### Example 8b

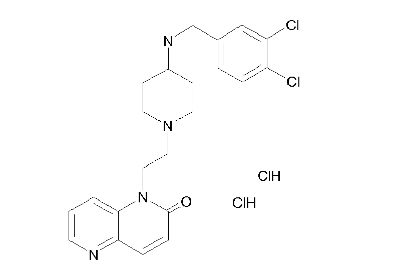

### 1-[2-(4-{[(3,4-Dichlorophenyl)methyl]amino}-1-piperidinyl)ethyl]-1,5-naphthyridin-2(1H)-one dihydrochloride

To a solution of Example 8 (940 mg) in CH₂Cl₂ (20 ml) was added dropwise a solution of HCl (4M solution in 1,4-dioxane, 1.1 ml). The mixture was stirred at room temperature for 10 minutes and the solvent was concentrated under vacuum to give the desired product (1.04 g). ¹H-NMR (δ, ppm, DMSO-d₆): 10.97-10.85 (m, 1 H), 9.82-9.67 (m, 2H), 8.56 (d, 1 H), 8.32-8.16 (m, 1 H), 7.99-7.94 (m, 2H), 7.74-7.58 (m, 3H), 6.90 (d, 1 H), 4.68-4.52 (m, 2H), 4.19 (s, 2H), 3.83-3.66 (m, 1 H), 3.21-3.04 (m, 2H), 2.40-1.90 (m, 4H). [ES MS] m/z 431 (MH+).

### Example 9

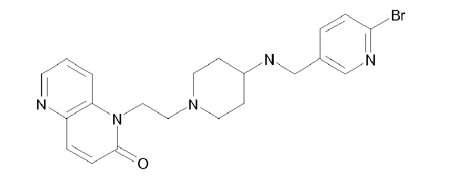

### 1-[2-(4-{[(6-Bromo-3-pyridinyl)methyl]amino}-1-piperidinyl)ethyl]-1,5-naphthyridin-2(1H)-one

To a solution of Intermediate 8 (60 mg) in 1,2-dichloroethane (5 ml) was added 6-bromo-3-pyridinecarbaldehyde (39 mg, from Aldrich) and sodium triacetoxyborohydride (140 mg). The resulting mixture was stirred at room temperature and more NaBH(OAc)₃ was added in several portions until starting material was not detected by LCMS. The solvent was removed to dryness under vacuum and the residue was dissolved in CH₂Cl₂. The mixture was washed with saturated NaHCO₃, dried over Na₂SO₄, and concentrated under vacuum. The residue obtained was purified by preparative HPLC (gradient elution: 10 to 100% CH₃CN/H₂O, X-terra 19x150 mm, uv detection 254 nm) to give 42 mg (43%) of the title compound. ¹H-NMR (δ ppm, CDCl₃): 8.54 (d, 1 H), 7.91 (d, 1 H), 7.80 (m, 1 H), 7.47 (dd, 1 H), 7.10 (s, 1 H), 6.91 (d, 1 H), 6.83 (s, 1 H), 4.40 (t, 2H), 3.97 (s, 2H), 2.98 (m, 2H), 2.66 (m, 2H), 2.21 (m, 2H), 1.90 (bd, 2H), 1.25-1.70 (m, 4H). [ES MS] m/z 447 (MH+).

### Example 10

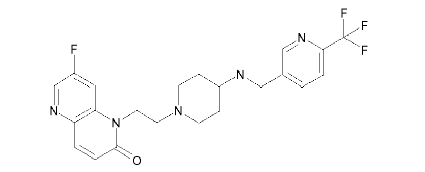

### 7-Fluoro-1-{2-[4-({[6-(trifluoromethyl)-3-pyridinyl]methyl}amino)-1-piperidinyl]ethyl}-1,5-naphthyridin-2(1H)-one

To a solution of Intermediate 20 (90 mg) and Et₃N (0.1 ml) in a mixture of CH₂Cl₂ (5 ml) and MeOH (1 ml) was added 6-(trifluoromethyl)-3-pyridinecarbaldehyde (41 mg, from Apollo). After stirring for 30 minutes, sodium triacetoxyborohydride (150 mg) was added. The resulting mixture was stirred at room temperature and more NaBH(OAc)₃ (600 mg) and a few drops of acetic acid were added in several portions until starting material was not detected by LCMS. The solvent was evaporated to dryness under vacuum, H₂O was added and pH was adjusted to 11 with 30% aqueous ammonia. The aqueous layer was extracted with EtOAc, dried over Na₂SO₄, concentrated under vacuum and purified by column chromatography on silica gel using CH₂Cl₂/MeOH 9:1 as eluent to give 45 mg (44%) of the title compound as a white solid. ¹H-NMR (δ, ppm, CDCl₃): 8.67 (s, 1 H), 8.42 (d, 1 H), 7.88 (d, 2H), 7.64 (d, 1 H), 7.53 (bd, 1 H), 6.86 (d, 1 H), 4.32 (t, 2H), 3.92 (s, 2H), 2.97 (bd, 2H), 2.66 (t, 2H), 2.56-2.49 (m, 1 H), 2.19 (t, 2H), 1.92 (bd, 2H), 1.46-1.36 (m, 2H). [ES MS] m/z 450 (MH+).

### Example 11

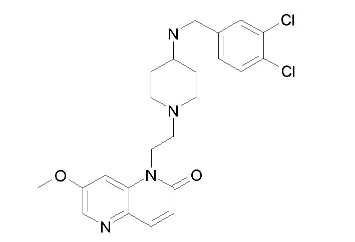

### 1-[2-(4-{[(3,4-Dichlorophenyl)methyl]amino}-1-piperidinyl)ethyl]-7-(methyloxy)-1,5-naphthyridin-2(1H)-one

To a solution of Intermediate 28 (189 mg) and Et₃N (0.2 ml) in a mixture of CH₂Cl₂ (6 ml) and MeOH (0.6 ml) was added 3,4-dichlorobenzaldehyde (76 mg, from Fluka). After stirring for 30 minutes, sodium triacetoxyborohydride (300 mg) was added. The resulting mixture was stirred at room temperature and more NaBH(OAc)₃ (900 mg) was added in several portions until starting material was not detected by LCMS. The resulting mixture was quenched with saturated NaHCO₃, extracted with a mixture of CH₂Cl₂/MeOH 8:2, dried over Na₂SO₄ and concentrated under vacuum. The residue obtained was purified by column chromatography on silica gel using CH₂Cl₂/MeOH 95:5 as eluent to give 82 mg (40%) of the title compound as a white solid. ¹H-NMR (δ, ppm, CDCl₃): 8.28 (d, 1 H), 7.84 (d, 1 H), 7.44 (s, 1 H), 7.38 (d, 2H), 7.25 (bd, 1 H), 7.16 (d, 1 H), 6.73 (d, 1 H), 4.38 (t, 2H), 3.98 (s, 3H), 3.77 (s, 2H), 2.99 (bd, 2H), 2.66 (t, 2H), 2.58-2.45 (m, 1 H), 2.28-2.15 (m, 2H), 1.95 (d, 2H), 1.50-1.35 (m, 2H). ES MS] m/z 461 (MH+).

### Example 11b

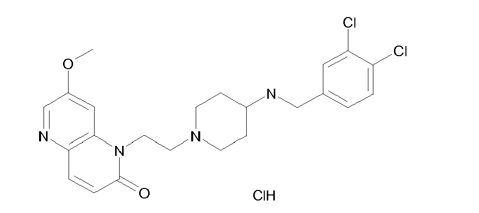

### 1-[2-(4-{[(3,4-Dichiorophenyl)methyl]amino}-1-piperidinyl)ethyl]-7-(methyloxy)-1,5-naphthyridin-2(1H)-one hydrochloride

To a solution of Example 11 (14.3 mg) in CH₂Cl₂ (0.5 ml) was added dropwise a solution of HCl (4M solution in 1,4-dioxane, 15.5 µl). The mixture was stirred at room temperature for 10 minutes and the solvent was concentrated under vacuum to give the desired product (12 mg). ¹H-NMR (δ, ppm, DMSO-d₆): 8.28 (d, 1 H), 7.88-7.83 (m, 2H), 7.70-7.68 (m, 1 H), 7.51-7.48 (m, 1 H), 7.42 (s, 1 H), 6.65 (d, 1 H), 4.40-4.36 (m, 2H), 4.11-4.06 (m, 2H), 3.98 (s, 3H), 3.11-3.04 (m, 2H), 2.97-2.83 (m, 1 H), 2.61-2.57 (m, 2H), 2.13-1.97 (m, 4H), 1.50-1.45 (m, 2H). [ES MS] m/z 461 (MH+).

### Example 11c

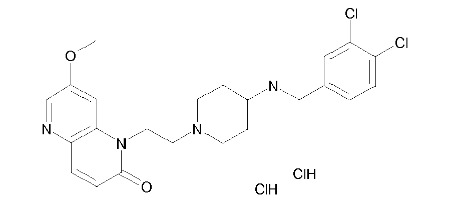

### 1-[2-(4-{[(3,4-Dichlorophenyl)methyl]amino}-1-piperidinyl)ethyl]-7-(methyloxy)-1,5-naphthyridin-2(1H)-one dihydrochloride

To a solution of Example 11 (230 mg) in CH₂Cl₂ (5 ml) was added dropwise a solution of HCl (4M solution in 1,4-dioxane, 249 µl). The mixture was stirred at room temperature for 10 minutes and the solvent was concentrated under vacuum to give the desired product (259 mg).
¹H-NMR (δ, ppm, DMSO-d₆): 11.32-11.18 (m, 1H), 9.75-9.56 (m, 2H), 8.31 (d, 1H), 7.93-7.90 (m, 2H), 7.74-7.57 (m, 3H), 6.69 (d, 1 H), 4.72-4.58 (m, 2H), 4.24-4.16 (m, 2H), 4.04 (s, 3H), 3.86-3.65 (m, 2H), 3.27-3.02 (m, 3H), 2.43-1.93 (m, 4H). [ES MS] m/z 461 (MH+).

### Example 12

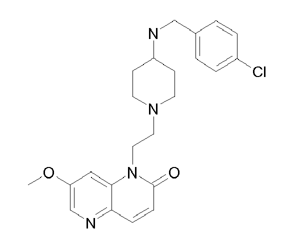

### 1-[2-(4-{[(4-Chlorophenyl)methyl]amino}-1-piperidinyl)ethyl]-7-(methyloxy)-1,5-naphthyridin-2(1H)-one

To a solution of Intermediate 28 (100 mg) and Et₃N (0.1 ml) in a mixture of CH₂Cl₂ (5 ml) and MeOH (1 ml) was added 4-chlorobenzaldehyde (34 mg, from Fluka). After stirring for 30 minutes, sodium triacetoxyborohydride (167 mg) was added. The resulting mixture was stirred at room temperature and more NaBH(OAc)₃ (668 mg) was added in several portions until starting material was not detected by LCMS. The resulting mixture was quenched with saturated NaHCO₃, extracted with CH₂Cl₂, dried over Na₂SO₄ and concentrated under vacuum. The residue obtained was purified by column chromatography on silica gel using CH₂Cl₂/MeOH 85:15 as eluent to give 66 mg (59%) of the title compound as a yellow solid. ¹H-NMR (δ, ppm, CDCl₃): 8.25 (d, 1 H), 7.86 (d, 1 H), 7.22-7.34 (m, 5H), 6.73 (d, 1 H), 4.44 (t, 2H), 4.00 (s, 2H), 3.79 (s, 2H), 3.07 (bd, 2H), 2.72 (t, 2H), 2.59 (bs, 1 H), 2.31 (bs, 2H), 1.96 (d, 2H), 1.41-1.60 (m, 2H). [ES MS] m/z 427 (MH+).

### Example 12b

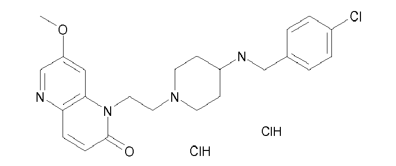

### 1-[2-(4-{[(4-Chlorophenyl)methyl]amino}-1-piperidinyl)ethyl]-7-(methyloxy)-1,5-naphthyridin-2(1H)-one dihydrochloride

To a solution of Example 12 (205 mg) in CH₂Cl₂ (5 ml) was added dropwise a solution of HCl (4M solution in 1,4-dioxane, 240 µl). The mixture was stirred at room temperature for 10 minutes and the solvent was concentrated under vacuum to give the desired product (215 mg). ¹H-NMR (δ, ppm, DMSO-d₆): 11.14-11.07 (m, 1 H), 9.57-9.48 (m, 1 H), 9.10-8.96 (m, 1 H), 8.30 (s, 1 H), 7.91-7.90 (m, 1 H), 7.57-7.50 (m, 5H), 6.69-6.67 (m, 1 H), 4.71-4.63 (m, 1 H), 4.43-4.34 (m, 1 H), 4.21-4.12 (m, 2H), 4.01 (s, 3H), 3.83-3.70 (m, 1 H), 3.17-2.98 (m, 3H), 2.17-1.96 (m, 3H), 1.62-1.47 (m, 1 H). [ES MS] m/z 427 (MH+).

### Example 12c

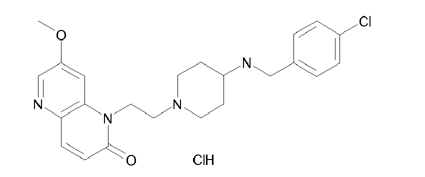

### 1-[2-(4-{[(4-Chlorophenyl)methyl]amino}-1-piperidinyl)ethyl]-7-(methyloxy)-1,5-naphthyridin-2(1H)-one hydrochloride

To a solution of Example 12 (328 mg) in CH₂Cl₂ (6 ml) was added dropwise a solution of HCl (3N solution in MeOH, 240 µl). The mixture was stirred at room temperature for 10 minutes and the solvent was concentrated under vacuum to give the desired product (215 mg). ¹H-NMR (δ, ppm, D₂O): 8.13 (d, 1 H), 7.78 (d, 1 H), 7.33-7.17 (m, 5H), 6.62 (d, 1 H), 4.29 (bt, 2H), 4.04 (s, 2H), 3.83 (s, 3H), 3.08 (bd, 3H), 2.60 (bt, 2H), 2.18 (bt, 2H), 2.03 (bd, 2H), 1.62-1.44 (m, 2H). [ES MS] m/z 427 (MH+).

### Example 13

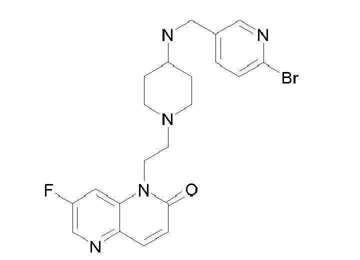

### 1-[2-(4-{[(6-Bromo-3-pyridinyl)methyl]amino}-1-piperidinyl)ethyl]-7-fluoro-1,5-naphthyridin-2(1H-one

To a solution of Intermediate 20 (100 mg) and Et₃N (0.11 ml) in a mixture of CH₂Cl₂ (5 ml) and MeOH (1 ml) was added 6-bromo-3-pyridinecarbaldehyde (49 mg, from Aldrich). After stirring for 30 minutes, sodium triacetoxyborohydride (171 mg) was added. The resulting mixture was stirred at room temperature and more NaBH(OAc)₃ (684 mg) was added in several portions until starting material was not detected by LCMS. The solvent was evaporated to dryness under vacuum, H₂O was added and pH was adjusted to 11 with aqueous 6N NaOH. The aqueous layer was extracted with a mixture of CH₂Cl₂/MeOH 95:5, dried over Na₂SO₄, concentrated under vacuum and purified by column chromatography on silica gel using CH₂Cl₂/MeOH 95:5 as eluent to give 52 mg (45%) of the title compound as a white solid. ¹H-NMR (δ ppm, CD₃OD): 8.48 (d, 1 H), 8.32 (d, 1 H), 7.92-8.00 (m, 2H), 7.72 (dd, 1 H), 7.56 (d, 1 H), 6.86 (d, 1 H), 4.42 (t, 2H), 3.78 (s, 2H), 3.04 (d, 2H), 2.65 (t, 2H), 2.43-2.55 (m, 2H), 2.16 (t, 2H), 1.99 (d, 2H), 1.33-1.49 (m, 2H). [ES MS] m/z 460 (MH+).

### Example 14

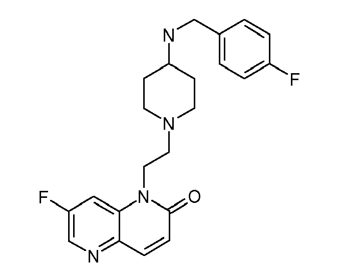

### 7-Fluoro-1-[2-(4-{[(4-fluorophenyl)methyl]amino}-1-piperidinyl)ethyl]-1,5-naphthyridin-2(1H)-one

To a solution of Intermediate 20 (90 mg) and Et₃N (0.11 ml) in a mixture of CH₂Cl₂ (5 ml) and MeOH (1 ml) was added 4-fluorobenzaldehyde (0.02 ml, from Acros). After stirring on an orbital shaker for 5 minutes, a solution of polymer-supported cyanoborohydride (242 mg, load = 2.5 mmol/g) and a few drops of acetic acid were added. The mixture was stirred at room temperature overnight. The resulting solution was filtered and the resin was washed with MeOH and CH₂Cl₂. The solvent was eliminated under vacuum. The residue obtained was purified by column chromatography on silica gel using CH₂Cl₂/MeOH 9:1 as eluent to give 20 mg (22%) of the title compound as a white solid.
¹H-NMR (δ, ppm, CD₃OD): 8.50 (d, 1H), 8.01-7.95 (m, 2H), 7.55-7.50 (m, 2H), 7.23-7.17 (m, 2H), 6.88 (d, 1 H), 4.49 (t, 2H), 4.21 (s, 2H), 3.29-3.18 (m, 3H), 2.87 (m, 2H), 2.38 (m, 2H), 2.18 (bd, 2H), 1.71 (m, 2H). [ES MS] m/z 399 (MH+).

### Example 15

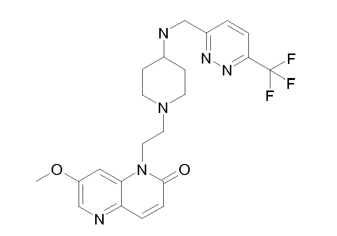

### 7-(Methyloxy)-1-{2-[4-({[6-(trifluoromethyl)-3-pyridazinyl]methyl}amino)-1-piperidinyl]ethyl}-1,5-naphthyridin-2(1H)-one

To a solution of Intermediate 29 (35.1 mg) in acetonitrile (1.5 ml) was added a mixture of K₂CO₃ (16.0 mg) and Intermediate 46 (14.0 mg). The reaction mixture was stirred at room temperature for 3h. The solid was filtered off and the solvent evaporated. The residue obtained was purified by column chromatography on silica gel using CH₂Cl₂/MeOH as eluent to give 16 mg (60%) of the title compound as a yellow solid.
¹H-NMR (δ, ppm, CDCl₃): 8.29 (d, 1 H), 7.88-7.77 (m, 3H), 7.23 (bd, 1 H), 6.75 (d, 1 H), 4.38 (bt, 2H), 4.26 (s, 2H), 3.99 (s, 3H), 3.07-2.96 (m, 2H), 2.73-2.53 (m, 3H), 2.22 (bt, 2H), 1.95 (bd, 2H), 1.56-1.38 (m, 2H). [ES MS] m/z 463 (MH+).

### Example 16

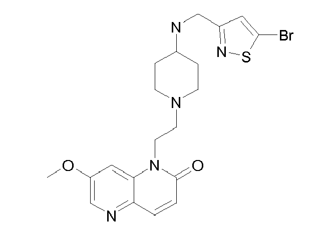

### 1-[2-(4-{[(5-Bromo-3-isothiazoiyl)methyl]amino}-1-piperidinyl)ethyl]-7-(methyloxy)-1,5-naphthyridin-2(1H)-one

To a solution of Intermediate 29 (92.0 mg) in acetonitrile (2 ml) and methanol (1 ml) was added a mixture of K₂CO₃ (41.5 mg) and 5-bromo-3-(bromomethyl)isothiazole (39.2 mg, Sunshine Chemlab). The reaction mixture was stirred at room temperature for 3h. The solid was filtered off and the solvent evaporated. The residue obtained was purified by column chromatography on silica gel using CH₂Cl₂/MeOH as eluent and further purified by trituration with a *t*-BuOMe/hexane 1:2 mixture to give 15.5 mg (21 %) of the title compound as a beige solid. ¹H-NMR (δ ppm, CDCl₃): 8.28 (d, 1 H), 7.85 (d, 1 H), 7.29-7.24 (m, 1 H), 7.19 (s, 1 H), 6.74 (d, 1 H), 4.39 (bt, 2H), 3.99 (s, 3H), 3.95 (s, 2H), 3.09-2.95 (m, 2H), 2.67 (bt, 2H), 2.63-2.50 (m, 1 H), 2.23 (bt, 2H), 1.93 (bd, 2H), 1.55-1.37 (m, 2H). [ES MS] m/z 478 and 480 (MH+).

### Examples 17-111

Examples 17-111 were prepared using methods analogous to those described for the above Examples, using the starting materials as indicated in the table below.

| **Ex.** | **Structure** | **chemical name** | **Physical data** | **Method analogous to which Ex.** | **Starting aldehyde* (source)** | **Starting Naphthyridone** |
|---|---|---|---|---|---|---|
| **17** | 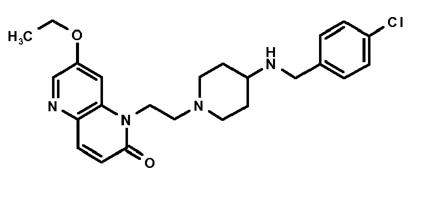 | 1-[2-(4-{[(4-chlorophenyl)met hyl]amino}-1-piperidinyl)ethyl]-7-(ethyloxy)-1,5- naphthyridin-2(1H)-one | ¹H-NMR (δ, ppm, DMSO-d₆): 8.25 (d, 1H); 7.84 (d, 1 H); 7.38-7.37 (m, 1 H); 7.34 (s, 4H); 6.64 (d, 1H); 4.32-4.22 (m, 4H); 3.69 (s, 2H); 2.91-2.87 (m, 2H); 2.38-2.30 (m, 1H); 2.03-1.95 (m, 2H); 1.78-1.73 (m, 2H); 1.39 (t, 3H); 1.27-1.16 (m, 2H). [ES MS] m/z 441 (MH+). | Example1 | 4-chlorobenzaldeh yde (Fluka) | Intermediate 38 |
| **18** | 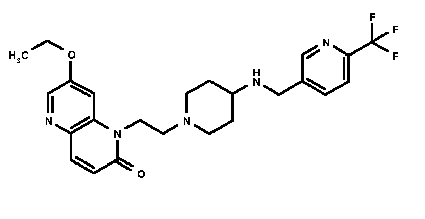 | 7-(ethyloxy)-1-{2-[4-({[6-(trifluoromethyl)-3-pyridinyl]methyl}a mino)-1-piperidinyl]ethyl}-1,5-naphthyridin-2(1H)-one | ¹H-NMR (δ, ppm, DMSO-d₆): 8.69 (s, 1H); 8.25 (d, 1H); 8.02 (d, 1H); 7.86-7.82 (m, 2H); 7.38-7.37 (m, 1H); 6.63 (d, 1 H); 4.35-4.22 (m, 4H); 3.82 (s, 2H); 2.91-2.88 (m, 2H); 2.37-2.29 (m, 1H); 2.04-1.97 (m, 2H); 1.79-1.75 (m, 2H)_{;} 1.39 (t, 3H); 1.26-1.15 (m, 2H). [ES MS] m/z 476 (MH+). | Example1 | 6-(trifluoromethyl)p yridine-3-carboxaldehyde (Apollo) | Intermediate 38 |
| **19** | 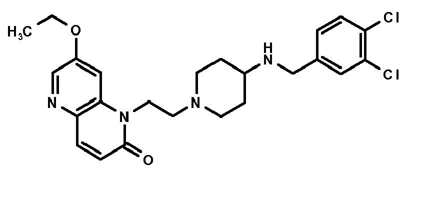 | 1-[2-(4-{[(3,4-dichlorophenyl)m ethyl]amino}-1-piperidinyl)ethyl]-7-(ethyloxy)-1,5₋ naphthyridin-2(1H)-one | ¹H-NMR (δ, ppm, DMSO-d₆): 8.25 (d,1H); 7.84 (d, 1 H); 7.59 (d, 1 H); 7.53 (d, 1 H); 7.38 (d, 1 H); 7.33-7.29 (m, 1H); 6.63 (d, 1 H); 4.34-4.22 (m, 4H); 3.69 (s, 2H); 2.90-2.87 (m, 2H); 2.35-2.25 (m, 1H); 2.03-1.96 (m, 2H); 1.76-1.72 (m, 2H); 1.39 (t, 3H); 1.25-1.14 (m, 2H). [ES MS] m/z 475 (MH+). | Example1 | 1-[2-(4-{[(3,4-dichlorophenyl)m ethyl]amino}-1-piperidinyl)ethyl]-7-(ethyloxy)-1,5-naphthyridin-2(1H)-one (Fluka) | Intermediate 38 |
| **20** | 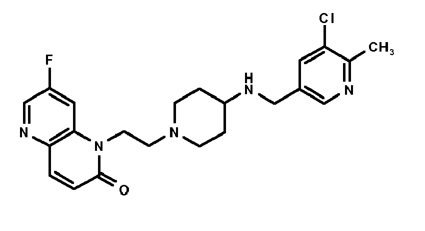 | 1-[2-(4-{[(5-ch loro-6-m ethyl-3-pyridinyl)methyl]a mino}-1-piperidinyl)ethyl]-7-fluoro-1,5-naphthyridin-2(1H)-one | ¹H-NMR (δ, ppm, CDCl₃): 8.36(d, 1H); 8.28 (d, 1H); 7.85 (d,1H); 7.54 (bd, 1 H); 7.34 (dd, 1H); 6.74 (d, 1H); 4.39 (bd, 2H); 3.99 (s, 3H); 3.92 (s, 2H); 3.08-2.96 (m, 2H); 2.72-2.62 (m, 2H); 2.62-2.51 (m, 1H); 2.32-2.14 (m, 2H); 1.99-1.80 (m, 2H); 1.76-1.59 (m, 3H). [ES MS] m/z 472 (MH+). | Example1 | 5-chloro-6-methyl-3-pyridinecarbalde hyde (Described in WO20006/13748 5 A1) | Intermediate 21 |
| **21** | 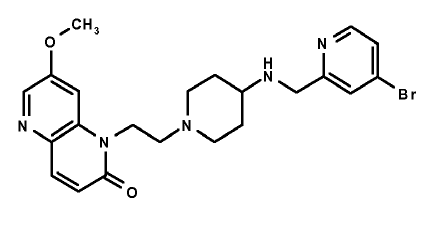 | 1-[2-(4-{[(4-bromo-2-pyridinyl)methyl]a mino}-1-piperidinyl)ethyl]-7-(methyloxy)-1,5-naphthyridin-2(1H)-one | ¹H-NMR (d, ppm, CDCl₃): 8.56 (d, 1 H); 8.46 (d, 1H); 8.28(d,1H); 8.88-8.83 (m, 2H); 7.22 (bd, 1 H); 4.37 (t, 2H); 3.98 (s, 3H); 3.82 (s, 2H); 2.98 (bd, 2H); 2.65 (bt, 2H); 2.19 (bt, 2H); 2.23 (bt, 2H); 1.87-1.99 (m, 2H); 160-1.37 (m, 4H). [ES MS] m/z 472 (MH+). | Example1 | 4-bromo-2-pyridinecarbalde hyde (PRINCETON) | Intermediate 29 |
| **22** | 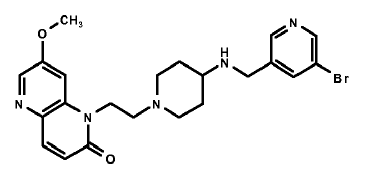 | 1-[2-(4-{[(5-bromo-3-pyridinyl)methyl]a mino}-1-piperidinyl)ethyl]-7-(methyloxy)-1,5-naphthyridin-2(1H)-one | ¹H-NMR (δ, ppm, CDCl₃): 8.27 (d, 1H); 7.84 (d, 1 H); 7.29-7.23 (m,2H); 7.18 (bd, 1H); 7.06 (dd, 1H); 7.74 (d, 1H); 4.37 (bt, 2H); 3.97 (s, 3H); 3.75 (s, 2H); 2.99 (bd, 2H); 2.65 (bt, 2H); 2.58-2.45 (m, 1 H); 2.36 (s, 3H); 2.19 (bt, 2H); 1.91 (bd, 2H). [ES MS] m/z 441 (MH+). | Example1 | 5-bromo-3-pyridinecarbalde hyde (Aldrich) | Intermediate 29 |
| **23** | 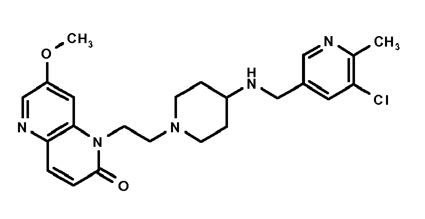 | 1-[2-(4-{[(5-chloro-6-methyl-3-pyridinyl)methyl]a mino}-1-piperidinyl)ethyl]-7-(methyloxy)-1,5-naphthyridin-2(1H)-one | ¹H-NMR (δ, ppm, CDCl₃): 8.55 (d, 1 H); 7.91 (d, 1 H); 7.81 (d, 1 H); 7.49-7.44 (m, 1 H); 7.29 (d, 1 H); 7.16 (d, 1H); 7.09 (d, 1 H); 6.91 (d, 1 H); 4.40 (t, 2H); 3.75 (s, 2H); 2.99 (d, 2H); 2.65 (t, 2H); 2.57-2.48 (m, 1H); 2.35 (s, 3H); 2.20 (t, 2H); 1.90 (d, 2H); 1.50-1.36 (m, 2H). [ES MS] m/z 411 (MH+). | Example1 | 5-chloro-6-methyl-3-pyridinecarbalde hyde (Described in WO20006/13748 5 A1) | Intermediate 29 |
| **24** | 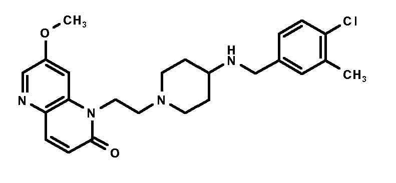 | 1-[2-(4-{[(4-chloro-3-methylphenyl)me thyl]amino}-1-piperidinyl)ethyl]-7-(methyloxy)-1,5-naphthyridin-2(1H)-one | ¹H-NMR (δ, ppm, CDCl₃): 8.56 (d, 1 H); 7.93 (d, 1 H); 7.72 (d,1 H); 7.48 (t, 1H); 7.46-7.44 (m, 1 H); 7.38 (d, 1 H); 7.29-7.24 (m, 1 H); 7.18 (dd, 1 H); 4.55-4.24 (m, 2H); 3.90-3.84 (m, 2H); 3.78 (d, 2H); 2.98 (dd, 2H); 2.70 (bt, 2H); 2.51-2.42 (m, 1H). [ES MS] m/z 447 (MH+). | Example1 | 4-chloro-3-methylbenzaldeh yde (Fluorochem) | Intermediate 29 |
| **25** | 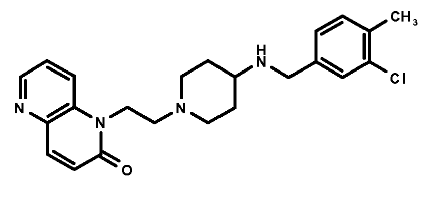 | 1-[2-(4-{[(3-chloro-4-methylphenyl)me thyl]amino}-1-piperidinyl)ethyl]-1,5-naphthyridin-2(1H)-one | ¹H-NMR (δ, ppm, CDCl₃): 8.41(d, 1H); 7.88 (d, 1 H); 7.54 (d, 1H); 7.08 (d, 2H); 7.03 (d, 1 H); 6.85 (d, 1 H); 4.31 (bt, 2H); 3.74 (s, 2H); 2.94 (bd, 2H); 2.64 (bt, 2H); 2.59-2.47 (m, 1H); 2.25 (s, 3H); 2.24 (s, 3H); 2.16 (bd, 2H); 1.91 (bd, 2H); 1.50-1.34 (bd, 3H). [ES MS] m/z 409 (MH+). | Example1 | 3-Chloro-4-methylbenzaldeh yde (Aldrich) | Intermediate 8 |
| **26** | 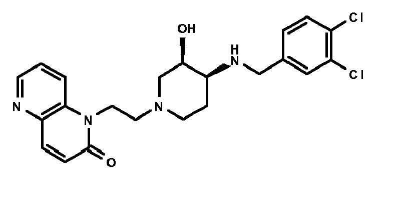 | 1-[2-((3R,4S)-4-{[(3,4-dichlorophenyl)m ethyl]amino}-3-hydroxy-1-piperidinyl)ethyl]-1,5-naphthyridin-2(1H)-one | ¹H-NMR (δ, ppm, CDCl₃): 8.54 (d, 1 H); 7.92 (d, 1 H); 7.80 (bd, 1 H); 7.49-7.44 (m, 1H); 7.28 (d, 1 H); 7.18 (bs, 1H); 7.07 (bd, 1 H); 6.91 (d,1H); 4.40 (t, 2H); 3.74 (s, 2H); 2.99 (d, 2H); 2.65 (t, 2H); 2.58-2.48 (m, 1H); 2.36 (s, 3H); 2.20 (bt, 2H); 1.90 (d, 2H); 1.52-1.35 (m, 2H). [ES MS] m/z 411 (MH+). | Example1 | 3,4-dichlorobenzalde hyde (Fluka) | Intermediate 35b |
| **27** | 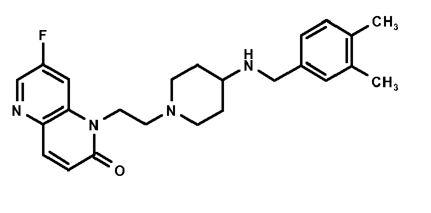 | 1-[2-(4-{[(3,4-dimethylphenyl) methyl]amino}-1-piperidinyl)ethyl]-7-fluoro-1,5-naphthyridin-2(1H)-one | ¹H-NMR (δ, ppm, CDCl₃): 8.55 (d, 1H); 7.92 (d, 1H); 7.83 (bd, 1H); 7.49-7.45 (m, 1H); 7.12 (t, 1H); 7.00-6.96 (m, 1H); 6.91 (d, 1 H); 4.41 (t, 2H); 3.77 (s, 2H); 3.00 (bd, 2H); 2.66 (t, 2H); 2.61-2.49 (m, 1H); 2.30-2.16 (m, 5H); 2.02-1.85 (m, 7H); 1.53-1.38 (m, 2H). [ES MS] m/z 395 (MH+). | Example1 | 3,4-Dimethylbenzald ehyde (Aldrich) | Intermediate 21 |
| **28** | 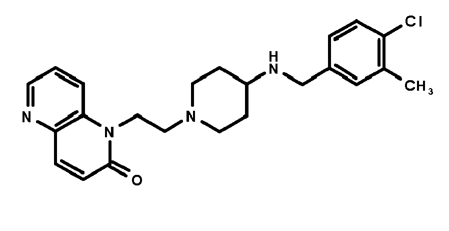 | 1-[2-(4-{[(4-chloro-3-methylphenyl)me thyl]amino}-1-piperidinyl)ethyl]-1,5-naphthyridin-2(1H)-one | ¹H-NMR (δ, ppm, CDCl3): 8.55 (d, 1 H); 7.90 (d, 1 H); 7.81 (bd, 1 H); 7.50-7.44 (m, 1H); 7.13-7.01 (m, 3H); 6.91 (d,1H); 4.39 (t, 2H); 3.75 (s, 2H); 2.97 (bd, 2H); 2.66 (t, 2H); 2.61-2.49 (m, 1H); 2.31-2.13 (m, 8H); 2.02-1.85 (m, 2H); 1.53-1.38 (m, 2H). [ES MS] m/z 391 (MH+). | Example1 | 4-Chloro-3-methylbenzaldeh yde ('Fluorochem) | Intermediate 8 |
| **29** | 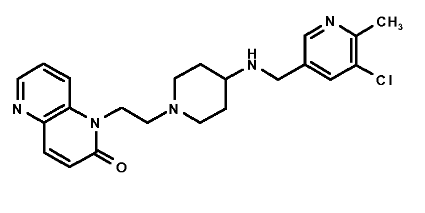 | 1-[2-(4-{[(5-chloro-6-methyl-3-pyridinyl)methyl]a mino}-1-piperidinyl)ethyl]-1,5-naphthyridin-2(1H)-one | ¹H-NMR(δ, ppm, CDCl₃): 8.41(d, 1 H); 7.88(d, 1 H); 7.55(bd, 1 H); 6.86(d, 1 H); 6.21(d, 1 H); 6.14(d, 1 H); 4.32(bt, 2H); 3.77(s, 2H); 2.96(bd, 2H); 2.65(bt, 2H); 2.58-2.44(m, 1H); 2.18(bt, 2H); 1.87(bd, 2H); 1.48-1.31 (m, 2H). [ES MS] m/z 449 (MH+). | Example1 | 5-chloro-6-methyl-3-pyridinecarbalde hyde (Described in WO20006/13748 5 A1) | Intermediate 8 |
| **30** | 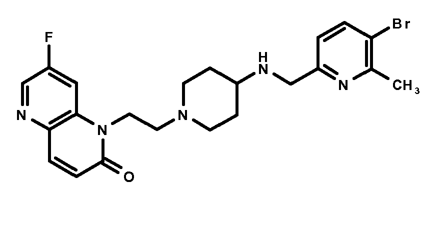 | 1-[2-(4-{[(5-bromo-6-methyl-2-pyridinyl)methyl]a mino}-1-piperidinyl)ethyl]-7-fluoro-1,5-naphthyridin-2(1H)-one | ¹H-NMR(δ, ppm, CDCl₃): 8.68(s, 1H); 8.54(dd, 1H); 7.91 (d, 1H); 7.89(d, 1 H); 7.78(bd, 1H); 7.64(d, 1H); 7.50-7.43(m, 1H); 6.92(d, 1H); 4.40(bt, 2H); 3.92(s, 2H); 2.99(bd, 2H); 2.66(bt, 2H); 2.59-2.46(m, 1H); 2.20(bt, 2H); 1.92(bd, 2H); 1.51-1.34(m, 2H). [ES MS] m/z 432 (MH+). | Example1 | 5-bromo-6-methyl-2-pyridinecarbalde hyde (Intermediate 52) | Intermediate 21 |
| **31** | 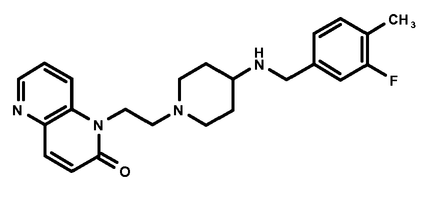 | 1-[2-(4-{[(3-fluoro-4-methylphenyl)me thyl]amino}-1-piperidinyl)ethyl]-1,5-naphthyridin-2(1H)-one | ¹H-NMR(δ, ppm, CDCl₃): 8.54(dd, 1H); 7.91 (d, 1H); 7.79(bd, 1H); 7.67(s, 1H); 7.48(d, 1H); 7.45(s, 2H); 6.92(d, 1H); 4.40(bt, 2H); 3.83(s, 2H); 2.99(bd, 2H); 2.65(bt, 2H); 2.58-2.45(m, 1H); 2.19(bt, 2H); 1.91(bd, 2H); 1.51-1.33(m, 2H). [ES MS] m/z 465. | Example1 | 3-Fluoro-4-methylbenzaldeh yde (Alfa Aesar) | Intermediate 8 |
| **32** | 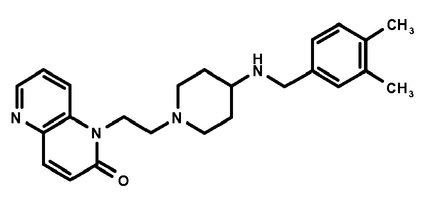 | 1-[2-(4-{[(3,4-dimethylphenyl) methyl]amino}-1-piperidinyl)ethyl]-1,5-naphthyridin-2(1H)-one | ¹H-NMR(δ, ppm, CDCl₃): 8.28(d, 1H); 8.18(d, 2H); 7.84(d, 1 H); 7.51 (d, 2H); 7.21 (bs, 1H); 6.74(d, 1H); 4.37(bt, 2H); 3.98(s, 3H); 3.93(s, 2H); 3.00(bd, 2H); 2.65(bt, 2H); 2.60-2.46(m, 1H); 2.19(bt, 2H); 1.92(bd, 2H); 1.48-1.35(m, 2H). [ES MS] m/z 438 (MH+). | Example1 | 3,4-Dimethylbenzald ehyde (Aldrich)(Aldrich) | Intermediate 8 |
| **33** | 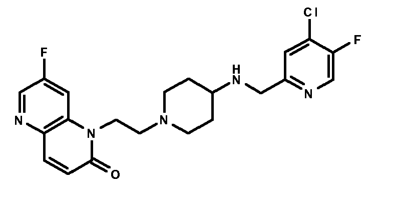 | 1-[2-(4-{[(4-chloro-5-fluoro-2-pyridinyl)methyl]a mino}-1-piperidinyl)ethyl]-7-fluoro-1,5-naphthyridin-2(1H)-one | ¹H-NMR(δ, ppm, CDCl₃): 8.41 (d, 1 H); 7.88 (d, 1 H); 7.54 (dd, 1 H); 7.44 (d, 1H); 7.27 (d, 1H); 7.17 (dd, 1H); 6.86 (d, 1 H); 4.32 (t, 2H); 3.86 (s, 2H); 2.96 (bs, 2H); 2.65 (t, 2H); 2.58-2.46 (m, 1 H); 2.20 (t, 2H); 1.92 (bd, 2H); 1.50-1.34 (m, 2H). [ES MS] m/z 449 (MH+). | Example1 | 4-chloro-5-fluoro-2-pyridinecarbalde hyde (Intermediate 48) | Intermediate 21 |
| **34** | 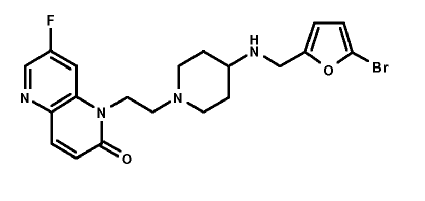 | 1-[2-(4-{[(5-bromo-2-furanyl)methyl]a mino}-1-piperidinyl)ethyl]-7-fluoro-1,5-naphthyridin-2(1 H)-one | ¹H-NMR (δ, ppm, CDCl₃): 8.31 (d, 1H); 8.27 (d, 1H); 7.85 (d, 1H); 7.72-7.69 (dd,1H); 7.56 (d,1H); 7.40 (d, 1 H); 6.65 (d, 1 H); 4.33 (t, 2H); 3.96 (s,3H); 3.68 (s, 2H); 2.92-2.88 (m, 2H); 2.35-2.26 (m, 2H); 2.05-1.97 (m, 2H); 1.77-1.73 (m,2H); 1.25-1.13 (m,2H). [ES MS] m/z 472 (MH+). | Example1 | 5-bromo-2-furancarbaldehyd e (Aldrich) | Intermediate 21 |
| **35** | 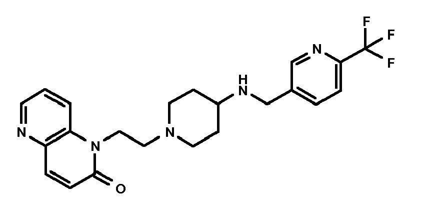 | 1-{2-[4-({[6-(trifluoromethyl)-3- pyridinyl]methyl}a mino)-1-piperidinyl]ethyl}-1,5-naphthyridin-2(1H)-one | ¹H-NMR (δ, ppm,CDCl₃): 8.42 (d, 1H); 7.89 (d, 1 H); 7.56-7.53 (m, 1 H); 7.23 (s, 1 H); 6.86 (d, 1 H); 4.35-4.30 (m, 2H); 3.77 (s, 2H); 2.99-2.95 (m, 2H); 2.68-2.63 (m, 2H); 2.55-2.46 (m, 1H); 2.23-2.15 (m, 2H); 1.93-1.88 (m, 2H); 1.47-1.33 (m, 2H). [ES MS] m/z 449 (MH+). | Example1 | 6-(trifluoromethyl)-3-pyridinecarbalde hyde (Apollo) | Intermediate 8 |
| **36** | 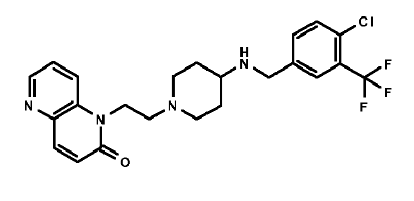 | 1-{2-[4-({[4-chloro-3-(trifluoromethyl)p henyl]methyl}ami no)-1-piperidinyl]ethyl}-1,5-naphthyridin-2(1H)-one | ¹H-NMR (δ, ppm, DMSO-d₆): 8.27 (d, 1 H); 7.85 (d, 1 H); 7.40-7.32 (m, 3H); 7.12-7.06 (m, 2H); 6.65 (d, 1H); 4.33 (t, 2H); 3.96 (s, 3H); 3.67 (s, 2H); 2.92-2.88 (m, 2H); 2.37-2.28 (m, 1 H); 2.04-1.97 (m, 2H); 1.78-1.74 (m, 2H); 1.26-1.14 (m, 2H). [ES MS] m/z 411 (MH+). | Example1 | 4-chloro-3-(trifluoromethyl)b enzaldehyde (Aldrich) | Intermediate 8 |
| **37** | 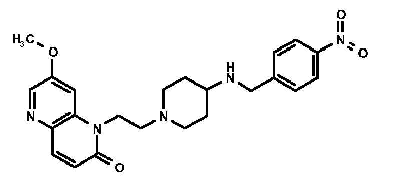 | 7-(methyloxy)-1-[2-(4-{[(4-nitrophenyl)meth yl]amino}-1-piperidinyl)ethyl]-1,5-naphthyridin-2(1H)-one | ¹H-NMR(δ, ppm, DMSO-d₆): 8.52-8.50 (m, 1H); 8.00 (d, 1H); 7.93 (d, 1H); 7.66-7.53 (m, 5H); 6.85 (d, 1H); 4.31 (t, 2H); 3.79 (s, 2H); 2.90-2.86 (m, 2H); 2.36-2.30 (m, 1 H); 2.04-1.97 (m, 2H); 1.79-1.74 (m, 2H); 1.28-1.18 (m, 2H). [ES MS] m/z 431. | Example1 | 4-nitrobenzaldehyd e (Aldrich) | Intermediate 29 |
| **38** | 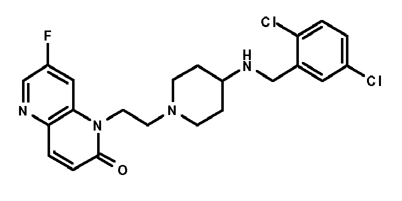 | 1-[2-(4-{[(2,5-dichlorophenyl)m ethyl]amino}-1-piperidinyl)ethyl]-7-fluoro-1,5-naphthyridin-2(1H)-one | ¹H-NMR (δ, ppm, DMSO-d₆): 8.52-8.50 (m, 1H); 8.00 (d, 1H); 7.93 (d, 1H); 7.62-7.58 (m, 1H); 7.34 (s, 4H); 6.85 (d, 1H); 4.31 (t, 2H); 3.70 (s, 2H); 2.90-2.86 (m, 2H); 2.39-2.31 (m, 1H); 2.04-1.96 (m, 2H); 1.77-1.74 (m, 2H); 1.26-1.16 (m, 2H). [ES MS] m/z 397 (MH+). | Example1 | 2,5-dichlorobenzalde hyde (Alfa Aesar) | Intermediate 21 |
| **39** | 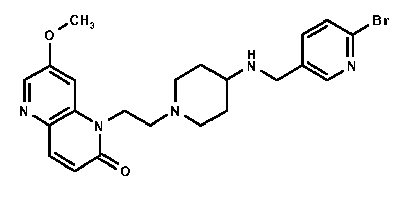 | 1-[2-(4-{[(6-bromo-3-pyridinyl)methyl]a mino}-1-piperidinyl)ethyl]-7-(methyloxy)-1,5-naphthyridin-2(1H)-one | 1 H-NMR(δ, ppm, CDCl₃): 8.42 (d, 1H); 7.88 (d, 1H); 7.55 (bd, 1H); 6.85 (d, 1 H); 6.74 (d, 1 H); 6.67 (d, 1 H), 4.32 (t, 2H); 3.92 (s, 2H); 2.95 (bd, 2H); 2.65 (t, 2H); 2.61-2.52 (m, 1 H); 2.23-2.16 (m, 2H); 1.89 (bd, 2H); 1.44-1.37 (m, 2H). [ES MS] m/z 421 (MH+). | Example1 | 6-bromo-3-pyridinecarbalde hyde (Aldrich) | Intermediate 29 |
| **40** | 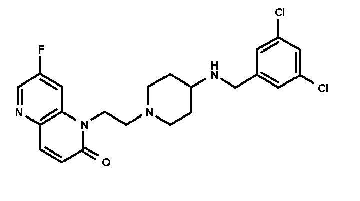 | 1-[2-(4-{[(3,5-dichlorophenyl)m ethyl]amino}-1-piperidinyl)ethyl]-7-fluoro-1,5-naphthyridin-2(1 H)-one | 1H-NMR(δ, ppm, CDCl₃): 8.42 (d, 1H); 7.96 (d, 1H); 7.88 (d, 1H); 7.68 (d, 1H); 7.55 (bd, 1H); 6.86 (d, 1H); 4.32 (t, 2H); 4.01 (s, 3H); 3.73 (s, 2H); 2.96 (bd, 2H); 2.65 (t, 2H); 2.55-2.47 (m, 1H); 2.22-2.15 (m, 2H); 1.90 (bd, 2H); 1.46-1.35 (m, 2H). [ES MS] m/z 446 (MH+). | Example1 | 3,5-dichlorobenzalde hyde (Aldrich) | Intermediate 21 |
| **41** | 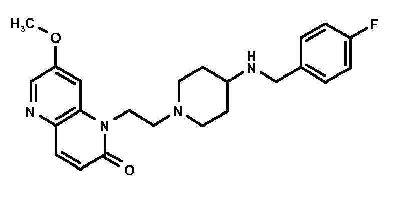 | 1-[2-(4-{[(4-fluorophenyl)met hyl]amino}-1-piperidinyl)ethyl]-7-(methyloxy)-1,5-naphthyridin-2(1H)-one | ¹H-NMR (δ, ppm, DMSO-d₆): 8.27 (d, 1H); 7.85 (d, 1H); 7.41-7.38 (m, 4H); 6.64 (d, 1 H); 4.33 (t, 2H); 3.96 (s, 3H); 3.69 (s, 2H); 2.91-2.87 (m, 2H); 2.34-2.25 (m, 1 H); 2.05-1.97 (m, 2H); 1.76-1.71 (m, 2H); 1.25-1.14 (m, 2H). [ES MS] m/z 461 (MH+). | Example1 | 4-fluorobenzaldehy de (Acros) | Intermediate 29 |
| **42** | 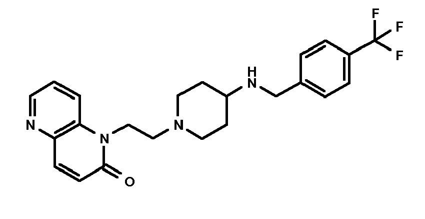 | 1-{2-[4-({[4-(trifluoromethyl)p henyl]methyl}ami no)-1-piperidinyl]ethyl}-1,5-naphthyridin-2(1H)-one | ¹H-NMR (δ, ppm, DMSO-d₆): 8.27 (d, 1H); 8.03-8.02 (m, 1H); 7.87-7.84 (m, 2H); 7.40-7.39 (m, 1H); 6.65 (d, 1H); 4.33 (t, 2H); 3.96 (s, 3H); 3.90 (s, 3H); 3.64 (s, 2H); 2.92-2.88 (m, 2H); 2.36-2.27 (m, 1H); 2.05-1.98 (m, 2H); 1.77-1.73 (m, 2H); 1.25-1.14 (m, 2H). [ES MS] m/z 458 (MH+). | Example1 | 4-(trifluoromethyl)b enzaldehyde (Aldrich) | Intermediate 8 |
| **43** | 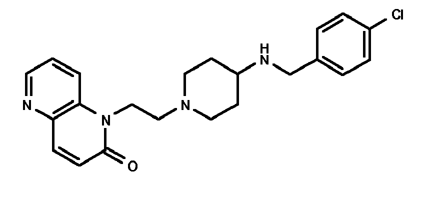 | 1-[2-(4-{[(4-chlorophenyl)met hyl]amino}-1-piperidinyl)ethyl]-1,5-naphthyridin-2(1H)-one | 1H-NMR(δ, ppm, CDCl₃): 8.55 (dd, 1H); 7.91 (d, 1H); 7.79 (bd, 1H); 7.46 (dd, 1H); 6.92 (d, 1H); 6.88 (d, 1H); 6.66 (d, 1H); 4.39 (t, 2H); 3.94 (s, 2H); 2.98 (bd, 2H); 2.65 (t, 2H); 2.57 (bs, 1H); 2.23-2.16 (m, 2H); 1.90 (bd, 2H); 1.47-1.35 (m, 2H). [ES MS] m/z 447 (MH+). | Example1 | 4-chlorobenzaldeh yde (Fluka) | Intermediate 8 |
| **44** | 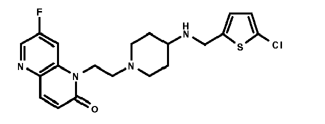 | 1-[2-(4-{[(5-chloro-2- thienyl)methyl]a mino}-1-piperidinyl)ethyl]-7-fluoro-1,5-naphthyridin-2(1H)-one | ¹H-NMR (δ, ppm, CDCl₃): 8.55 (dd, 1H); 7.91 (d, 1H); 7.79 (bd, 1H); 7.49-7.44 (m, 1H); 7.37 (s, 1H); 6.92 (d, 1H); 4.40 (bt, 2H); 3.98 (s, 2H); 2.96 (bd, 2H); 2.66 (bt, 2H); 2.60-2.47 (m, 1H); 2.18 (bt, 2H); 1.87 (bd, 2H); 1.47-1.31 (m, 2H). [ES MS] m/z 448 (MH+). | Example1 | 5-chloro-2-thiophenecarbald ehyde (Aldrich) | Intermediate 21 |
| **45** | 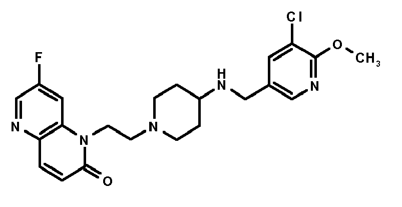 | 1-{2-[4-({[5-chloro-6-(methyloxy)-3-pyridinyl]methyl}a mino)-1-piperidinyl]ethyl}-7-fluoro-1,5-naphthyridin-2(1H)-one | ¹H-NMR (δ, ppm, CDCl₃): 8.26 (d, 1H); 7.85 (d, 1H); 7.21 (s, 1H); 6.76-6.72 (m, 2H); 4.36 (bt, 2H); 3.98 (s, 3H); 3.93 (s, 2H); 2.98 (bd, 2H); 2.65 (bt, 2H); 2.60-2.49 (m, 1H); 2.19 (bt, 2H); 1.88 (bd, 2H); 1.48-1.31 (m, 2H). [ES MS] m/z 555 (MH+). | Example1 | 5-chloro-6-(methyloxy)-3-pyridinecarbalde hyde (Asymchem) | Intermediate 21 |
| **46** | 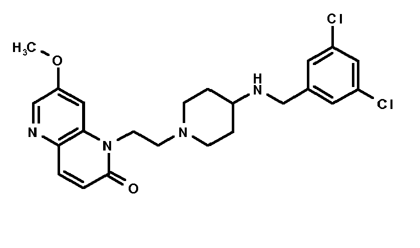 | 1-[2-(4-{[(3,5-dichlorophenyl)m ethyl]amino}-1-piperidinyl)ethyl]-7-(methyloxy)-1,5-naphthyridin-2(1H)-one | ¹H-NMR (δ, ppm, CDCl₃): 8.55 (dd, 1H); 7.91 (d, 1H); 7.79 (bd, 1H); 7.49-7.44 (m, 1H); 7.34 (d, 1H); 7.17 (dd, 1H); 6.91 (d, 1 H); 6,87 (d, 1 H); 4.40 (t, 2H); 3.89 (s, 3H); 3.73 (s, 2H); 2.96 (bd, 2H); 2.65 (t, 2H); 2.59-2.45 (m, 1H); 2.19 (bt, 2H); 1.89 (bd, 2H); 1.52-1.33 (m, 2H). [ES MS] m/z 427 (MH+). | Example1 | 3,5-dichlorobenzalde hyde (Aldrich) | Intermediate 29 |
| **47** | 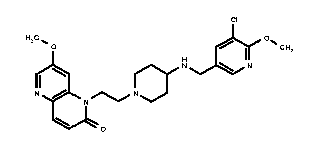 | 1-{2-[4-({[5-chloro-6-(methyloxy)-3-pyridinyl]methyl}a mino)-1-piperidinyl]ethyl}-7-(methyloxy)-1,5-naphthyridin-2(1H)-one | ¹H-NMR(δ, ppm, CDCl₃): 8.55 (dd, 1 H); 7.91 (d, 1 H); 7.78 (bd, 1 H); 7.61 (d, 1H); 7.55 (d, 1H); 7.49-7.44 (m, 1H); 7.13 (dd, 1H); 6.92 (d, 1H); 4.39 (t, 2H); 3.75 (s, 2H); 2.98 (bd, 2H); 2.65 (t, 2H); 2.58-2.44 (m, 1H); 2.19 (bt, 2H); 1.87 (bd, 2H); 1.49-1.32 (m, 2H). [ES MS] m/z 519 (MH+). | Example1 | 5-chloro-6-(methyloxy)-3-pyridinecarbalde hyde (Asymchem) | Intermediate 29 |
| **48** | 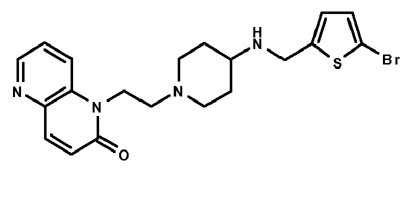 | 1-[2-(4-{[(5-bromo-2-thienyl)methyl]a mino}-1-piperidinyl)ethyl]-1,5-naphthyridin-2(1 H)-one | ¹H-NMR (δ, ppm, CDCl₃): 8.28 (d, 1H); 7.84 (d, 1H); 7.61 (d, 2H); 7.45 (d, 2H); 7.21 (s, 1H); 6.74 (d, 1H); 4.37 (bt, 2H); 3.98 (s, 3H); 3.88 (s, 2H); 2.97 (bd, 2H); 2.65 (bt, 2H); 2.59-2.45 (m, 1H); 2.19 (bt, 2H); 1.89 (bd, 2H); 1.52-1.34 (m, 2H). [ES MS] m/z 418 (MH+). | Example1 | 5-bromo-2-thiophenecarbald ehyde (Aldrich) | Intermediate 8 |
| **49** | 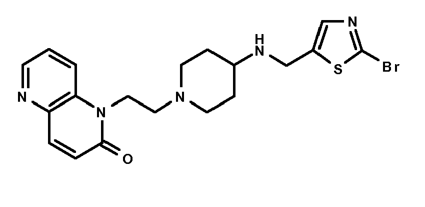 | 1-[2-(4-{[(2-bromo-1,3-thiazol-5-yl)methyl]amino}-1-piperidinyl)ethyl]-1,5-naphthyridin-2(1H)-one | ¹H-NMR (δ, ppm, DMSO-d₆): 8.27 (d, 1 H); 7.86 (bd, 1 H); 7.40-7.39 (m, 1 H); 7.00 (d, 1 H); 6.77 (bd, 1 H); 6.65 (d, 1 H); 4.33 (t, 2H); 3.97 (s, 3H); 3.84 (s, 2H); 2.92-2.88 (m, 2H); 2.43-2.32 (m, 1H); 2.05-1.98 (m, 2H); 1.77-1.73 (m, 2H); 1.24-1.14 (m, 2H). [ES MS] m/z 477 (MH+). | Example1 | 2-bromo-1,3-thiazole-5-carbaldehyde (Combi-Blocks) | Intermediate 8 |
| **50** | 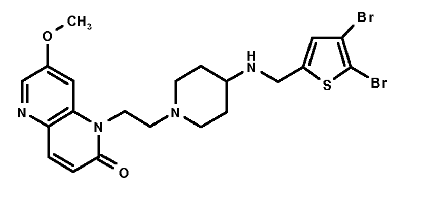 | 1-[2-(4-{[(4,5-dibromo-2-thienyl)methyl]a mino}-1-piperidinyl)ethyl]-7-(methyloxy)-1,5-naphthyridin-2(1 H)-one | ¹H-NMR(δ, ppm, DMSO-d₆): 8.52-8.50 (m, 1H); 7.99 (d, 1H); 7.92 (d, 1H); 7.62-7.58 (m, 1H); 6.94 (s, 1 H); 6.84 (d, 1H); 4.31 (t, 2H); 3.83 (s, 2H); 2.89-2.86 (m, 2H); 2.38-2.31 (m, 1 H); 2.04-1.97 (m, 2H); 1.76-1.71 (m, 2H); 1.24-1.13 (m, 2H). [ES MS] m/z 525 (MH+). | Example1 | 4,5-dibromo-2-thiophenecarbald ehyde (Acros) | Intermediate 29 |
| **51** | 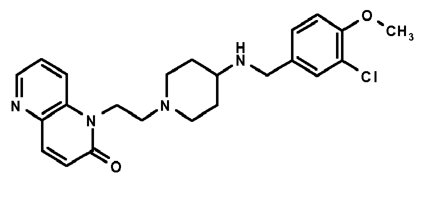 | 1-{2-[4-({[3-chloro-4-(methyloxy)phen yl]methyl}amino)-1-piperidinyl]ethyl}-1,5-naphthyridin-2(1H)-one | 1H-NMR (δ, ppm, CDCl₃): 8.42 (d, 1H); 7.89 (d, 1H); 7.55 (bs, 1H); 6.86 (d, 1 H); 6.60 (s, 1 H); 4.33 (bs, 2H); 3.89 (s, 2H); 2.97-2.95 (m, 2H); 2.68-2.63 (m, 2H); 2.57 (bs, 1H); 2.23-2.18 (m, 2H); 2.13 (s, 3H); 1.91-1.87 (m, 2H); 1.41 (bs, 2H). [ES MS] m/z 479 (MH+). | Example1 | 3-chloro-4-(methyloxy)benz aldehyde (Aldrich) | Intermediate 8 |
| **52** | 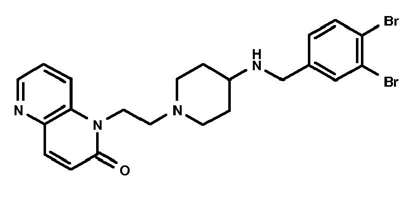 | 1-[2-(4-{[(3,4-dibromophenyl)m ethyl]amino}-1-piperidinyl)ethyl]-1,5-naphthyridin-2(1H)-one | ¹H-NMR (δ, ppm, CDCl₃): 8.55 (dd, 1H); 7.92 (d, 1H); 7.81 (bd, 1H); 7.49-7.45 (m, 1H); 7.33 (s, 1H); 7.28-7.18 (m, 3H); 6.92 (d, 1H); 4.40 (bt, 2H); 3.79 (s, 2H); 2.97 (bd, 2H); 2.66 (bt, 2H); 2.59-2.46 (m, 1 H); 2.20 (bt, 2H); 1.89 (bd, 2H); 1.53-1.35 (m, 2H). [ES MS] m/z 397 (MH+). | Example1 | 3,4-dibromobenzalde hyde (Alfa Aesar) | Intermediate 8 |
| **53** | 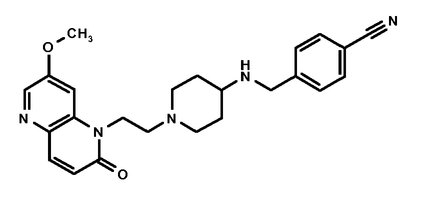 | 4-{[(1-{2-[7-(methyloxy)-2-oxo-1,5-naphthyridin-1 (2H)-yl]ethyl}-4-piperidinyl)amino ]methyl}benzonitr]methyl}benzonitrile | 1H-NMR(δ, ppm, CDCl₃): 8.55 (dd, 1H); 7.92 (d, 1H); 7.79 (bd, 1H); 7.47 (dd, 1H); 7.27-7.23 (m, 3H); 6.92 (d, 1H); 4.39 (t, 2H); 3.77 (s, 2H); 2.98 (bd, 2H); 2.65 (t, 2H); 2.54-2.47 (m, 1H); 2.23-2.16 (m, 2H); 1.90 (bd, 2H); 1.47-1.34 (m, 2H). [ES MS] m/z 431 (MH+). | Example1 | 4-formylbenzonitril e (Fluka) | Intermediate 29 |
| **54** | 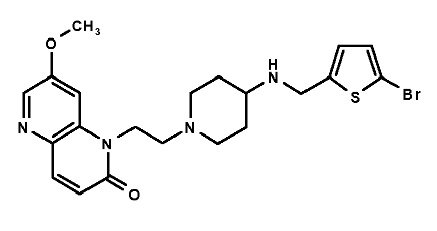 | 1-[2-(4-{[(5-bromo-2-thienyl)methyl]a mino}-1-piperidinyl)ethyl]-7-(methyloxy)-1,5-naphthyridin-2(1 H)-one | 1H-NMR(δ, ppm, CDCl₃): 8.42 (d, 1H); 7.95 (d, 1H); 7.88 (d, 1H); 7.54 (bd, 1 H); 7.48 (d, 1 H); 7.28 (d, 1H); 6.85 (d, 1 H); 4.32 (t, 2H); 3.85 (s, 2H); 2.96 (bd, 2H); 2.65 (t, 2H); 2.57 (s, 3H); 2.55-2.48 (m, 1 H); 2.22-2.15 (m, 2H); 1.90 (bd, 2H); 1.47-1.35 (m, 2H). [ES MS] m/z 440 (MH+). | Example1 | 5-bromo-2-thiophenecarbald ehyde (Aldrich) | Intermediate 29 |
| **55** | 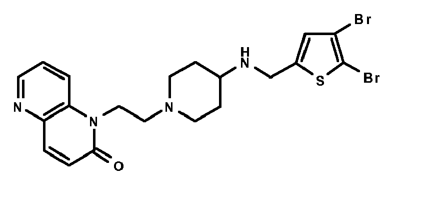 | 1-[2-(4-{[(4,5-dibromo-2-thienyl)methyl]a mino}-1-piperidinyl)ethyl]-1,5-naphthyridin-2(1H)-one | 1H-NMR(δ, ppm, CDCl₃): 8.42 (d, 1H); 7.89 (d, 1H); 7.53 (bd, 1H); 7.16 (s, 1H); 6.86 (d, 1H); 4.32 (t, 2H); 4.12 (s, 2H); 2.96 (bd, 2H); 2.65 (t, 2H); 2.60-2.54 (m, 1H); 2.22-2.14 (m, 2H); 1.92 (bd, 2H); 1.47-1.36 (m, 2H). [ES MS] m/z 466 (MH+). | Example1 | 4,5-dibromo-2-thiophenecarbald ehyde (Acros) | Intermediate 8 |
| **56** | 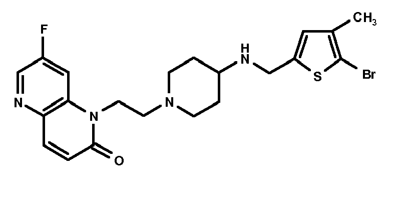 | 1-[2-(4-{[(5-bromo-4-methyl-2-thienyl)methyl]a mino}-1-piperidinyl)ethyl]-7-fluoro-1,5-naphthyridin-2(1H)-one | ¹H-NMR (δ, ppm, CDCl₃): 8.41 (d, 1H); 7.88 (d, 1H); 7.53 (dd, 1H); 7.31 (d, 1 H); 7.18-7.08 (m, 2H); 6.85 (d, 1H); 4.31 (t, 2H); 3.75 (s, 2H); 2.95 (bd, 2H); 2.64 (t, 2H); 2.58-2.45 (m, 1H); 2.35 (s, 3H); 2.18 (bt, 2H); 1.87 (bd, 2H); 1.49-1.31 (m, 2H). [ES MS] m/z 429 (MH+). | Example1 | 5-bromo-4-methyl-2-thiophenecarbald ehyde (FRONTIER) | Intermediate 21 |
| **57** | 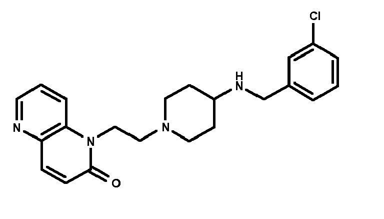 | 1-[2-(4-{[(3-chlorophenyl)met hyl]amino}-1-piperidinyl)ethyl]-1,5-naphthyridin-2(1H)-one | 1H-NMR(δ, ppm, CDCl₃): 8.42 (d, 1H); 7.88 (d, 1H); 7.83 (d, 1H); 7.53 (dd, 1 H); 7.38 (dd, 1H); 6.86 (d, 1H); 4.31 (t, 2H); 4.01 (s, 3H); 3.75 (s, 2H); 2.95 (bd, 2H); 2.64 (t, 2H); 2.55-2.45 (m, 1 H); 2.22-2.14 (m, 2H); 1.89 (bd, 2H); 1.45-1.32 (m, 2H). [ES MS] m/z 430 (MH+). | Example1 | 3-chlorobenzaldeh yde (Aldrich) | Intermediate 8 |
| **58** | 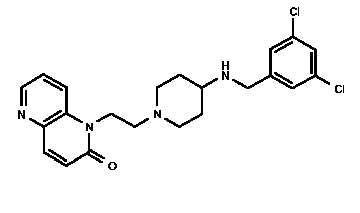 | 1-[2-(4-{[(3,5-dichlorophenyl)m ethyl]amino}-1-piperidinyl)ethyl]-1,5-naphthyridin-2(1H)-one | 1 H-NMR(δ, ppm, CDCl₃): 8.28 (d, 1H); 7.83 (d, 1H); 7.23 (d, 1H); 6.73 (d, 1H); 6.22 (d, 1H); 6.15 (d, 1H); 4.36 (t, 2H); 3.97 (s, 3H); 3.77 (s, 2H); 2.98 (bd, 2H); 2.64 (t, 2H); 2.56-2.46 (m, 1H); 2.18 (t, 2H); 1.87 (bd, 2H); 1.47-1.34 (m, 2H). [ES MS] m/z 461 (MH+). | Example1 | 3,5-dichlorobenzalde hyde (Aldrich) | Intermediate 8 |
| **59** | 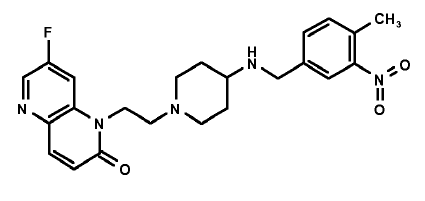 | 7-fluoro-1-[2-(4-{[(4-methyl-3-nitrophenyl)meth yl]amino}-1-piperidinyl)ethyl]-1,5-naphthyridin-2(1 H)-one | ¹H-NMR (δ, ppm, CDCl₃): 8.28 (d, 1H); 7.85 (d, 1H); 7.20 (d, 1H); 7.16 (s, 1H); 6.74 (d, 1H); 4.36 (t, 2H); 4.12 (s, 2H); 3.98 (s, 3H); 2.96 (bd, 2H); 2.65 (t, 2H); 2.62-2.52 (m, 1H); 2.18 (t, 2H); 1.90 (bd, 2H); 1.50-1.32 (m, 2H). [ES MS] m/z 478 (MH+). | Example1 | 4-methyl-3-nitrobenzaldehyd e (Alfa Aesar) | Intermediate 21 |
| **60** | 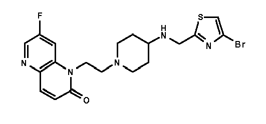 | 1-[2-(4-{[(4-bromo-1,3-thiazol-2-yl)methyl]amino}-1-piperidinyl)ethyl]-7-fluoro-1,5-naphthyridin-2(1H)-one | 1H-NMR(δ, ppm, CDCl₃): 8.55 (dd, 1 H); 7.90 (d, 1 H); 7.78 (d, 1 H); 7.46 (dd, 1 H); 6.91 (d, 1 H); 6.59 (s, 1 H); 4.38 (t, 2H); 3.89 (s, 2H); 2.96 (bd, 2H); 2.64 (t, 2H); 2.59-2.52 (m, 1 H); 2.19 (t, 2H); 2.13 (s, 3H); 1.88 (bd, 2H); 1.50-1.33 (m, 2H). [ES MS] m/z 461 (MH+). | Example1 | 4-bromo-1,3-thiazole-2-carbaldehyde (Frontier) | Intermediate 21 |
| **61** | 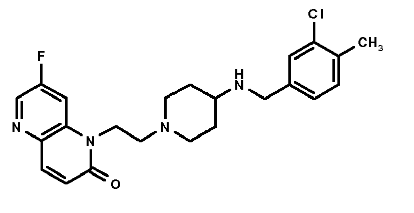 | 1-[2-(4-{[(3-chloro-4-methylphenyl)me thyl]amino}-1-piperidinyl)ethyl]-7-fluoro-1,5-naphthyridin-2(1H)-one | 1H-NMR(δ, ppm, CDCl₃): 8.28 (d, 1H); 7.86-7.83 (m, 2H); 7.38 (dd, 1H); 7.27-7.21 (m, 1H); 6.74 (d, 1H); 4.36 (t, 2H); 4.01 (s, 3H); 3.98 (s, 3H); 3.75 (s, 2H); 2.98 (d, 2H); 2.65 (t, 2H); 2.56-2.46 (m, 1H); 2.18 (t, 2H); 1.90 (bd, 2H); 1.47-1.34 (m, 2H). [ES MS] m/z 442 (MH+). | Example1 | 3-chloro-4-methylbenzaldeh yde (Aldrich) | Intermediate 21 |
| **62** | 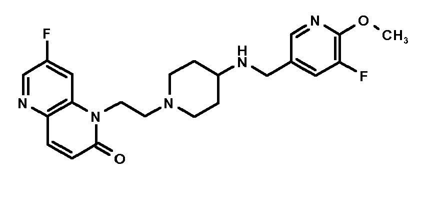 | 7-fluoro-1-{2-[4-({[5-fluoro-6-(methyloxy)-3-pyridinyl]methyl}a mino)-1-piperidinyl]ethyl}-1,5-naphthyridin-2(1H)-one | 1H-NMR(δ, ppm, CDCl₃): 8.28 (d, 1 H); 8.07 (d, 1 H); 7.84 (d, 1 H); 7.57 (dd, 1H); 7.23 (bs, 1H); 6.76-6.71 (m, 2H); 4.37 (bd, 2H); 3.98 (s, 3H); 3.93 (s, 3H); 3.74 (s, 2H); 2.99 (bd, 2H); 2.65 (bt, 2H); 2.54 (bs, 1H); 2.19 (bt, 2H); 1.91 (bd, 2H); 1.51-1.41 (m, 2H). [ES MS] m/z 424 (MH+). | Example1 | 5-fluoro-6-(methyloxy)-3-pyridinecarbalde hyde (Asychem) | Intermediate 21 |
| **63** | 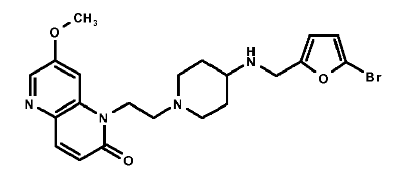 | 1-[2-(4-{[(5-bromo-2-furanyl)methyl]a mino}-1-piperidinyl)ethyl]-7-(methyloxy)-7-(methyloxy)-1,5-naphthyridin- 2(1H)-one | 1H-NMR(δ, ppm, CDCl₃): 8.27 (d, 1H); 7.84 (d, 1H); 7.29-7.23 (m, 2H); 7.15-7.11 (m, 1H); 6.73 (d, 1H); 4.38 (t, 2H); 3.98 (s, 3H); 3.87 (s, 2H); 3.01 (bd, 2H); 2.66 (t, 2H); 2.56 (bs, 1 H); 2.50 (s, 3H); 2.20 (bs, 2H); 1.94 (bd, 2H); 1.53-1.41 (m, 2H). [ES MS] m/z 426 (MH+). | Example1 | 5-bromo-2-furancarbaldehyd e (Aldrich) | Intermediate 29 |
| **64** | 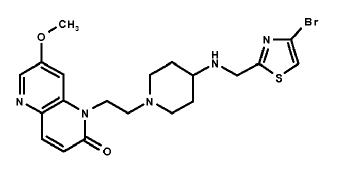 | 1-[2-(4-{[(4-bromo-1,3-thiazol-2-yl)methyl]amino}-1-piperidinyl)ethyl]-7-(methyloxy)-1,5-naphthyridin-2(1H)-one | 1H-NMR(δ, ppm, CDCl₃): 8.27 (d, 1H); 7.84 (d, 1H); 7.31-7.08 (m, 4H); 6.74 (d, 1H); 4.36 (t, 2H); 3.97 (s, 3H); 3.75 (s, 2H); 2.98 (bd, 2H); 2.64 (t, 2H); 2.55-2.47 (m, 1 H); 2.35 (s, 3H); 2.18 (bt, 2H); 1.90 (bd, 2H); 1.52-1.36 (m, 2H). [ES MS] m/z 441 (MH+). | Example1 | 4-bromo-1,3-thiazole-2-carbaldehyde (Frontier) | Intermediate 29 |
| **65** | 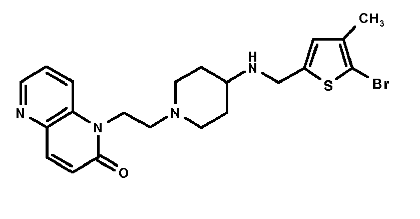 | 1-[2-(4-{[(5-bromo-4-methyl-2-thienyl)methyl]a mino}-1-piperidinyl)ethyl]-1,5-naphthyridin-2(1H)-one | ¹H-NMR(δ, ppm, CDCl₃): 8.28 (d, 1H); 7.93 (s, 1 H); 7.84 (d, 1 H); 7.62-7.58 (m, 1H); 7.23-7.19 (m, 1H); 6.74 (d, 1H), 4.40-4.36 (m, 2H); 3.98 (s, 3H); 3.77 (s, 2H); 3.03-2.99 (m, 2H); 2.68-2.64 (m, 2H); 2.56-2.49 (m, 1 H); 2.28 (s, 3H); 2.24-2.18 (m, 2H); 1.95-1.90 (m, 2H), 1.49-1.41 (m, 2H). [ES MS] m/z 426(MH+) | Example1 | 5-bromo-4-methyl-2-thiophenecarbald ehyde (Frontier) | Intermediate 8 |
| **66** | 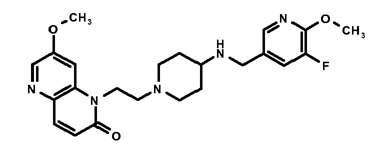 | 1-{2-[4-({[5-fluoro-6-(methyloxy)-3-pyridinyl]methyl}a mino)-1-piperidinyl]ethyl}-7-(methyloxy)-1,5-naphthyridin-2(1H)-one | ¹H-NMR(δ, ppm, CDCl₃): 8.28 (d, 1H); 7.85 (d, 1 H); 7.25 (d, 1 H); 6.96-6.87 (m, 3H); 6.75 (d, 1H), 4.38 (bt, 2H); 3.98 (s, 3H) 3.74 (s, 2H); 3.04-2.94 (m, 2H); 2.65 (bt, 2H); 2.61-2.50 (m,1H); 2.31 (s, 6H); 2.20 (dt, 2H); 1.94 (bd, 2H); 1.53-1.36 (m, 2H). [ES MS] m/z 421 (MH+). | Example1 | 5-fluoro-6-(methyloxy)-3-pyridinecarbalde hyde (Asymchem) | Intermediate 29 |
| **67** | 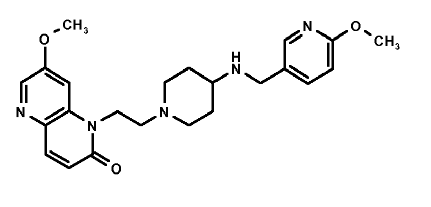 | 7-(methyloxy)-1-{2-[4-({[6-(methyloxy)-3-pyridinyl]methyl}a mino)-1-piperidinyl]ethyl}-1,5-naphthyridin-2(1 H)-one | ¹H-NMR(δ, ppm, CDCl₃): 8.28 (d, 1H); 7.84 (d, 1H); 7.25 (d, 1H); 7.12-7.01 (m, 3H); 6.74 (d, 1 H), 4.37 (bt, 2H); 3.98 (s, 3H) 3.75 (s, 2H); 3.04-2.93 (m, 2H); 2.65 (bt, 2H); 2.61-2.49 (m, 1H); 2.26 (s, 3H); 2.25 (s, 3H); 2.19 (dt, 2H); 1.93 (bd, 2H); 1.52-1.35 (m, 2H). [ES MS] m/z 421 (MH+). | Example1 | 6-(methyloxy)-3-pyridinecarbalde hyde (Aldrich) | Intermediate 29 |
| **68** | 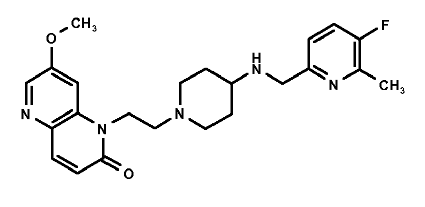 | 1-[2-(4-{[(5-fluoro-6-methyl-2-pyridinyl)methyl]a mino}-1-piperidinyl)ethyl]-7-(methyloxy)-1,5-naphthyridin-2(1H)-one | ¹H-NMR (δ, ppm, CDCl₃): 8.28 (d, 1H); 7.84 (d, 1H); 7.25-7.23 (m, 1H); 6.90-6.77 (m, 3H); 6.74 (d, 1H); 4.37 (t, 2H); 3.98 (s, 3H); 3.76 (s, 2H); 2.99 (bd, 2H); 2.65 (t, 2H); 2.56-2.48 (m, 1H); 2.33 (s, 3H); 2.19 (bt, 2H); 1.91 (bd, 2H); 1.49-1.36 (m, 2H). [ES MS] m/z 425 (MH+). MS] m/z 425 (MH+). | Example1 | 5-fluoro-6-methyl-2-pyridinecarbalde hyde (Asymchem) | Intermediate 29 |
| **69** | 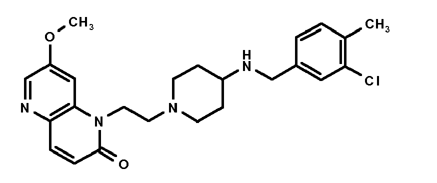 | 1-[2-(4-{[(3-chloro-4-methylphenyl)me thyl]amino}-1-piperidinyl)ethyl]-7-(methyloxyl-7-(methyloxy)-1,5-naphthyridin-2(1H)-one | ¹H-NMR(δ, ppm, CDCl₃): 8.28 (d, 1H); 7.85 (d, 1H); 7.25 (bd, 1H); 7.14-7.09 (m, 1H); 7.00-6.96 (m, 2H); 6.74 (d, 1H); 4.37 (t, 2H); 3.98 (s, 3H); 3.77 (s, 2H); 2.99 (bd, 2H); 2.65 (t, 2H); 2.58-2.48 (m, 1H); 2.25 (d, 3H); 2.18 (bd, 2H); 1.91 (bd, 2H); 1.49-1.36 (m, 2H). [ES MS] m/z 425 (MH+). | Example1 | 3-chloro-4-3-chloro-4-methylbenzaldeh yde (Aldrich) | Intermediate 29 Intermediate 29 |
| **70** | 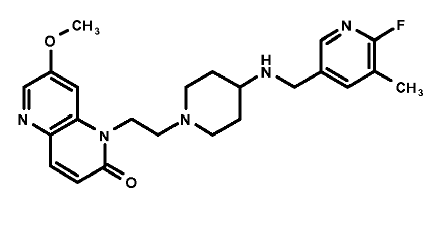 | 1-[2-(4-{[(6-fluoro-5-methyl-3-pyridinyl)methyl]a mino}-1-piperidinyl)ethyl]-7-(methyloxy)-1,5-naphthyridin-2(1H)-one | ¹H-NMR (δ, ppm, CDCl₃): 8.42 (d, 1H); 7.88 (d, 1H); 7.88 (d, 1H); 7.55 (dd, 1H); 7.28 (bs, 1H); 7.18 (bs, 1H); 7.07 (bdd, 1H); 6.86 (d, 1H); 4.31 (t, 2H); 3.74 (s, 2H); 2.95 (bd, 2H); 2.64 (t, 2H); 2.56-2.46 (m, 1H); 2.18 (t, 2H); 1.90 (bd, 2H); 1.47-1.34 (m, 2H). [ES MS] m/z 429 (MH+). | Example1 | 6-fluoro-5-methyl-3-pyridinecarbalde hyde (Asymchem) | Intermediate 29 |
| **71** | 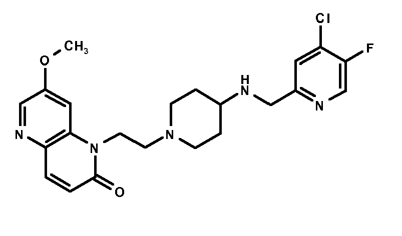 | 1-[2-(4-{[(4-chloro-5-fluoro-2-pyridinyl)methyl]a mino}-1-piperidinyl)ethyl]-7-(methyloxy)-1,5-naphthyridin-2(1H)-one | ¹H-NMR (δ, ppm, CDCl₃): 8.47 (bd, 1 H); 8.42 (d, 1 H); 7.90 (bs, 1 H); 7.90 (bs, 1H); 7.87 (bs, 1H); 7.54 (dd, 1H); 6.86 (d, 1H); 4.32 (t, 2H); 3.82 (s, 2H); 2.96 (bd, 2H); 2.65 (t, 2H); 2.57-2.47 (m, 1 H); 2.19 (bt, 2H); 1.91 (bd, 2H); 1.46-1.34 (m, 2H). [ES MS] m/z 460 (MH+). | Example1 | 4-chloro-5-fluoro-2-pyridinecarbalde hyde (Intermediate 48) | Intermediate 29 |
| **72** | 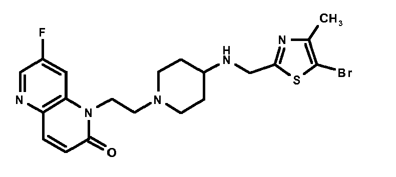 | 1-[2-(4-{[(5-bromo-4-methyl-1,3-thiazol-2-yl)methyl]amino}-1-piperidinyl)ethyl]-piperidinyl)ethyl]-7-fluoro-1,5-naphthyridin-2(1H)-one | ¹H-NMR(δ, ppm, CDCl₃): 8.54 (d, 1H); 7.91 (d, 1 H); 7.80 (m, 1 H); 7.47 (dd, 1H); 7.10 (s, 1 H); 6.91 (d, 1 H); 6.83 (s, 1H); 4.40 (t, 2H); 3.97 (s, 2H); 2.98 (m, 2H); 2.66 (m, 2H); 2.21 (m, 2H); 1.90 (bd, 2H); 1.25-1.70 (m, 4H). [ES MS] m/z 447 (MH+). | Example7 | 5-bromo-4-methyl-1,3-thiazole-2-carbaldehyde (Intermediate 55) | Intermediate 21 |
| **73** | 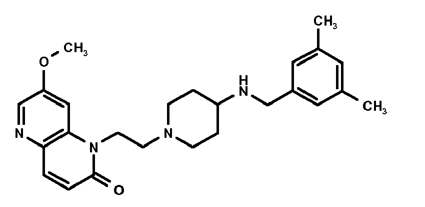 | 1-[2-(4-{[(3,5-Dimethylphenyl) methyl]amino}-1-piperidinyl)ethyl]-7-(methyloxy)-1,5-naphthyridin-2(1 H)-one | ¹H-NMR (δ, ppm, CDCl₃): 8.41 (d, 1H); 7.88 (d, 1H); 7.61 (d, 1H); 7.54 (d, 2H); 7.13 (dd, 1H); 6.85 (d, 1H); 4.32 (bt, 2H); 3.75 (s, 2H); 2.96 (bd, 2H); 2.65 (bt, 2H); 2.57-2.44 (m, 1 H); 2.19 (bt, 2H); 1.89 (bd, 2H); 1.47-1.31 (m, 2H). [ES MS] m/z 537 (MH+). | Example8 | 3,5-Dimethylbenzald ehyde (Alfa Aesar) | Intermediate 29 |
| **74** | 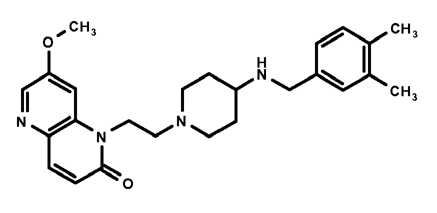 | 1-[2-(4-{[(3,4-Dimethylphenyl) methyl]amino}-1-piperidinyl)ethyl]-7-(methyloxy)-1,5-naphthyridin-2(1 H)-one | 1H-NMR(δ, ppm, CDCl₃): 8.42 (d, 1H); 7.88 (d, 1H); 7.55 (bd, 1H); 6.87 (d, 1H); 6.85 (d, 1H); 6.66 (d, 1H); 4.33 (t, 2H); 3.94 (s, 2H); 2.96 (bd, 2H); 2.66 (t, 2H); 2.57 (bs, 1 H); 2.23-2.17 (m, 2H); 1.90 (bd, 2H); 1.45-1.35 (m, 2H). [ES MS] m/z 465 (MH+). | Example8 | 3,4-Dimethylbenzald ehyde (Aldrich) | Intermediate 29 |
| **75** | 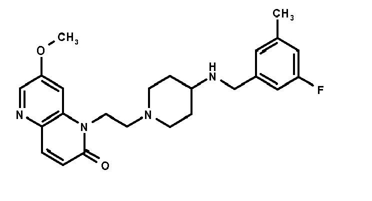 | 1-[2-(4-{[(3-fluoro-5-methylphenyl)me thyl]amin o}-1-piperidinyl)ethyl]-7-(methyloxy)-1,5-naphthyridin-2(1H)-one | 1H-NMR (δ, ppm, CDCl₃): 8.41 (d, 1H); 7.88 (d, 1H); 7.61-7.51 (m, 3H); 7.45 (d, 2H); 6.86 (d, 1H); 4.33 (bt, 2H); 3.87 (s, 2H); 2.95 (bd, 2H); 2.66 (bt, 2H); 2.60-2.48 (m, 1 H); 2.20 (bt, 2H); 1.90 (bd, 2H); 1.49-1.36 (m, 2H). [ES MS] m/z 449 (MH+). | Example8 | 3-fluoro-5-methylbenzaldeh yde (Apollo) | Intermediate 29 |
| **76** | 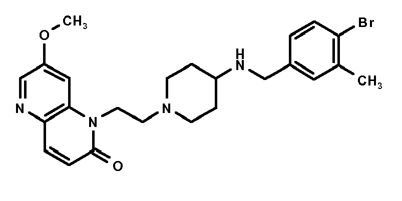 | 1-[2-(4-{[(4-bromo-3-methylphenyl)me thyl]amino}-1-piperidinyl)ethyl]-7-(methyloxy)-1,5-naphthyridin-2(1H)-one | ¹H-NMR (δ, ppm, CDCl₃): 8.41 (d, 1H); 7.88 (d, 1H); 7.55 (bd, 1H); 7.21-6.99 (m, 3H); 6.85 (d, 1H); 4.32 (t, 2H); 3.76 (s, 2H); 2.96 (bd, 2H); 2.65 (t, 2H); 2.57-2.45 (m, 1H); 2.19 (bt, 2H); 1.90 (bd, 2H); 1.48-1.33 (m, 2H). [ES MS] m/z 417 (MH+). | Example8 | 4-bromo-3-methylbenzaldeh yde (Intermediate 64) | Intermediate 29 |
| **77** | 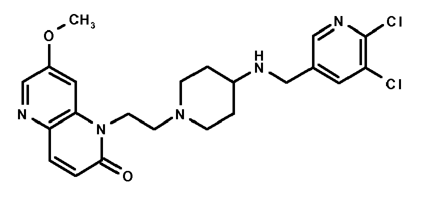 | 1-[2-(4-{[(5,6-dichloro-3-pyridinyl)methyl]a mino}-1-piperidinyl)ethyl]-7-(methyloxy)-1,5-naphthyridin-2(1H)-one | ¹H-NMR(δ, ppm, CDCl₃):8,28(d,1H);7,85(d, 1H);7,61 (d, 1H);7,54(d,1H);7,11(dd,1H);6,75(d,1 H);4,40(bs,2H);3,99(s,3H);3,75(s,2H); 3,01 (bs,2H);2,68(bs,2H);2,54(bs,1H); 2,25(bs,2H);1,90(bs,2H);1,47(bs,2H);[ ES MS] m/z 549 (MH+) | Example8 | 5,6-dichloro-3-pyridinecarbalde hyde (Intermediate 58) | Intermediate 29 |
| **78** | 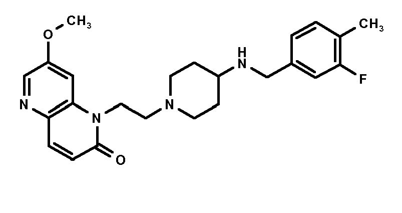 | 1-[2-(4-{[(3-fluoro-4-methylphenyl)me thyl]amino}-1-piperidinyl)ethyl]-7-(methyloxy)-1,5-naphthyridin-2(1H)-one | ¹H-NMR(δ, ppm, CDCl₃):8,28(d,1H);7,85(d,1H);7,58(d, 2H);7,45(d,2H);7,32(bs,1H);6,73(d,1H );4,43(bs, 2H);4,08(s,3H);3,87(s,2H);3,06(bs,2H );2,71 (bs,2H);2,59(bs,1H);2,25(bs,2H );1,95(bs,2H);1,39-1,58(m,2H).([ES MS] m/z 461 (MH+) | Example8 | 3-fluoro-5-methylbenzaldeh yde (Apollo) | Intermediate 29 |
| **79** | 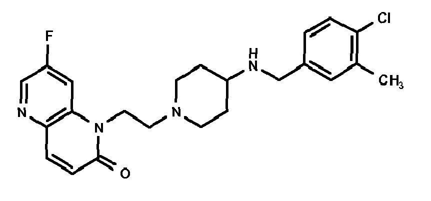 | 1-[2-(4-{[(4-chloro-3-methylphenyl)me thyl]amino}-1-piperidinyl)ethyl]-7-fluoro,-1,5- naphthyridin-2(1H)-one | ¹H-NMR(δ, ppm, CDCl₃): 8,28(d, 1H);7,85(d,1H);7,28(bs,1H);6,98-7,27(m,3H);6,73(d,1H);4,41(bs,2H);3, 99(s,3H);3,77(S,2H);3,04(bs,2H);2,70 (bs,2H);2,55(bs,1H);2,25(bs,2H);1,94 (bs,2H);1,67(bs,2H);1,48(bs,2H) [ES MS] | Example8 | 4-cloro-3-methylbenzaldeh yde (Fluorochem) | Intermediate 21 |
| **80** | 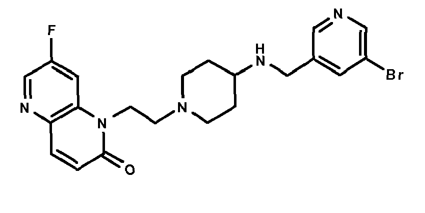 | 1-[2-(4-{[(5-bromo-3-pyridinyl)methyl]a mino}-1-piperidinyl)ethyl]-7-fluoro-1,5-naphthyridin-2(1H)-one | ¹H-NMR(δ, ppm, CDCl₃):8,28(d,1H);7,91(d,1H);7,49(d, 1H);4,44(s,1H);6,73(d,1H);4,48(t,2H) 4.0(s,1H);3,90(s,2H);3,11(d,2H);2,68( t,2H);2,19(t,2H);2,99(d,2H);1,42-1,76(m,2H).[ES MS] m/z 427 (MH+) | Example8 | 5-bromo-3-pyridinecarbalde hyde (Aldrich) | Intermediate 21 |
| **81** | 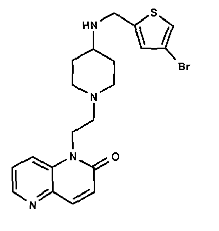 | 1-[2-(4-{[(4-bromo-2-thienyl)methyl]a mho}-1-piperidinyl)ethyl]-1,5-naphthyridin-2(1H)-one | ¹H-NMR(δ, ppm, CDCl₃):8,26(d,1H);7,84(d,1H);7,66(s, 1H);7,44(s,2H);7,29(bs,1H);6,73(d,1H );4,43(t,2H);3,97(s,3H);3,83(s,2H);3,0 8(d,2H);2,72(t,2H);2,51-2,65(m,1H);2,32(t,2H);1,95(d,2H),1,4 1-1,59(m,2H).[ES MS] m/z 495 (MH+). | Example9 | 4-bromo-2-thiophenecarbald hyde (Aldrich) | Intermediate 8 |
| **82** | 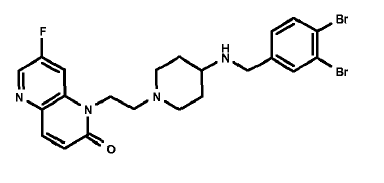 | 1-[2-(4-{[(3,4-dibromophenyl)m ethyl]amino}-1-piperidinyl)ethyl]-7-fluoro-1,5-naphthyridin-2(1 H)-one | 1H-NMR(δ, ppm, CDCl₃):8,35(dd,2H);6,74(d,1H);4,39(b s,2H);3,98(s,3H);3,86(s,2H);2,99(bs,2 H);2,67(bs,2H);2,52(bs,2H);2,32(bs,1 H);2,17(bs,2H);1,91 (bs,2H);1,49-1,60(m,2H):([ES MS] m/z 461 (MH+). | Example10 | 3,4-dibromobenzalde hyde (Alfa Aesar) | Intermediate 20 |
| **83** | 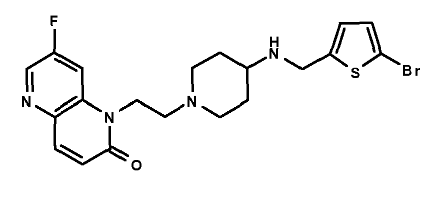 | 1-[2-(4-{[(5-bromo-2-thienyl)methyl]a mino}-1-piperidinyl)ethyl]-7-fluoro-1,5-naphthyridin-2(1H)-one | ¹H-NMR(δ, ppm, CDCl₃):8,65(d,1H);8,28(d,1H);7,9(dd, 1H);7,92(d,1H);7,49(d,1H);7,40(d,1H) ;6,74(d,1H);4,49(t,2H);4,09(s,2H);4,0 3(s,3H);3,09-3,18(m,2H);2,83(bs,1H);2,70(t,2H);2, 21(t,2H);2,04(d,2H);1,47-1,64(m,2H).([ES MS] m/z 472 (MH+). | Example10 | 5-bromo-2-thiophenecarbald ehyde (Aldrich) | Intermediate 20 |
| **84** | 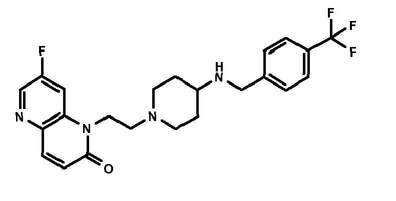 | 7-fluoro-1-{2-[4-({[4-(trifluoromethyl)p henyl]methyl}ami no)-1-piperidinyl]ethyl}-1,5-naphthyridin-2(1H)-one | ¹H-NMR(δ, ppm, DMSO-d₆): 8.27 (d, 1 H); 7.86 (d, 1 H); 7.49 (s, 1 H); 7.40-7.39 (m, 1H); 6.64 (d, 1H); 4.33 (t, 2H); 3.96 (s, 3H); 3.87 (s, 2H); 2.92-2.88 (m, 2H); 2.396-2.31 (m, 1 H); 2.05-1.97 (m, 2H); 1.76-1.72 (m, 2H); 1.24-1.15 (m, 2H). [ES MS] m/z 434 (MH+). | Example10 | 4-(trifluoromethyl)b enzaldehyde (Aldrich) | Intermediate 20 |
| **85** | 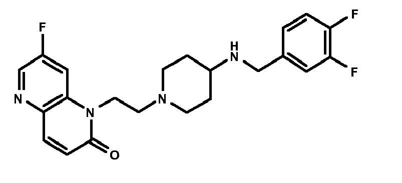 | 1-[2-(4-{[(3,4-difluorophenyl)m ethyl]amino}-1-piperidinyl)ethyl]-7-fluoro-1,5-naphthyridin-2(1H)-one | ¹H-NMR(δ, ppm, DMSO-d₆): 8.27 (d, 1H); 7.85 (d, 1H); 7.40-7.39 (m, 1H); 6.72 (s, 1 H); 6.65 (d, 1 H); 4.33 (t, 2H); 3.96 (s, 3H); 3.79 (s, 2H); 2.91-2.88 (m, 2H); 2.40-2.30 (m, 1 H); 2.06 (s, 3H); 2.03-1.97 (m, 2H); 1.76-1.72 (m, 2H); 1.23-1.15 (m, 2H). [ES MS] m/z 491 (MH+). | Example10 | 3,4-difluorobenzalde hyde (Aldrich) | Intermediate 20 |
| **86** | 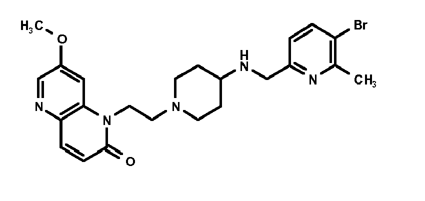 | 1-[2-(4-{[(5-bromo-6-methyl-2-pyridinyl)methyl]a mino}-1-piperidinyl)ethyl]-7-(methyloxy)-1,5-naphthyridin-2(1H)-one | 1H-NMR(δ, ppm, CDCl₃):8.43(d,1H);7,90(d,1H);7.49-7.35(m,2H);7.38(d,1H);7.18(dd,1H);6. 87(d,1H);4.49-4.37(m,1H);4.30-4.17(m,1H);3.88(bs,1H);3.83(d,1H);3. 74(d,1H);3.13(bd,1H);2.85(bd,1H);2.6 9(t,2H);2.54-2.42(m,1H);2.3(d,1H);2.20(td,1H). [ES MS ]m/z 465 (MH+) | Example1 | 5-bromo-6-methyl-2-pyridinecarbalde hyde (Intermediate 52) | Intermediate 29 |
| **87** | 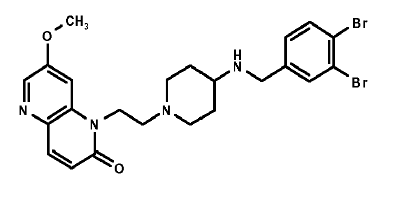 | 1-[2-(4-{[(3,4-dibromophenyl)m ethyl]amino}-1-piperidinyl)ethyl]-7-(methyloxy)-1,5-naphthyridin-2(1 H)-one | 1H-NMR(δ, ppm, CDCl₃): 8,68(d, 1H);8.29(d,1H);7,90(d,1H);7.86(d,1H) ;7.65(d,1H);7.09(d,1H);6.75(d,1H);4.5 7-4.34(m,1H);4.28-3.94(m,1H);3.99(s,3H);3.95(bs,1H);3. 91 (bs,2H);3.17(d,1H);2.85(d,1H);2.75 -2.55(m,2H);2.46-2.44(m,1 H);2.3(d,1H);2.18(td,1H). [ES MS ]m/z 478 (MH+) | Example12 | 3,4-dibromobenzalde hyde (Alfa Aesar) | Intermediate 28 |
| **88** | 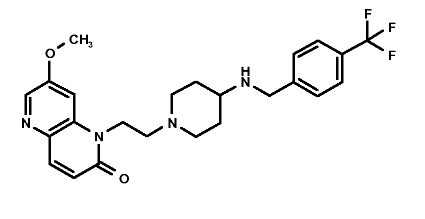 | 7-(methyloxy)-1-{2-[4-({[4-(trifluoromethyl)p henyl]methyl}ami no)-1-piperidinyl]ethyl}-1,5-naphthyridin-2(1H)-one | 1H-NMR(δ, ppm, CDCl₃): 8,29(d, 1H);7,85(d,1H);7,45(d,1H);7.38(d,1H) ;7.18(dd,1H);7.12(d,1H);6.75(d,1H);4. 55-4.41(m,1H);4.32-4.20(m,1H);3.99(bs,1H);3.89(bs,1H); 3.78(q,2H);3.15(d,1H);2.87(d,1H);2.7 7-2.63(m,2H);2.53-2.44(m,1H);2.3(d,1H);2.21(td,1H). [ES MS ]m/z 477 (MH+) | Example12 | 4-(trifluoromethyl)b enzaldehyde (Aldrich) | Intermediate 28 |
| **89** | 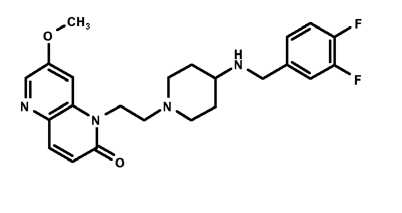 | 1-[2-(4-{[(3,4-difluorophenyl)m ethyl]amino}-1-piperidinyl)ethyl]-7-(methyloxy)-1,5-naphthyridin-2(1H)-one | 1H-NMR(δ, ppm, CDCl₃):8.68(s,1H);8.29(d,1H);7,90(d, 1H);7.86(d,1H);7.65(d,1H);7.09(d,1H) ;6.76(d,1H);4.57-4.44(m,1H);4.30-4.14(m,1H);3.99(s,3H);3.97-3.85(m,4H);3.17(bd,1H);2.87(bd,1H); 2.76-2.64(m,2H);2.52-2.40(m,1H);2.3(d,1H);2.20(td,1H). [ES MS ]m/z 478 (MH+) | Example12 | 3,4-difluorobenzalde hyde (Aldrich) | Intermediate 28 |
| **90** | 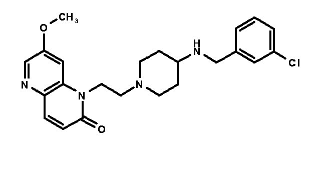 | 1-[2-(4-{[(3-chlorophenyl)met hyl]amino}-1-piperidinyl)ethyl]-7-(methyloxy)-1,5-naphthyridin-2(1H)-one | 1H-NMR(δ, ppm, CDCl₃):8.29(d,1H);7,85(d,1H);7.45(d, 1H);7.18(dd,1H);7.11(d,1H);6.75(d,1 H);4.55-4.41(m,1H);4.33-4.18(m,1H);3.99(s,3H);3.88(bs,1H);3. 83(d,2H);3.74(d,1H);3.15(bd,1H);2.86 (bd,1H);2.74-2.63(m,2H);2.53-2.42(m,1H);2.3(d,1H);2.20(td,1H). [ES MS ]m/z 477 (MH+) | Example12 | 3-chlorobenzaldeh yde (Aldrich) | Intermediate 28 |
| **91** | 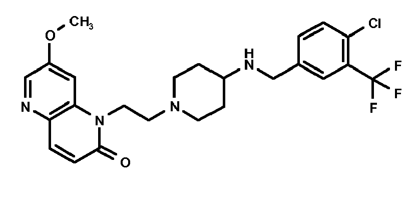 | 1-{2-[4-({[4-chloro-3-(trifluoromethyl)p henyl]methyl}ami no)-1-piperidinyl]ethyl}-7-(methyloxy)-1,5-naphthyridin-2(1H)-one | 1H-NMR(δ, ppm, CDCl₃):8.68(s,1H);8.56(dd,1H);7,97-7.87(m,2H); 7.70(d,1H); 7.65(d,1H);7.51-7.43(m,1H);6.94(d,1H);4.59-4.46(m,1H);4.33-4.20(m,1H);4.01-3.63(m,3H);3.21-3.12(bd,1H);2.90-2.80(bd,1H);2.74-2.66(m,2H);2.50-2.41(m,1H);2.28(d,1H);2.19(td,1H). [ES MS ]m/z 448 (MH+) | Example12 | 4-chloro-3(trifluoromethyl)b enzaldehyde (Aldrich) | Intermediate 28 |
| **92** | 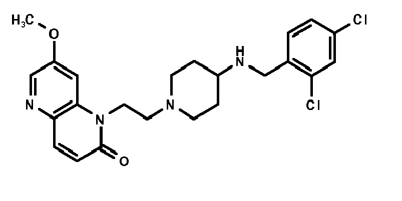 | 1-[2-(4-{[(2,4-dichlorophenyl)m ethyl]amino}-1-piperidinyl)ethyl]-7-(methyloxy)1,5-naphthyridin-2(1H)-one | 1H-NMR(δ, ppm, CDCl₃):8.68(s,1H);8.44(d,1H);7,90(d, 1H);7.64(d,1H);7,42(dd,1H);6.87(d,1 H);4.53-4.40(m,1H);4.14-4.27(m,1H);4.02-3.84(m,3H);3.21-3.12(m,1H);2.80-2.90(m,1H);2.75-2.64(m,2H);2.51-2.42(m,1H);2.3(d,1H);2.20(td,1H). [ES MS ]m/z 466 (MH+) | Example12 | 2,4-dichlorobenzalde hyde (Fluka) | Intermediate 28 |
| **93** | 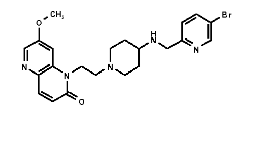 | 1-[2-(4-{[(5-bromo-2-pyridinyl)methyl]a mino}-1-piperidinyl)ethyl]-7-(methyloxy)-1,5-naphthyridin-2(1 H)-one | ¹H-NMR(δ, ppm, DMSO-d₆): 8.55(m, 1H); 8.01-7.97(m, 1H); 7.93(d, 1H); 7.39(s, 1H); 7.31-7.23(m, 3H); 6.82(d, 1H); 4.31-4.27(t, 2H); 3.68(s, 2H); 2.88-2.84(m, 2H); 2.34-2.26(m, 1 H); 2,02-1.95(t, 2H); 1.76-1.71(m, 2H); 1.22-1.10(m, 2H). | Example12 | 5-bromo-2-pyridinecarbalde hyde (Alfaaesar) | Intermediate 28 |
| **94** | 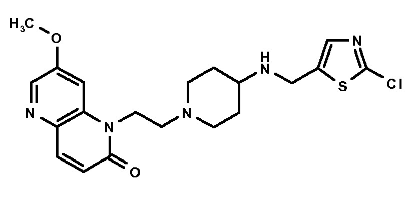 | 1-[2-(4-{[(2-chloro-1,3-thiazol-5-yl)methyl]amino}1-piperidinyl)ethyl]-7-(methyloxy)1,5-naphthyridin-2(1H)-one | ¹H-NMR(δ, ppm, DMSO-d₆): 8.55(d, 1 H); 7.98(br d, 1 H); 7.93(d, 1 H); 7.33(s, 4H); 6.82(d, 1H); 4.29(t, 2H); 3.67(s, 2H); 2.88-2.84(m, 2H); 2.34-2.25(m, 1 H); 2.01-1.95(m, 2H); 1.75-1.71 (m, 2H); 1.22-1.14(m, 2H). [ES MS] m/z 415 (MH+) | Example12 | 2-chloro-1,3-thiazole-5-carbaldehyde (ABCR) | Intermediate 28 |
| **95** | 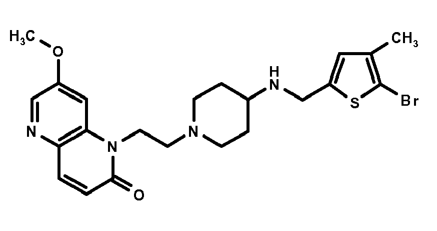 | 1-[2-(4-{[(5-bromo-4-methyl-2-thienyl)methyl]a mino}-1-piperidinyl)ethyl]-7-(methyloxy)1,5-naphthyridin-2(1H)-one | ¹H-NMR(δ, ppm, CDCl₃): 8.27(d,1H);7.91 (d,1H);7.50(d,1H);7.2 7(d,1H);6.95(s,1H);6.73(d,1H);4,46(t, 2H);4.03(s,3H);3.96(s,2H);3.06(d,2H); 2.65(t,2H);2.5-2.61(m,1H);2.17(t,2H),1.93(d,2H),1.4 3(qd,2H)..([ES MS] 477 m/z (MH+). | Example12 | 5-bromo-4-methyl-2-thiophenecarbald ehyde (FRONTIER) | Intermediate 28 |
| **96** | 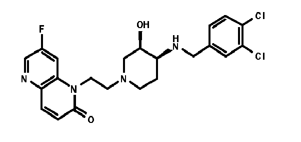 | 1-[2-((3R,4S)-4-{[(3,4-dichlorophenyl)m ethyl]amino}-3-hydroxy-1-piperidinyl)ethyl]-7-fluoro-1,5-naphthyridin-2(1H)-one | ¹H-NMR(δ, ppm, CDCl₃):8.28(d,1H);7.91(d,1H);7.49(d, 1H);6.96(d,1H);6.90(d,1H);6.73(d,1H) ;4.50(t,2H);4.16(s,2H);4.03(s,3H);3.1 7(d,2H);2.83-2.96(m,1H);2.76(t,2H);2.28(t,2H);2.03 (d,2H);1.46-1.63(m,2H).([ES MS] 433 m/z (MH+) | Example12 | 3,4-dichlorobenzalde hyde (Fluka) | Intermediate 31 |
| **97** | 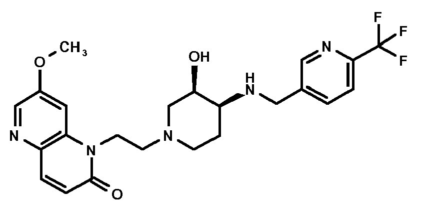 | 1-{2-[(3R,4S)-3-hydroxy-4-({[6-(trifluoromethyl)-3-pyridinyl]methyl}a mino)-1-piperidinyl]ethyl}-7-(methyloxy)-1,5-naphthyridin-2(1H)-one | 1H-NMR(δ, ppm, CDCl3):8.48(d,1H);7.92-8.0(m,2H);6.87(d,1H);6.85(s,1H);4.42 (t,2H);3.91 (s,2H);3.03(d,2H);2.64(t,2 H);2.45-2.58(m,1H);2.16(t,2H);1.90(d,2H);1.3 0-1.46(m,2H).([ES MS] 466 m/z (MH+). | Example12 | 5-(2-furanyl)-4H-pyrazole-3-carboxylic acid (Apollo) | Intermediate 33 |
| **98** | 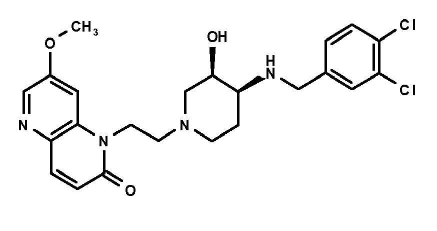 | 1-[2-((3R,4S)-4-{[(3,4-dichlorophenyl)m ethyl]amino}-3-hydroxy-1-piperidinyl)ethyl]-7-(methyloxy)-1,5-naphthyridin-2(1H)-one | ¹H-NMR(δ, ppm, CDCl₃):8.49(d,1H);7.91-8.01(m,2H);6.83-6.92(m,2H);4.43(t,2H);3.97(s,2H);3.0 8(d,3H);2.69(t,2H);2.55-2.64(m,1H);2.22(t,2H);1.94-(d,2H);1.39-1.53(m,2H)..([ES MS] 543 m/z (MH+) | Example12 | 3,4-dichlorobenzalde hyde (Fluka) | Intermediate 33 |
| **99** | 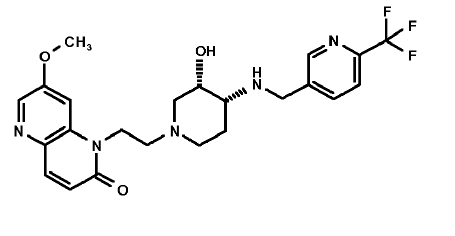 | 1-{2-[(3S,4R)-3-hydroxy-4-({[6-(trifluoromethyl)-3-pyridinyl]methyl}a mino)-1-piperidinyl]ethyl}- 7-(methyloxy)1,5-naphthyridin-2(1H)-one | ¹H-NMR(δ, ppm, MeOD₃):8.48(d,1H);7.91-8.01(m,2H);7.26(s,1H);6.94(s,1H);6.8 6(d,1H);4.42(t,2H);3.95(s,2H);3.03(d, 2H);2.64(t,2H);2.48-2.60(m,1H);2.15(t,2H);1.91(d,2H);1.3 1-1.48(m,2H).([ES MS] 465 m/z (MH+). | Example12 | 6-(trifluoromethyl)-3-pyridinecarbalde hyde (Apollo) | Intermediate 37 |
| **100** | 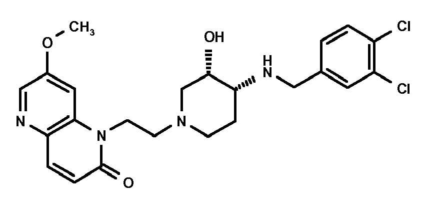 | 1-[2-((3S,4R)-4-{[(3,4-dichlorophenyl)m ethyl]amino}-3-hydroxy-1-piperidinyl)ethyl]-7-(methyloxy)-1,5-naphthyridin-2(1H)-one | ¹H-NMR (δ, ppm, CDCl₃): 8.42 (d, 1 H); 8.31 (d, 1H); 7.86 (d, 1H); 7.65 (d, 1H); 7.54 (d, 1H); 6.86 (d, 1H); 4.32 (t, 2H); 3.79 (s, 2H); 2.96 (bd, 2H); 2.65 (t, 2H); 2.60 (s, 3H); 2.55-2,46 (m, 1H); 2.18 (bt, 2H); 1.90 (bd, 2H); 1.46-1.33 (m, 2H). [ES MS] m/z 430 (MH+). | Example12 | 3,4-dichlorobenzalde hyde (Fluka) | Intermediate 37 |
| **101** | 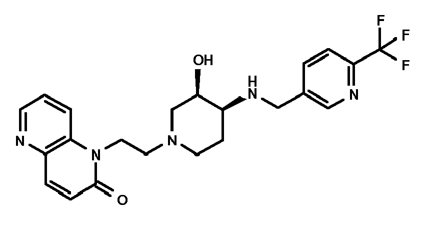 | 1-{2-[(3R,4S)-3-hydroxy-4-({[6-(trifluoromethyl)-3pyridinyl]methyl}a mino)-1-piperidinyl]ethyl}-1,5-naphthyridin-2(1H)-one | ¹H-NMR (δ, ppm, CDCl₃): 8.31 (d, 1H); 8.28 (d, 1H); 7.84 (d, 1H); 7.66 (d, 1H); 7.23 (dd, 1H); 6.74 (d, 1H); 4.36 (t, 2H); 3.98 (s, 3H); 3.79 (s, 2H); 2.99 (bd, 2H); 2.65 (t, 2H); 2.60 (s, 3H); 2.65-2.48 (m, 1H); 2.19 (bt, 2H); 1.91 (bd, 2H); 1.48-1.35 (m, 2H). [ES MS] m/z 442 (MH+). | Example12 | 6-(trifluoromethyl)-3-pyridinecarbalde hyde (Apollo) | Intermediate 35 |
| **102** | 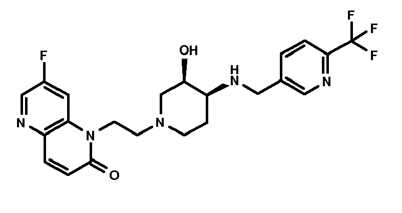 | 7-fluoro-1-{2-[(3R,4S)-3-hydroxy-4-({[6-(trifluoromethyl)3-pyridinyl]methyl}a mino)-1-piperidinyl]ethyl}-1,5-naphthyridin-2(1H)-one | ¹H-NMR (δ, ppm, CDCl₃): 8.55 (d, 1H); 8.31 (d, 1H); 7.92 (d, 1H); 7.79 (d, 1 H); 7.65 (d, 1 H); 7.47 (dd, 1 H); 6.92 (d, 1H); 4.39 (t, 2H); 3.79 (s,2H); 2.98 (bd, 2H); 2.65 (t, 2H); 2.60 (s, 3H); 2.57-2.47 (m, 1 H); 2.19 (bt, 2H); 1.90 (bd, 2H); 1.47-1.35 (m, 2H). [ES MS] m/z 412 (MH+). | Example12 | 6-(trifluoromethyl)-3-pyridinecarbalde hyde (Apollo) | Intermediate 31 |
| **103** | 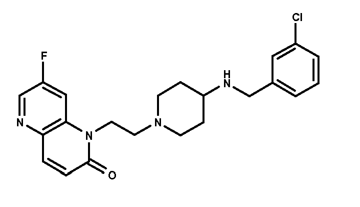 | 1-[2-(4-{[(3-chlorophenyl)met hyl]amino}-1-piperidinyl)ethyl]-7-fluoro-1,5-naphthyridin-2(1H)-one | ¹H-NMR(δ, ppm, CDCl₃): 8.28 (d, 1H); 7.84 (d, 1H); 7.19 (d, 1H); 6.74 (d, 1H); 4.36 (t, 2H); 4.02 (d, 2H); 3.98 (s, 3H); 3.05-2.91 (m, 2H); 2.65 (t, 2H); 2.60-2.49 (m, 1H); 2.19 (dt, 2H); 1.91 (brd, 2H); 1.49-1.32 (m, 2H). [ES MS] m/z 468 (MH+). | Example12 | 3-chlorobenzaldeh yde (Aldrich) | Intermediate 20 |
| **104** | 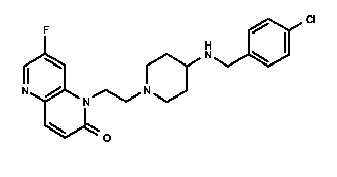 | 1-[2-(4-{[(4-chlorophenyl)met hyl]amino}-1-piperidinyl)ethyl]-7-fluoro-1,5-naphthyridin-2(1H)-one | 1H-NMR(δ, ppm, CDCl₃): 8,27(d, 1H);7,85(d,1H);7,60(d,1H);7,48(d,1H) ;6,73(d,1H);3.99(t,3H);3.96(s,3H);3,9 0(s,2H);3.05(d,2H);2,68(t,2H);2,56(bs ,1H);2.24(bs,2H);1.93(d,2H);1.86-1.64(m,2H);1,42-1.57(m,2H); [ES MS ]m/z 469 (MH+) | Example12 | 4-chlorobenzaldeh yde (Fluka) | Intermediate 20 |
| **105** | 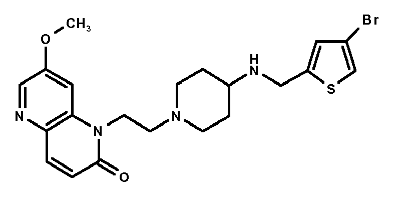 | 1-[2-(4-{[(4-bromo-2-thienyl)methyl]a mino}-1-piperidinyl)ethyl]-7-(methyloxy)-1-5-naphthyridin-1,5-naphthyridin-2(1H)-one | ¹H-NMR(δ, ppm, CDCl₃): 8.29 (d, 1H); 7.88-7.77 (m, 3H); 7.23 (bd, 1H); 6.75 (d, 1H); 4.38 (bt, 2H); 4.26 (s, 2H); 3.99 (s, 3H); 3.07-2.96 (m, 2H); 2.73-2.53 (m, 3H); 2.22 (bt, 2H); 1.95 (bd, 2H); 1.56-1.38 (m, 2H). [ES MS] m/z 463 (MH+). | Example13 | 4-bromo-2-thiophenecarbald ehyde (Aldrich) | Intermediate 28 |
| **106** | 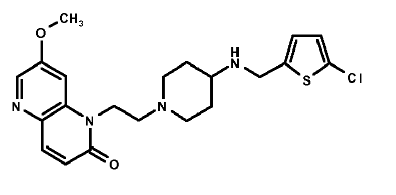 | 1-[2-(4-{[(5-chloro-2-thienyl)methyl]a mino}-1-piperidinyl)ethyl]-7-(methyloxy)-1,5-naphthyridin2(1H)-one | ¹H-NMR (δ, ppm, DMSO-d₆): 8.59-8.54 (m,2H); 8.01-7.92 (m, 2H); 7.70 (d, 1H); 6.82 (d, 1H); 4.29 (t, 2H); 3.77 (s, 2H); 2.88-2.84 (m, 2H); 2.37-2.29 (m, 2H); 2.03-1.96 (m, 2H); 1.75-1.71 (m, 2H); 1.22-1.11 (m, 2H). [ES MS] m/z 434 (MH+). | Example13 | 5-chloro-2-thiophenecarbald ehyde (Aldrich) | Intermediate 28 |
| **107** | 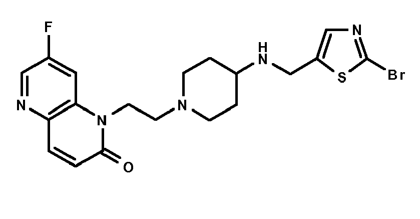 | 1-[2-(4-{[(2-bromo-1,3-thiazol-5-yl)methyl]amino}-1-piperidinyl)ethyl]-7-fluoro-1,5-naphthyridin-2(1H)-one | ¹H-NMR(δ, ppm, DMSO): 8.60 (d, 1H); 8.27 (d, 1H); 7.85 (d, 1H); 7.73 (d, 1H); 7.40 (d, 1H); 6.64 (d, 1H); 4.36-4.31 (t, 2H); 3.97 (s, 3H); 3.81 (s, 2H); 2.92-2.89 (m, 2H); 2.41-2.34(m, 1H); 2.06-1.98(m, 2H); 1.79-1.74 (m, 2H); 1.27-1.16 (m, 2H) [ES MS] m/z 446(MH+) | Example13 | 2-bromo-1,3-thiazole-5-carbaldehyde (Combi-Blocks) | Intermediate 20 |
| **108** | 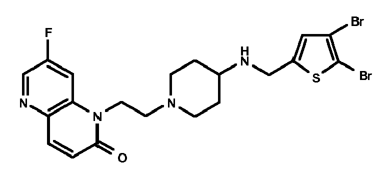 | 1-[2-(4-{[(4,5-dibromo-2-thienyl)methyl]a mino}-1-piperidinyl)ethyl]7-fluoro-1,5-naphthyridin-2(1H)-one | ¹H-NMR (δ, ppm, CDCl₃): 8.42 (d, 1H); 7.89 (d, 1H); 7.74 (d, 1H); 7.56 (bd, 1H); 7.04 (d, 1H); 6.86 (d, 1H); 4.32 (t, 2H); 3.85 (s, 2H); 2.96 (bd, 2H); 2.67-2.63 (m, 2H); 2.64 (s, 3H); 2.58-2.49 (m, 1H); 2.19 (bt, 2H); 1.92 (bd, 2H); 1.50-1.38 (m, 2H). [ES MS] m/z 474 (MH+). | Example13 | 4,5-dibromo-2-thiophenecarbald ehyde (Acros) | Intermediate 20 |
| **109** | 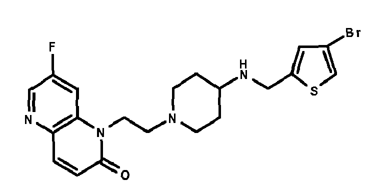 | 1-[2-(4-{[(4-bromo-2-thienyl)methyl]a mino}-1-piperidinyl)ethyl]-7-fluoro-1,5-naphthyridin-2(1H)-one | 1H-NMR(δ, ppm, CDCl₃): 8.27 (d, 1H); 7.84 (d, 1H); 7.73 (d, 1H); 7.26 (s, 1H); 7.02 (d, 1H); 6.73 (d, 1H); 4.39 (bs, 2H); 3.98 (s, 3H); 3.85 (s, 2H); 3.01 (bd, 2H); 2.69-2.64 (m, 5H); 2.56 (bs, 1 H); 2.22 (bs, 2H); 1.94 (bd, 2H); 1.54-1.47 (m, 2H). [ES MS] m/z 486 (MH+). | Example13 | 4-bromo-2-thiophenecarbald ehyde (Aldrich) | Intermediate 20 |
| **110** | 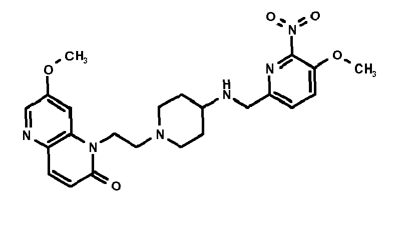 | 7-(methyloxy)-1-{2-[4-({[5-(methyloxy)-6-nitro-2-pyridinyl]methyl}a mino)-1-piperidinyl]ethyl}-1,5-naphthyridin-2(1H)-one | 1H-NMR(δ, ppm, CDCl₃): 8,42(d, 1H);7,88(d,1H);7,56(d,1H);6.85(d,1H) ;4.43(t,2H);4.03(s,2H);2,97(d,2H);2.6 6(t,2H);2,56(bs,1H);2,35(s,3H);2.20(b s,2H);1.95(d,2H);1.63(bs,2H);1,35-1.50(m,2H); [ES MS ]m/z 480 (MH+) | Example15 | 6-(bromomethyl)-3-(methyloxy)-2-nitropyridine (*not an aldehyde) (Intermediate 43) | Intermediate 29 |
| **111** | 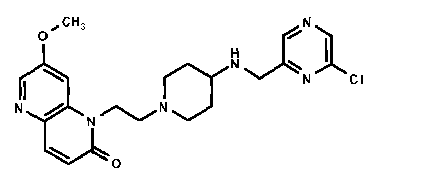 | 1-[2-(4-{[(6-chloro-2-pyrazinyl)methyl] amino}-1-piperidinyl)ethyl]-7-(methyloxyl-7-(methyloxy)-1,5-naphthyridin-2(1H)-one | ¹H-NMR(δ, ppm, CDCl₃): 8.27 (s, 1H); 7.84 (d, 1H); 6.63 (d, 1H); 4.37 (t, 2H); 4.02 (s, 2H); 3.98 (s, 3H); 3.04-2.90 (m, 2H); 2.71-2.51 (m, 3H); 2.32 (s, 3H); 2.29 (s, 3H); 2.20 (t, 2H); 2.00-1.85 (m, 2H); 1.53-1,34 (m, 2H). [ES MS ]m/z 428 (MH+). | Example15 | 2-(Bromomethyl)-6-chloropyrazine (*not an aldehyde) (Intermediate 57) | Intermediate 29 |

### Biological Activity

### General Antimicrobial Activity Assay

Whole-cell antimicrobial activity was determined by broth microdilution using the Clinical and Laboratory Standards Institute (CLSI) recommended procedure, Document M7-A7, "Methods for Dilution Susceptibility Tests for Bacteria that Grow Aerobically". The compounds were tested in serial two-fold dilutions ranging from 0.016 to 64 µg/mL.

Compounds were evaluated against gram-positive organisms, selected from i) *Staphylococcus aureus*, ii) *Streptococcus pneumoniae*, and iii) *Enterococcus faecalis*. Two compounds were additionally evaluated against *Streptococcus pyogenes*, and *Enterococcus faecium.*

In addition, compounds were evaluated against gram-negative organisms selected from iv) *Haemophilus influenzae*, v) *Moraxella catarrhalis* and vi) *Escherichia coli*. Two compounds were additionally evaluated against *Pseudomonas aeruginosa*, *Proteus mirabilis, Enterobacter cloacae, Enterobacter aerogenes, Klebsiella pneumoniae* and *Stenotrophomonas maltophilia.*

The minimum inhibitory concentration (MIC) was determined as the lowest concentration of compound that inhibited visible growth. A mirror reader was used to assist in determining the MIC endpoint.

### Results of the general antimicrobial activity assay

Examples 1, 1b, 2, 2b, 3, 3b, 4, 4b, 5-8, 8b, 9-11, 11c, 12, 12b, 12c, 13-54, 56-71, 73-86 89-109 and 111 were tested against organisms i)-vi) listed above in the antimicrobial activity assay. Examples 23 and 82 were additionally tested against the other organisms listed above. All tested Examples showed an MIC value of 2µg/ml or lower against a strain of at least one of the organisms listed above, with the exception of Example 14 and 57 which showed an MIC value of 4 against a strain of at least one of the organisms listed above; Example 60 which showed an MIC value of 32 against a strain of at least one of the organisms listed above; and Example 58 which showed no activity against organisms i)-vi). For at least one strain of each organism i)-vi) listed hereinabove, at least one tested Example had an MIC value of 2µg/ml or lower.

### Mycobacterium tuberculosis H37Rv Inhibition Assay

The measurement of the minimum inhibitory concentration (MIC) for each tested compound was performed in 96 wells flat-bottom, polystyrene microtiter plates. Ten two-fold drug dilutions in neat DMSO starting at 400µM were performed. Five µl of these drug solutions were added to 95 µl of Middlebrook 7H9 medium. (Lines A-H, rows 1-10 of the plate layout). Isoniazid was used as a positive control, 8 two-fold dilution of Isoniazid starting at 160 µgml⁻¹ was prepared and 5 µl of this control curve was added to 95µl of Middlebrook 7H9 medium (Difco catalogue ref. 271310). (Row 11, lines A-H). Five µl of neat DMSO were added to row 12 (growth and Blank controls).

The inoculum was standardised to approximately 1x10⁷ cfu/ml and diluted 1 in 100 in Middlebrook 7H9 broth (Middlebrook ADC enrichment, a dehydrated culture media which supports growth of mycobacterial species available from Becton Dickinson Catalogue Ref. 211887), to produce the final inoculum of H37Rv strain (ATCC25618). One hundred µl of this inoculum was added to the entire plate but G-12 and H-12 wells (Blank controls). All plates were placed in a sealed box to prevent drying out of the peripheral wells and they were incubated at 37°C without shaking for six days. A resazurin solution was prepared by dissolving one tablet of resazurin (Resazurin Tablets for Milk Testing; Ref 330884Y VWR International Ltd) in 30 ml sterile PBS (phosphate buffered saline). 25 µl of this solution was added to each well. Fluorescence was measured (Spectramax M5 Molecular Devices, Excitation 530nm, Emission 590nm) after 48 hours to determine the MIC value.

### Results of the Mycobacterium tuberculosis H37Rv Inhibition Assay

Examples 1, 1b, 2, 2b, 3, 3b, 4, 4b, 5-8, 8b, 9-11, 11c, 12, 12b, 12c and 13-111 were tested in the *Mycobacterium tuberculosis* H37Rv inhibition assay. Examples 1, 1b, 2, 2b, 3, 3b, 4, 4b, 5-8, 8b, 9-11, 11b, 11c, 12, 12b, 12c, 13-59 and 61-111 showed an MIC value of 2.4 µg/ml or lower. Examples 1, 1 b, 2, 2b, 3, 3b, 4, 4b, 5, 7-8, 8b, 10, 11, 11b, 11c, 12, 12b, 12c, 13, 15-33, 35-37, 39-47, 50, 52-56, 58, 59, 61-63, 65-67, 69-80, 82-102 and 104-110 showed an MIC value of 1.0 µg/ml or lower.

## Claims

**1.** A compound of Formula (I) or a pharmaceutically acceptable salt, solvate or N-oxide thereof: 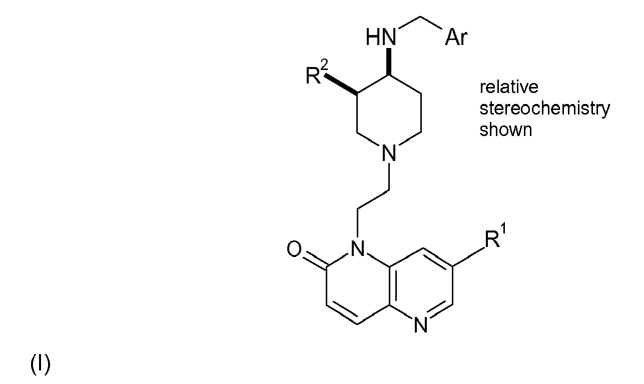 Wherein:
R¹ represents hydrogen; halo; or C₁₋₃alkoxy-;
R² represents hydrogen or hydroxy;
Ar represents a group selected from: phenyl, pyridyl, pyridazinyl, pyrimidinyl, pyrazinyl, thiazolyl, furanyl, imidazolyl and thiophenyl;
wherein
Ar is substituted by a first group R³, wherein R³ represents a group selected from: halo, CF₃, C₁₋₃alkyl, nitro and cyano; when represents pyridyl, R³ alternatively represents C₁₋₃alkoxy-;
Ar is optionally substituted by a second group R⁴;
when R³ represents halo, the optional R⁴ group represents halo;
when R³ represents CF₃, the optional R⁴ group represents halo;
when R³ represents C₁₋₃alkyl, the optional R⁴ group is selected from halo, CF₃, C₁₋₃alkyl, nitro and C₁₋₃alkoxy-;
when R³ represents nitro, the optional R⁴ group is selected from halo and CF₃;
when R₃ represents cyano, the optional R⁴ group is selected from halo, CF₃, C₁₋₃alkyl and nitro;
when R³ represents C₁₋₃alkoxy-, the optional R⁴ group is selected from halo and nitro.

**2.** A compound of Formula (I), or a pharmaceutically acceptable salt, solvate or N-oxide thereof, according to claim 1, wherein Ar represents a group selected from: phenyl, pyridyl, pyridazinyl, pyrazinyl, thiazolyl, furanyl and thiophenyl.

**3.** A compound of Formula (I), or a pharmaceutically acceptable salt, solvate or N-oxide thereof, according to claim 1 or claim 2, wherein the substituent R⁴ is present.

**4.** A compound of Formula (I) according to any preceding claim, wherein when Ar represents phenyl, the substituent R³ and optional substituent R⁴ occur in the meta- or para- positions relative to the point of attachment of Ar to the remainder of the molecule.

**5.** A compound of Formula (I) according to any preceding claim, wherein the absolute stereochemistry is

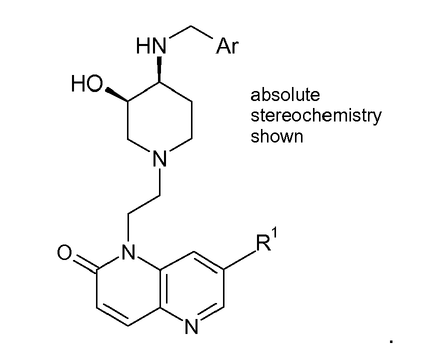

**5.** A pharmaceutically acceptable salt of a compound of Formula (I) according to any preceding claim.

**6.** A compound of Formula (I) selected from the list:
1-[2-(4-{[(5-bromo-2-pyridinyl)methyl]amino}-1-piperidinyl)ethyl]-7-fluoro-1,5-naphthyridin-2(1 H)-one;
1-[2-(4-{[(5-bromo-2-pyridinyl)methyl]amino}-1-piperidinyl)ethyl]-7-fluoro-1,5-naphthyridin-2(1 H)-one dihydrochloride;
1-[2-(4-{[(2-bromo-1,3-thiazol-5-yl)methyl]amino}-1-piperidinyl)ethyl]-7-(methyloxy)-1,5-naphthyridin-2(1H)-one;
1-[2-(4-{[(2-bromo-1,3-thiazol-5-yl)methyl]amino}-1-piperidinyl)ethyl]-7-(methyloxy)-1,5-naphthyridin-2(1H)-one hydrochloride;
1-[2-(4-{[(3,4-dichlorophenyl)methyl]amino}-1-piperidinyl)ethyl]-7-fluoro-1,5-naphthyridin-2(1 H)-one;
1-[2-(4-{[(3,4-dichlorophenyl)methyl]amino}-1-piperidinyl)ethyl]-7-fluoro-1,5-naphthyridin-2(1 H)-one;
7-(methyloxy)-1-{2-[4-({[6-(trifluoromethyl)-3-pyridinyl]methyl}amino)-1-piperidinyl]ethyl}-1,5-naphthyridin-2(1H)-one;
7-(methyloxy)-1-{2-[4-({[6-(trifluoromethyl)-3-pyridinyl]methyl}amino)-1-piperidinyl]ethyl}-1,5-naphthyridin-2(1H)-one hydrochloride;
1-[2-(4-{[(4-fluoro-3-methylphenyl)methyl]amino}-1-piperidinyl)ethyl]-7-(methyloxy)-1,5-naphthyridin-2(1H)-one;
1-[2-(4-{[(4,5-dimethyl-1,3-thiazol-2-yl)methyl]amino}-1-piperidinyl)ethyl]-7-(methyloxy)-1,5-naphthyridin-2(1H)-one;
1-[2-(4-{[(4,5-dichloro-1,3-thiazol-2-yl)methyl]aminol-1-piperidinyl)ethyl]-7-(methyloxy)-1,5-naphthyridin-2(1H)-one;
1-[2-(4-{[(3,4-dichlorophenyl)methyl]amino}-1-piperidinyl)ethyl]-1,5-naphthyridin-2(1H)-one;
1-[2-(4-{[(3,4-dichlorophenyl)methyl]amino}-1-piperidinyl)ethyl]-1,5-naphthyridin-2(1H)-one dihydrochloride;
1-[2-(4-{[(6-bromo-3-pyridinyl)methyl]amino}-1-piperidinyl)ethyl]-1,5-naphthyridin-2(1H)-one;
7-fluoro-1-{2-[4-({[6-(trifluoromethyl)-3-pyridinyl]methyl}amino)-1-piperidinyl]ethyl}-1,5-naphthyridin-2(1H)-one;
1-[2-(4-{[(3,4-dichlorophenyl)methyl]amino}-1-piperidinyl)ethyl]-7-(methyloxy)-1,5-naphthyridin-2(1H)-one;
1-[2-(4-{[(3,4-dichlorophenyl)methyl]amino}-1-piperidinyl)ethyl]-7-(methyloxy)-1,5-naphthyridin-2(1H)-one hydrochloride;
1-[2-(4-{[(3,4-dichlorophenyl)methyl]amino}-1-piperidinyl)ethyl]-7-(methyloxy)-1,5-naphthyridin-2(1H)-one dihydrochloride;
1-[2-(4-{[(4-chlorophenyl)methyl]amino}-1-piperidinyl)ethyl]-7-(methyloxy)-1,5-naphthyridin-2(1H)-one;
1-[2-(4-{[(4-chlorophenyl)methyl]amino}-1-piperidinyl)ethyl]-7-(methyloxy)-1,5-naphthyridin-2(1H)-one dihydrochloride;
1-[2-(4-{[(4-chlorophenyl)methyl]amino}-1-piperidinyl)ethyl]-7-(methyloxy)-1,5-naphthyridin-2(1H)-one hydrochloride;
1-[2-(4-{[(6-bromo-3-pyridinyl)methyl]amino}-1-piperidinyl)ethyl]-7-fluoro-1,5-naphthyridin-2(1 H)-one;
7-fluoro-1-[2-(4-{[(4-fluorophenyl)methyl]amino}-1-piperidinyl)ethyl]-1,5-naphthyridin-2(1 H)-one;
7-(methyloxy)-1-{2-[4-({[6-(trifluoromethyl)-3-pyridazinyl]methyl}amino)-1-piperidinyl]ethyl}-1,5-naphthyridin-2(1H)-one;
1-[2-(4-{[(5-Bromo-3-isothiazolyl)methyl]amino}-1-piperidinyl)ethyl]-7-(methyloxy)-1,5-naphthyridin-2(1H)-one;
1-[2-(4-{[(4-chlorophenyl)methyl]amino}-1-piperidinyl)ethyl]-7-(ethyloxy)-1,5-naphthyridin-2(1 H)-one;
7-(ethyloxy)-1-{2-[4-({[6-(trifluoromethyl)-3-pyridinyl]methyl}amino)-1-piperidinyl]ethyl}-1,5-naphthyridin-2(1H)-one;
1-[2-(4-{[(3,4-dichlorophenyl)methyl]amino}-1-piperidinyl)ethyl]-7-(ethyloxy)-1,5-naphthyridin-2(1H)-one;
1-[2-(4-{[(5-chloro-6-methyl-3-pyridinyl)methyl]amino}-1-piperidinyl)ethyl]-7-fluoro-1,5-naphthyridin-2(1H)-one;
1-[2-(4-{[(4-bromo-2-pyridinyl)methyl]amino}-1-piperidinyl)ethyl]-7-(methyloxy)-1,5-naphthyridin-2(1H)-one;
1-[2-(4-{[(5-bromo-3-pyridinyl)methyl]amino}-1-piperidinyl)ethyl]-7-(methyloxy)-1,5-naphthyridin-2(1H)-one;
1-[2-(4-{[(5-chloro-6-methyl-3-pyridinyl)methyl]amino}-1-piperidinyl)ethyl]-7-(methyloxy)-1,5-naphthyridin-2(1H)-one;
1-[2-(4-{[(4-chloro-3-methylphenyl)methyl]amino}-1-piperidinyl)ethyl]-7-(methyloxy)-1,5-naphthyridin-2(1H)-one;
1-[2-(4-{[(3-chloro-4-methylphenyl)methyl]amino}-1-piperidinyl)ethyl]-1,5-naphthyridin-2(1 H)-one;
1-[2-((3R,4S)-4-{[(3,4-dichlorophenyl)methyl]amino}-3-hydroxy-1-piperidinyl)ethyl]-1,5-naphthyridin-2(1H)-one;
1-[2-(4-{[(3,4-dimethylphenyl)methyl]amino}-1-piperidinyl)ethyl]-7-fluoro-1,5-naphthyridin-2(1 H)-one;
1-[2-(4-{[(4-chloro-3-methylphenyl)methyl]amino}-1-piperidinyl)ethyl]-1,5-naphthyridin-2(1 H)-one;
1-[2-(4-{[(5-chloro-6-methyl-3-pyridinyl)methyl]amino}-1-piperidinyl)ethyl]-1,5-naphthyridin-2(1 H)-one;
1-[2-(4-{[(5-bromo-6-methyl-2-pyridinyl)methyl]amino}-1-piperidinyl)ethyl]-7-fluoro-1,5-naphthyridin-2(1H)-one;
1-[2-(4-{[(3-fluoro-4-methylphenyl)methyl]amino}-1-piperidinyl)ethyl]-1,5-naphthyridin-2(1 H)-one;
1-[2-(4-{[(3,4-dimethylphenyl)methyl]amino}-1-piperidinyl)ethyl]-1,5-naphthyridin-2(1H)-one;
1-[2-(4-{[(4-chloro-5-fluoro-2-pyridinyl)methyl]amino}-1-piperidinyl)ethyl]-7-fluoro-1,5-naphthyridin-2(1H)-one;
1-[2-(4-{[(5-bromo-2-furanyl)methyl]amino}-1-piperidinyl)ethyl]-7-fluoro-1,5-naphthyridin-2(1 H)-one;
1-{2-[4-({[6-(trifluoromethyl)-3-pyridinyl]methyl}amino)-1-piperidinyl]ethyl}-1,5-naphthyridin-2(1 H)-one;
1-{2-[4-({[4-chloro-3-(trifluoromethyl)phenyl]methyl}amino)-1-piperidinyl]ethyl}-1,5-naphthyridin-2(1H)-one;
7-(methyloxy)-1-[2-(4-{[(4-nitrophenyl)methyl]amino}-1-piperidinyl)ethyl]-1,5-naphthyridin-2(1 H)-one;
1-[2-(4-{[(2,5-dichlorophenyl)methyl]amino}-1-piperidinyl)ethyl]-7-fluoro-1,5-naphthyridin-2(1 H)-one;
1-[2-(4-{[(6-bromo-3-pyridinyl)methyl]amino}-1-piperidinyl)ethyl]-7-(methyloxy)-1,5-naphthyridin-2(1H)-one;
1-[2-(4-{[(3,5-dichlorophenyl)methyl]amino}-1-piperidinyl)ethyl]-7-fluoro-1,5-naphthyridin-2(1 H)-one;
1-[2-(4-{[(4-fluorophenyl)methyl]amino}-1-piperidinyl)ethyl]-7-(methyloxy)-1,5-naphthyridin-2(1 H)-one;
1-{2-[4-({[4-(trifluoromethyl)phenyl]methyl}amino)-1-piperidinyl]ethyl}-1,5-naphthyridin-2(1 H)-one;
1-[2-(4-{[(4-chlorophenyl)methyl]amino}-1-piperidinyl)ethyl]-1,5-naphthyridin-2(1H)-one;
1-[2-(4-{[(5-chloro-2-thienyl)methyl]amino}-1-piperidinyl)ethyl]-7-fluoro-1,5-naphthyridin-2(1 H)-one;
1-{2-[4-({[5-chloro-6-(methyloxy)-3-pyridinyl]methyl}amino)-1-piperidinyl]ethyl}-7-fluoro-1,5-naphthyridin-2(1H)-one;
1-[2-(4-{[(3,5-dichlorophenyl)methyl]amino}-1-piperidinyl)ethyl]-7-(methyloxy)-1,5-naphthyridin-2(1H)-one;
1-{2-[4-({[5-chloro-6-(methyloxy)-3-pyridinyl]methyl}amino)-1-piperidinyl]ethyl}-7-(methyloxy)-1,5-naphthyridin-2(1H)-one;
1-[2-(4-{[(5-bromo-2-thienyl)methyl]amino}-1-piperidinyl)ethyl]-1,5-naphthyridin-2(1H)-one;
1-[2-(4-{[(2-bromo-1,3-thiazol-5-yl)methyl]amino}-1-piperidinyl)ethyl]-1,5-naphthyridin-2(1 H)-one;
1-[2-(4-{[(4,5-dibromo-2-thienyl)methyl]amino}-1-piperidinyl)ethyl]-7-(methyloxy)-1,5-naphthyridin-2(1H)-one;
1-{2-[4-({[3-chloro-4-(methyloxy)phenyl]methyl}amino)-1-piperidinyl]ethyl}-1,5-naphthyridin-2(1H)-one;
1-[2-(4-{[(3,4-dibromophenyl)methyl]amino}-1-piperidinyl)ethyl]-1,5-naphthyridin-2(1H)-one;
4-{[(1-{2-[7-(methyloxy)-2-oxo-1,5-naphthyridin-1(2H)-yl]ethyl}-4-piperidinyl)amino]methyl}benzonitrile;
1-[2-(4-{[(5-bromo-2-thienyl)methyl]amino}-1-piperidinyl)ethyl]-7-(methyloxy)-1,5-naphthyridin-2(1H)-one;
1-[2-(4-{[(4,5-dibromo-2-thienyl)methyl]amino}-1-piperidinyl)ethyl]-1,5-naphthyridin-2(1H)-one;
1-[2-(4-{[(5-bromo-4-methyl-2-thienyl)methyl]amino}-1-piperidinyl)ethyl]-7-fluoro-1,5-naphthyridin-2(1H)-one;
1-[2-(4-{[(3-chlorophenyl)methyl]amino}-1-piperidinyl)ethyl]-1,5-naphthyridin-2(1H)-one;
1-[2-(4-{[(3,5-dichlorophenyl)methyl]amino}-1-piperidinyl)ethyl]-1,5-naphthyridin-2(1H)-one;
7-fluoro-1-[2-(4-{[(4-methyl-3-nitrophenyl)methyl]amino}-1-piperidinyl)ethyl]-1,5-naphthyridin-2(1H)-one;
1-[2-(4-{[(4-bromo-1,3-thiazol-2-yl)methyl]amino}-1-piperidinyl)ethyl]-7-fluoro-1,5-naphthyridin-2(1H)-one;
1-[2-(4-{[(3-chloro-4-methylphenyl)methyl]amino}-1-piperidinyl)ethyl]-7-fluoro-1,5-naphthyridin-2(1H)-one;
7-fluoro-1-{2-[4-({[5-fluoro-6-(methyloxy)-3-pyridinyl]methyl}amino)-1-piperidinyl]ethyl}-1,5-naphthyridin-2(1H)-one;
1-[2-(4-{[(5-bromo-2-furanyl)methyl]amino}-1-piperidinyl)ethyl]-7-(methyloxy)-1,5-naphthyridin-2(1H)-one;
1-[2-(4-{[(4-bromo-1,3-thiazol-2-yl)methyl]amino}-1-piperidinyl)ethyl]-7-(methyloxy)-1,5-naphthyridin-2(1H)-one;
1-[2-(4-{[(5-bromo-4-methyl-2-thienyl)methyl]amino}-1-piperidinyl)ethyl]-1,5-naphthyridin-2(1 H)-one;
1-{2-[4-({[5-fluoro-6-(methyloxy)-3-pyridinyl]methyl}amino)-1-piperidinyl]ethyl}-7-(methyloxy)-1,5-naphthyridin-2(1H)-one;
7-(methyloxy)-1-{2-[4-({[6-(methyloxy)-3-pyridinyl]methyl}amino)-1-piperidinyl]ethyl}-1,5-naphthyridin-2(1H)-one;
1-[2-(4-{[(5-fluoro-6-methyl-2-pyridinyl)methyl]amino}-1-piperidinyl)ethyl]-7-(methyloxy)-1,5-naphthyridin-2(1H)-one;
1-[2-(4-{[(3-chloro-4-methylphenyl)methyl]amino}-1-piperidinyl)ethyl]-7-(methyloxy)-1,5-naphthyridin-2(1H)-one;
1-[2-(4-{[(6-fluoro-5-methyl-3-pyridinyl)methyl]amino}-1-piperidinyl)ethyl]-7-(methyloxy)-1,5-naphthyridin-2(1H)-one;
1-[2-(4-{[(4-chloro-5-fluoro-2-pyridinyl)methyl]amino}-1-piperidinyl)ethyl]-7-(methyloxy)-1,5-naphthyridin-2(1H)-one;
1-[2-(4-{[(5-bromo-4-methyl-1,3-thiazol-2-yl)methyl]amino}-1-piperidinyl)ethyl]-7-fluoro-1,5-naphthyridin-2(1H)-one;
1-[2-(4-{[(3,5-Dimethylphenyl)methyl]amino}-1-piperidinyl)ethyl]-7-(methyloxy)-1,5-naphthyridin-2(1H)-one;
1-[2-(4-{[(3,4-Dimethylphenyl)methyl]amino}-1-piperidinyl)ethyl]-7-(methyloxy)-1,5-naphthyridin-2(1H)-one;
1-[2-(4-{[(3-fluoro-5-methylphenyl)methyl]amino}-1-piperidinyl)ethyl]-7-(methyloxy)-1,5-naphthyridin-2(1H)-one;
1-[2-(4-{[(4-bromo-3-methylphenyl)methyl]amino}-1-piperidinyl)ethyl]-7-(methyloxy)-1,5-naphthyridin-2(1H)-one;
1-[2-(4-{[(5,6-dichloro-3-pyridinyl)methyl]amino}-1-piperidinyl)ethyl]-7-(methyloxy)-1,5-naphthyridin-2(1H)-one;
1-[2-(4-{[(3-fluoro-4-methylphenyl)methyl]amino}-1-piperidinyl)ethyl]-7-(methyloxy)-1,5-naphthyridin-2(1H)-one;
1-[2-(4-{[(4-chloro-3-methylphenyl)methyl]amino}-1-piperidinyl)ethyl]-7-fluoro-1,5-naphthyridin-2(1H)-one;
1-[2-(4-{[(5-bromo-3-pyridinyl)methyl]amino}-1-piperidinyl)ethyl]-7-fluoro-1,5-naphthyridin-2(1 H)-one;
1-[2-(4-{[(4-bromo-2-thienyl)methyl]amino}-1-piperidinyl)ethyl]-1,5-naphthyridin-2(1H)-one;
1-[2-(4-{[(3,4-dibromophenyl)methyl]amino}-1-piperidinyl)ethyl]-7-fluoro-1,5-naphthyridin-2(1 H)-one;
1-[2-(4-{[(5-bromo-2-thienyl)methyl]amino}-1-piperidinyl)ethyl]-7-fluoro-1,5-naphthyridin-2(1 H)-one;
7-fluoro-1-{2-[4-({[4-(trifluoromethyl)phenyl]methyl}amino)-1-piperidinyl]ethyl}-1,5-naphthyridin-2(1H)-one;
1-[2-(4-{[(3,4-difluorophenyl)methyl]amino}-1-piperidinyl)ethyl]-7-fluoro-1,5-naphthyridin-2(1 H)-one;
1-[2-(4-{[(5-bromo-6-methyl-2-pyridinyl)methyl]amino}-1-piperidinyl)ethyl]-7-(methyloxy)-1,5-naphthyridin-2(1H)-one;
1-[2-(4-{[(3,4-dibromophenyl)methyl]amino}-1-piperidinyl)ethyl]-7-(methyloxy)-1,5-naphthyridin-2(1H)-one;
7-(methyloxy)-1-{2-[4-({[4-(trifluoromethyl)phenyl]methyl}amino)-1-piperidinyl]ethyl}-1,5-naphthyridin-2(1H)-one;
1-[2-(4-{[(3,4-difluorophenyl)methyl]amino}-1-piperidinyl)ethyl]-7-(methyloxy)-1,5-naphthyridin-2(1H)-one;
1-[2-(4-{[(3-chlorophenyl)methyl]amino}-1-piperidinyl)ethyl]-7-(methyloxy)-1,5-naphthyridin-2(1H)-one;
1-{2-[4-({[4-chloro-3-(trifluoromethyl)phenyl]methyl}amino)-1-piperidinyl]ethyl}-7-(methyloxy)-1,5-naphthyridin-2(1H)-one;
1-[2-(4-{[(2,4-dichlorophenyl)methyl]amino}-1-piperidinyl)ethyl]-7-(methyloxy)-1,5-naphthyridin-2(1H)-one;
1-[2-(4-{[(5-bromo-2-pyridinyl)methyl]amino}-1-piperidinyl)ethyl]-7-(methyloxy)-1,5-naphthyridin-2(1H)-one;
1-[2-(4-{[(2-chloro-1,3-thiazol-5-yl)methyl]amino}-1-piperidinyl)ethyl]-7-(methyloxy)-1,5-naphthyridin-2(1H)-one;
1-[2-(4-{[(5-bromo-4-methyl-2-thienyl)methyl]amino}-1-piperidinyl)ethyl]-7-(methyloxy)-1,5-naphthyridin-2(1H)-one
1-[2-((3R,4S)-4-{[(3,4-dichlorophenyl)methyl]amino}-3-hydroxy-1-piperidinyl)ethyl]-7-fluoro-1,5-naphthyridin-2(1H)-one;
1-{2-[(3R,4S)-3-hydroxy-4-({[6-(trifluoromethyl)-3-pyridinyl]methyl}amino)-1-piperidinyl]ethyl}-7-(methyloxy)-1,5-naphthyridin-2(1H)-one;
1-[2-((3R,4S)-4-{[(3,4-dichlorophenyl)methyl]amino}-3-hydroxy-1-piperidinyl)ethyl]-7-(methyloxy)-1,5-naphthyridin-2(1H)-one;
1-{2-[(3S,4R)-3-hydroxy-4-({[6-(trifluoromethyl)-3-pyridinyl]methyl}amino)-1-piperidinyl]ethyl}-7-(methyloxy)-1,5-naphthyridin-2(1H)-one;
1-[2-((3S,4R)-4-{[(3,4-dichlorophenyl)methyl]amino}-3-hydroxy-1-piperidinyl)ethyl]-7-(methyloxy)-1,5-naphthyridin-2(1H)-one;
1-{2-[(3R,4S)-3-hydroxy-4-({[6-(trifluoromethyl)-3-pyridinyl]methyl}amino)-1-piperidinyl]ethyl}-1,5-naphthyridin-2(1H)-one;
7-fluoro-1-{2-[(3R,4S)-3-hydroxy-4-({[6-(trifluoromethyl)-3-pyridinyl]methyl}amino)-1-piperidinyl]ethyl}-1,5-naphthyridin-2(1H)-one;
1-[2-(4-{[(3-chlorophenyl)methyl]amino}-1-piperidinyl)ethyl]-7-fluoro-1,5-naphthyridin-2(1 H)-one;
1-[2-(4-{[(4-chlorophenyl)methyl]amino}-1-piperidinyl)ethyl]-7-fluoro-1,5-naphthyridin-2(1 H)-one;
1-[2-(4-{[(4-bromo-2-thienyl)methyl]amino}-1-piperidinyl)ethyl]-7-(methyloxy)-1,5-naphthyridin-2(1H)-one;
1-[2-(4-{[(5-chloro-2-thienyl)methyl]amino}-1-piperidinyl)ethyl]-7-(methyloxy)-1,5-naphthyridin-2(1H)-one;
1-[2-(4-{[(2-bromo-1,3-thiazol-5-yl)methyl]amino}-1-piperidinyl)ethyl]-7-fluoro-1,5-naphthyridin-2(1H)-one;
1-[2-(4-{[(4,5-dibromo-2-thienyl)methyl]amino}-1-piperidinyl)ethyl]-7-fluoro-1,5-naphthyridin-2(1H)-one;
1-[2-(4-{[(4-bromo-2-thienyl)methyl]amino}-1-piperidinyl)ethyl]-7-fluoro-1,5-naphthyridin-2(1 H)-one;
7-(methyloxy)-1-{2-[4-({[5-(methyloxy)-6-nitro-2-pyridinyl]methyl}amino)-1-piperidinyl]ethyl}-1,5-naphthyridin-2(1H)-one;
1-[2-(4-{[(6-chloro-2-pyrazinyl)methyl]amino}-1-piperidinyl)ethyl]-7-(methyloxy)-1,5-naphthyridin-2(1H)-one;
or a pharmaceutically acceptable salt, solvate or N-oxide thereof.

**7.** A pharmaceutical composition comprising a compound of Formula (I), or a pharmaceutically acceptable salt, solvate or N-oxide thereof, according to any preceding claim and one or more pharmaceutically acceptable carriers, excipients or diluents.

**8.** A method of treatment of tuberculosis in mammals, particularly in man, which method comprises the administration to a mammal in need of such treatment an effective amount of a compound of Formula (I), or a pharmaceutically acceptable salt, solvate or N-oxide thereof.

**9.** A compound of Formula (I), or a pharmaceutically acceptable salt, solvate or N-oxide thereof, according to any preceding claim, for use in therapy.

**10.** A compound of Formula (I), or a pharmaceutically acceptable salt, solvate or N-oxide thereof, for use in the treatment of tuberculosis in mammals, particularly in man.

**11.** Use of a compound of Formula (I), or a pharmaceutically acceptable salt, solvate or N-oxide thereof, in the manufacture of a medicament for use in the treatment of tuberculosis in mammals, particularly in man.

**12.** A process for the preparation of compounds of Formula (I) according to claim 1, from a reaction between a compound of Formula (II), wherein R¹ and R² are as defined for Formula (I) in claim 1, or an acid salt of a compound of Formula (II), and a compound of Formula (III), wherein Ar is as defined for Formula (I) in claim 1, according to the Scheme below.

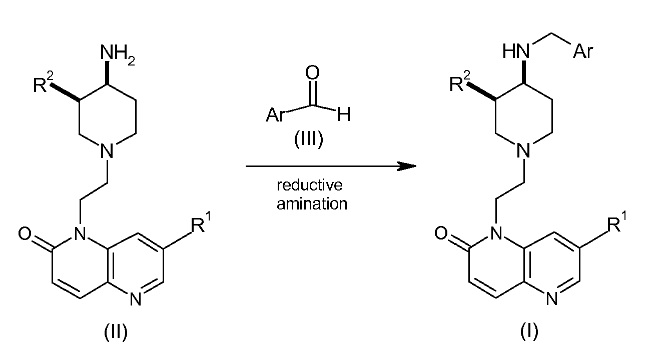

**13.** A process for the preparation of compounds of Formula (I), wherein R¹ and Ar are as defined for Formula (I) in claim 1 and R² is hydrogen, from a reaction between a compound of Formula (II), wherein R¹ is as defined for Formula (I) in claim 1 and R² is hydrogen, and a compound of Formula (IV), wherein hal is a halo group, according to the Scheme below.
